# EUROPEAN PATENT SPECIFICATION

(11) **EP 2 453 012 B1**
(45) Date of publication and mention of the grant of the patent: **01.06.2016**
(21) Application number: 10190629.5
(22) Date of filing: 10.11.2010
(51) Int. Cl.: C12N 9/02, C12N 15/82

(54) **HPPD variants and methods of use**
HPPD-Varianten und Anwendungsverfahren
Variants de HPPD et procédés d'utilisation

(43) Date of publication of application: 16.05.2012
(60) Divisional application: 13164693.7
(73) Proprietor: Bayer CropScience AG, 40789 Monheim am Rhein (DE)
(72) Inventor: Lange, Gudrun, Dr., 65779 Kelkheim (DE); Poree, Fabien, Dr., 65936 Frankfurt (DE); Laber, Bernd, Dr., 65510 Idstein (DE); Freigang, Jörg, Dr., 40764 Langenfeld (DE); Schulz, Arno, Dr., 65817 Eppstein (DE)

(56) References cited:
- WO-A1-99/24585
- WO-A1-2009/144079
- WO-A2-02/46387
- WO-A2-2010/085705
- KAKIDANI HITOSHI ET AL: "Three-dimensional modeling of plant 4-hydroxyphenylpyruvate dioxygenase, a molecular target of triketone-type herbicides", JOURNAL OF PESTICIDE SCIENCE - NIPPON NOYAKU GAKKAISHI, NIPPON NOYAKU GAKKAI, TOKYO, JP, vol. 28, no. 4, 1 January 2003 (2003-01-01), pages 409-415, XP008100740, ISSN: 0385-1559
- FRITZE IRIS M ET AL: "The crystal structures of Zea mays and Arabidopsis 4-hydroxyphenylpyruvate dioxygenase", PLANT PHYSIOLOGY (ROCKVILLE), vol. 134, no. 4, April 2004 (2004-04), pages 1388-1400, XP002631689, ISSN: 0032-0889
- YANG CHENG ET AL: "Structural basis for herbicidal inhibitor selectivity revealed by comparison of crystal structures of plant and mammalian 4-hydroxyphenylpyruvate dioxygenases", BIOCHEMISTRY, vol. 43, no. 32, 17 August 2004 (2004-08-17), pages 10414-10423, XP002631690, ISSN: 0006-2960

### SEQUENCE LISTING

<110> Bayer CropScience AG
<120> HPPD variants and methods of use
<130> BCS 10-1029
<160> 48
<170> PatentIn version 3.3
<210> 1
   <211> 1335
   <212> DNA
   <213> Arabidopsis thaliana
<400> 1
   atgggccacc aaaacgccgc cgtttcagag aatcaaaacc atgatgacgg cgctgcgtcg 60
   tcgccgggat tcaagctcgt cggattttcc aagttcgtaa gaaagaatcc aaagtctgat 120
   aaattcaagg ttaagcgctt ccatcacatc gagttctggt gcggcgacgc aaccaacgtc 180
   gctcgtcgct tctcctgggg tctggggatg agattctccg ccaaatccga tctttccacc 240
   ggaaacatgg ttcacgcctc ttacctactc acctccggtg acctccgatt ccttttcact 300
   gctccttact ctccgtctct ctccgccgga gagattaaac cgacaaccac agcttctatc 360
   ccaagtttcg atcacggctc ttgtcgttcc ttcttctctt cacatggtct cggtgttaga 420
   gccgttgcga ttgaagtaga agacgcagag tcagctttct ccatcagtgt agctaatggc 480
   gctattcctt cgtcgcctcc tatcgtcctc aatgaagcag ttacgatcgc tgaggttaaa 540
   ctatacggcg atgttgttct ccgatatgtt agttacaaag cagaagatac cgaaaaatcc 600
   gaattcttgc cagggttcga gcgtgtagag gatgcgtcgt cgttcccatt ggattatggt 660
   atccggcggc ttgaccacgc cgtgggaaac gttcctgagc ttggtccggc tttaacttat 720
   gtagcggggt tcactggttt tcaccaattc gcagagttca cagcagacga cgttggaacc 780
   gccgagagcg gtttaaattc agcggtcctg gctagcaatg atgaaatggt tcttctaccg 840
   attaacgagc cagtgcacgg aacaaagagg aagagtcaga ttcagacgta tttggaacat 900
   aacgaaggcg cagggctaca acatctggct ctgatgagtg aagacatatt caggaccctg 960
   agagagatga ggaagaggag cagtattgga ggattcgact tcatgccttc tcctccgcct 1020
   acttactacc agaatctcaa gaaacgggtc ggcgacgtgc tcagcgatga tcagatcaag 1080
   gagtgtgagg aattagggat tcttgtagac agagatgatc aagggacgtt gcttcaaatc 1140
   ttcacaaaac cactaggtga caggccgacg atatttatag agataatcca gagagtagga 1200
   tgcatgatga aagatgagga agggaaggct taccagagtg gaggatgtgg tggttttggc 1260
   aaaggcaatt tctctgagct cttcaagtcc attgaagaat acgaaaagac tcttgaagcc 1320
   aaacagttag tggga 1335
<210> 2
   <211> 445
   <212> PRT
   <213> Arabidopsis thaliana
<400> 2
   Met Gly His Gln Asn Ala Ala Val Ser Glu Asn Gln Asn His Asp Asp
   1               5                   10                  15
   Gly Ala Ala Ser Ser Pro Gly Phe Lys Leu Val Gly Phe Ser Lys Phe
               20                  25                  30
   Val Arg Lys Asn Pro Lys Ser Asp Lys Phe Lys Val Lys Arg Phe His
           35                  40                  45
   His Ile Glu Phe Trp Cys Gly Asp Ala Thr Asn Val Ala Arg Arg Phe
       50                  55                  60
   Ser Trp Gly Leu Gly Met Arg Phe Ser Ala Lys Ser Asp Leu Ser Thr
   65                  70                  75                  80
   Gly Asn Met Val His Ala Ser Tyr Leu Leu Thr Ser Gly Asp Leu Arg
                   85                  90                  95
   Phe Leu Phe Thr Ala Pro Tyr Ser Pro Ser Leu Ser Ala Gly Glu Ile
               100                 105                 110
   Lys Pro Thr Thr Thr Ala Ser Ile Pro Ser Phe Asp His Gly Ser Cys
           115                 120                 125
   Arg Ser Phe Phe Ser Ser His Gly Leu Gly Val Arg Ala Val Ala Ile
       130                 135                 140
   Glu Val Glu Asp Ala Glu Ser Ala Phe Ser Ile Ser Val Ala Asn Gly
   145                 150                 155                 160
   Ala Ile Pro Ser Ser Pro Pro Ile Val Leu Asn Glu Ala Val Thr Ile
                   165                 170                 175
   Ala Glu Val Lys Leu Tyr Gly Asp Val Val Leu Arg Tyr Val Ser Tyr
               180                 185                 190
   Lys Ala Glu Asp Thr Glu Lys Ser Glu Phe Leu Pro Gly Phe Glu Arg
           195                 200                 205
   Val Glu Asp Ala Ser Ser Phe Pro Leu Asp Tyr Gly Ile Arg Arg Leu
       210                 215                 220
   Asp His Ala Val Gly Asn Val Pro Glu Leu Gly Pro Ala Leu Thr Tyr
   225                 230                 235                 240
   Val Ala Gly Phe Thr Gly Phe His Gln Phe Ala Glu Phe Thr Ala Asp
                   245                 250                 255
   Asp Val Gly Thr Ala Glu Ser Gly Leu Asn Ser Ala Val Leu Ala Ser
               260                 265                 270
   Asn Asp Glu Met Val Leu Leu Pro Ile Asn Glu Pro Val His Gly Thr
           275                 280                 285
   Lys Arg Lys Ser Gln Ile Gln Thr Tyr Leu Glu His Asn Glu Gly Ala
       290                 295                 300
   Gly Leu Gln His Leu Ala Leu Met Ser Glu Asp Ile Phe Arg Thr Leu
   305                 310                 315                 320
   Arg Glu Met Arg Lys Arg Ser Ser Ile Gly Gly Phe Asp Phe Met Pro
                   325                 330                 335
   Ser Pro Pro Pro Thr Tyr Tyr Gln Asn Leu Lys Lys Arg Val Gly Asp
               340                 345                 350
   Val Leu Ser Asp Asp Gln Ile Lys Glu Cys Glu Glu Leu Gly Ile Leu
           355                 360                 365
   Val Asp Arg Asp Asp Gln Gly Thr Leu Leu Gln Ile Phe Thr Lys Pro
       370                 375                 380
   Leu Gly Asp Arg Pro Thr Ile Phe Ile Glu Ile Ile Gln Arg Val Gly
   385                 390                 395                 400
   Cys Met Met Lys Asp Glu Glu Gly Lys Ala Tyr Gln Ser Gly Gly Cys
                   405                 410                 415
   Gly Gly Phe Gly Lys Gly Asn Phe Ser Glu Leu Phe Lys Ser Ile Glu
   420 425 430
   Glu Tyr Glu Lys Thr Leu Glu Ala Lys Gln Leu Val Gly
   435 440 445
<210> 3
   <211> 1341
   <212> DNA
   <213> Oryza sativa
<400> 3
   atgcctccca ctcccacccc caccgccacc accggcgccg tctcggccgc tgcggcggcg      60
   ggggagaacg cggggttccg cctcgtcggg caccgccgct tcgtccgcgc caacccgcgg     120
   agcgaccggt tccaggcgct cgcgttccac cacgtcgagc tctggtgcgc cgacgccgcg     180
   tccgccgcgg gccggttcgc cttcgccctg ggcgcgccgc tcgccgccag gtccgacctc     240
   tccacgggga actccgcgca cgcctccctc ctcctccgct ccgcctccgt cgcgttcctc     300
   ttcaccgccc cctacggcgg cgaccacggc gtcggcgcgg acgcggccac caccgcctcc     360
   atcccttcct tctccccagg cgccgcgcgg aggttcgccg cggaccacgg cctcgcggtg     420
   cacgccgtgg cgctgcgcgt cgccgacgcg gccgacgcct tccgcgccag cgtcgcggcc     480
   ggtgcgcgcc cggcgttcca gcccgccgac ctcggcggtg gcttcggcct cgcggaggtg     540
   gagctctacg gcgacgtcgt gctccgcttc gtcagccacc cggacggcgc cgacgcgccc     600
   ttcctcccgg gtttcgaggg cgtcagcaac ccgggcgccg tggactacgg cctccgccgg     660
   ttcgaccacg tcgtcggcaa cgtgccggag ctcgctccgg tagccgcgta catctccggg     720
   ttcaccgggt tccacgagtt cgccgagttc accgccgagg acgtgggcac cgccgagagc     780
   ggcctcaact cggtggtgct cgccaacaac gcggagaccg tgctgctgcc gctcaacgag     840
   ccggtgcacg gcaccaagcg gcggagccag atacagacgt acctggacca ccacggcggc     900
   ccgggggtgc agcacatcgc gctggccagc gacgacgtgc tcgggacgct gagggagatg     960
   cgggcgcgct ccgccatggg cggcttcgag ttcttggcgc cgccgccgcc caactactac    1020
   gacggcgtgc ggcggcgcgc cggggacgtg ctctcggagg agcagatcaa cgagtgccag    1080
   gagctcgggg tgctcgtgga cagggatgac cagggggtgt tgctccagat cttcaccaag   1140
   ccagtaggag acaggccaac ctttttcttg gagatgatac aaaggattgg gtgcatggag    1200
   aaggatgaga gtgggcagga gtaccagaag ggcggctgcg gcgggtttgg gaagggcaac    1260
   ttctcggagc tgttcaagtc cattgaggag tatgagaaat cccttgaagc caagcaagcc    1320
   cctacagttc aaggatccta g                                              1341
<210> 4
   <211> 446
   <212> PRT
   <213> Oryza sativa
<400> 4
   Met Pro Pro Thr Pro Thr Pro Thr Ala Thr Thr Gly Ala Val Ser Ala
   1               5                   10                  15
   Ala Ala Ala Ala Gly Glu Asn Ala Gly Phe Arg Leu Val Gly His Arg
               20                  25                  30
   Arg Phe Val Arg Ala Asn Pro Arg Ser Asp Arg Phe Gln Ala Leu Ala
           35                  40                  45
   Phe His His Val Glu Leu Trp Cys Ala Asp Ala Ala Ser Ala Ala Gly
       50                  55                  60
   Arg Phe Ala Phe Ala Leu Gly Ala Pro Leu Ala Ala Arg Ser Asp Leu
   65                  70                  75                  80
   Ser Thr Gly Asn Ser Ala His Ala Ser Leu Leu Leu Arg Ser Ala Ser
                   85                  90                  95
   Val Ala Phe Leu Phe Thr Ala Pro Tyr Gly Gly Asp His Gly Val Gly
               100                 105                 110
   Ala Asp Ala Ala Thr Thr Ala Ser Ile Pro Ser Phe Ser Pro Gly Ala
           115                 120                 125
   Ala Arg Arg Phe Ala Ala Asp His Gly Leu Ala Val His Ala Val Ala
       130                 135                 140
   Leu Arg Val Ala Asp Ala Ala Asp Ala Phe Arg Ala Ser Val Ala Ala
   145                 150                 155                 160
   Gly Ala Arg Pro Ala Phe Gln Pro Ala Asp Leu Gly Gly Gly Phe Gly
                   165                 170                 175
   Leu Ala Glu Val Glu Leu Tyr Gly Asp Val Val Leu Arg Phe Val Ser
               180                 185                 190
   His Pro Asp Gly Ala Asp Ala Pro Phe Leu Pro Gly Phe Glu Gly Val
           195                 200                 205
   Ser Asn Pro Gly Ala Val Asp Tyr Gly Leu Arg Arg Phe Asp His Val
       210                 215                 220
   Val Gly Asn Val Pro Glu Leu Ala Pro Val Ala Ala Tyr Ile Ser Gly
   225                 230                 235                 240
   Phe Thr Gly Phe His Glu Phe Ala Glu Phe Thr Ala Glu Asp Val Gly
                   245                 250                 255
   Thr Ala Glu Ser Gly Leu Asn Ser Val Val Leu Ala Asn Asn Ala Glu
               260                 265                 270
   Thr Val Leu Leu Pro Leu Asn Glu Pro Val His Gly Thr Lys Arg Arg
           275                 280                 285
   Ser Gln Ile Gln Thr Tyr Leu Asp His His Gly Gly Pro Gly Val Gln
       290                 295                 300
   His Ile Ala Leu Ala Ser Asp Asp Val Leu Gly Thr Leu Arg Glu Met
   305                 310                 315                 320
   Arg Ala Arg Ser Ala Met Gly Gly Phe Glu Phe Leu Ala Pro Pro Pro
                   325                 330                 335
   Pro Asn Tyr Tyr Asp Gly Val Arg Arg Arg Ala Gly Asp Val Leu Ser
               340                 345                 350
   Glu Glu Gln Ile Asn Glu Cys Gln Glu Leu Gly Val Leu Val Asp Arg
           355                 360                 365
   Asp Asp Gln Gly Val Leu Leu Gln Ile Phe Thr Lys Pro Val Gly Asp
       370                 375                 380
   Arg Pro Thr Phe Phe Leu Glu Met Ile Gln Arg Ile Gly Cys Met Glu
   385                 390                 395                 400
   Lys Asp Glu Ser Gly Gln Glu Tyr Gln Lys Gly Gly Cys Gly Gly Phe
                   405                 410                 415
   Gly Lys Gly Asn Phe Ser Glu Leu Phe Lys Ser Ile Glu Glu Tyr Glu
               420                 425                 430
   Lys Ser Leu Glu Ala Lys Gln Ala Pro Thr Val Gln Gly Ser
           435                 440                 445
<210> 5
   <211> 1281
   <212> DNA
   <213> Zea mays
<400> 5
   atgcccccga cccccacagc cgccgcagcc ggcgccgccg tggcggcggc atcagcagcg      60
   gagcaggcgg cgttccgcct cgtgggccac cgcaacttcg tccgcttcaa cccgcgctcc     120
   gaccgcttcc acacgctcgc gttccaccac gtggagctct ggtgcgccga cgcggcctcc     180
   gccgcgggcc gcttctcctt cggcctgggc gcgccgctcg ccgcacgctc cgacctctcc     240
   acgggcaact ccgcgcacgc gtccctgctg ctccgctccg gctccctctc cttcctcttc     300
   acggcgccct acgcgcacgg cgccgacgct gccaccgccg cgctgccctc cttctccgcc     360
   gccgccgcgc ggcgcttcgc agccgaccac ggcctcgcgg tgcgcgccgt cgcgctccgc     420
   gtcgccgacg ccgaggacgc cttccgcggc ttccgcctcg ccgaggtcga gctctacggc     480
   gacgtcgtgc tccggtacgt gagctacccg gacggcgccg cgggcgagcc cttcctgccg     540
   gggttcgagg gcgtggccag ccccggggcg gccgactacg ggctgagcag gttcgaccac     600
   atcgtcggca acgtgccgga gctggcgccc gccgccgcct acttcgccgg cttcacgggg     660
   ttccacgagt tcgccgagtt cacgacggag gacgtgggca ccgcggagag cggcctcaac     720
   tccatggtgc tcgccaacaa ctcggagaac gtgctgctcc cgctcaacga gccggtgcac     780
   ggcaccaagc gccgcagcca gatacaaacg ttcctggacc accacggcgg ccccggcgtg     840
   cagcacatgg cgctggccag cgacgacgtg ctcaggacgc tgagggagat gcaggcgcgc     900
   tcggccatgg gcggcttcga gttcatggcg cctcccacat ccgactacta tgacggcgtg     960
   aggcggcgcg ccggggacgt gctcacggaa gcacagatta aggagtgcca ggagctaggg    1020
   gtgctggtgg acagggatga ccagggcgtg ctgctccaaa tcttcaccaa gccagtgggg    1080
   gacaggccaa cgctgttctt ggaaatcatc caaaggatcg ggtgcatgga gaaggatgag    1140
   aaggggcaag aataccaaaa gggtggctgc ggcgggttcg gcaagggaaa cttctcgcag    1200
   ctgttcaagt ccatcgagga ttatgagaag tcccttgaag ccaagcaagc tgctgcagca    1260
   gctgcagctc agggatccta g                                              1281
<210> 6
   <211> 426
   <212> PRT
   <213> Zea mays
<400> 6
   Met Pro Pro Thr Pro Thr Ala Ala Ala Ala Gly Ala Ala Val Ala Ala
   1               5                   10                  15
   Ala Ser Ala Ala Glu Gln Ala Ala Phe Arg Leu Val Gly His Arg Asn
               20                  25                  30
   Phe Val Arg Phe Asn Pro Arg Ser Asp Arg Phe His Thr Leu Ala Phe
           35                  40                  45
   His His Val Glu Leu Trp Cys Ala Asp Ala Ala Ser Ala Ala Gly Arg
       50                  55                  60
   Phe Ser Phe Gly Leu Gly Ala Pro Leu Ala Ala Arg Ser Asp Leu Ser
   65                  70                  75                  80
   Thr Gly Asn Ser Ala His Ala Ser Leu Leu Leu Arg Ser Gly Ser Leu
                   85                  90                  95
   Ser Phe Leu Phe Thr Ala Pro Tyr Ala His Gly Ala Asp Ala Ala Thr
               100                 105                 110
   Ala Ala Leu Pro Ser Phe Ser Ala Ala Ala Ala Arg Arg Phe Ala Ala
           115                 120                 125
   Asp His Gly Leu Ala Val Arg Ala Val Ala Leu Arg Val Ala Asp Ala
       130                 135                 140
   Glu Asp Ala Phe Arg Gly Phe Arg Leu Ala Glu Val Glu Leu Tyr Gly
   145                 150                 155                 160
   Asp Val Val Leu Arg Tyr Val Ser Tyr Pro Asp Gly Ala Ala Gly Glu
                   165                 170                 175
   Pro Phe Leu Pro Gly Phe Glu Gly Val Ala Ser Pro Gly Ala Ala Asp
               180                 185                 190
   Tyr Gly Leu Ser Arg Phe Asp His Ile Val Gly Asn Val Pro Glu Leu
           195                 200                 205
   Ala Pro Ala Ala Ala Tyr Phe Ala Gly Phe Thr Gly Phe His Glu Phe
       210                 215                 220
   Ala Glu Phe Thr Thr Glu Asp Val Gly Thr Ala Glu Ser Gly Leu Asn
   225                 230                 235                 240
   Ser Met Val Leu Ala Asn Asn Ser Glu Asn Val Leu Leu Pro Leu Asn
                   245                 250                 255
   Glu Pro Val His Gly Thr Lys Arg Arg Ser Gln Ile Gln Thr Phe Leu
               260                 265                 270
   Asp His His Gly Gly Pro Gly Val Gln His Met Ala Leu Ala Ser Asp
           275                 280                 285
   Asp Val Leu Arg Thr Leu Arg Glu Met Gln Ala Arg Ser Ala Met Gly
       290                 295                 300
   Gly Phe Glu Phe Met Ala Pro Pro Thr Ser Asp Tyr Tyr Asp Gly Val
   305                 310                 315                 320
   Arg Arg Arg Ala Gly Asp Val Leu Thr Glu Ala Gln Ile Lys Glu Cys
                   325                 330                 335
   Gln Glu Leu Gly Val Leu Val Asp Arg Asp Asp Gln Gly Val Leu Leu
               340                 345                 350
   Gln Ile Phe Thr Lys Pro Val Gly Asp Arg Pro Thr Leu Phe Leu Glu
           355                 360                 365
   Ile Ile Gln Arg Ile Gly Cys Met Glu Lys Asp Glu Lys Gly Gln Glu
       370                 375                 380
   Tyr Gln Lys Gly Gly Cys Gly Gly Phe Gly Lys Gly Asn Phe Ser Gln
   385                 390                 395                 400
   Leu Phe Lys Ser Ile Glu Asp Tyr Glu Lys Ser Leu Glu Ala Lys Gln
                   405                 410                 415
   Ala Ala Ala Ala Ala Ala Ala Gln Gly Ser
               420                 425
<210> 7
   <211> 1323
   <212> DNA
   <213> Avena sativa
<400> 7
   atgccgccca cccccgccac cgccaccggc gccgccgcgg ccgccgtgac tccagagcac      60
   gcggcccgga gctttccccg agtggtccgc gtcaacccgc gcagcgaccg cttccccgtg     120
   ctctccttcc accacgtcga gctctggtgc gccgacgccg cctcagcggc cggacgcttc    180
   tccttcgcgc tcggcgcgcc gctcgccgcc cggtccgacc tctccacggg gaactccgcg    240
   cacgcctccc tcctgctccg ctcgggcgcc ctcgccttcc tcttcacggc gccctacgcg    300
   ccgccgccgc aggaggccgc cacggccgca gccaccgcct ccatcccctc cttctccgcc    360
   gacgccgcgc ggacgttcgc cgccgcccac ggcctcgcgg tgcgctccgt cggggtccgc    420
   gtcgctgacg ccgccgaggc cttccgcgtc agcgtagccg gcggcgctcg cccggccttc    480
   gccccagccg acctcggcca tggcttcggc ctcgccgagg tcgagctcta cggcgacgtc    540
   gtgctacgct tcgtcagcta cccggacgag acagacctgc cattcctgcc agggttcgag    600
   cgcgtgagca gccccggcgc cgtggactac ggcctcacgc ggttcgacca cgtcgtgggc    660
   aacgtcccgg agatggcccc ggtcatagac tacatgaaag gcttcttggg gttccacgag    720
   ttcgccgagt tcaccgccga ggacgtgggc acgaccgaga gcgggctcaa ctcggtggtg    780
   ctcgccaaca actccgaggc cgtgctgctg ccgctcaacg agcccgtgca cggcacaaag    840
   cgacggagcc agatacagac gtacctggag tatcacggcg ggcccggcgt gcagcacatc    900
   gcgctcgcca gcaacgacgt gctcaggacg ctcagggaga tgcgggcgcg cacgcccatg    960
   ggcggcttcg agttcatggc gccaccgcag gcgaaatact atgaaggcgt gcggcgcatc   1020
   gcaggtgacg tgctctcgga agagcagatc aaggaatgcc aggagctggg ggtgctagtc   1080
   gacagggatg atcaaggggt gttgctccaa atcttcacca agccagtagg ggacaggcca   1140
   acgtttttcc tggagatgat ccaaagaatc gggtgcatgg agaaggacga ggtcgggcaa   1200
   gagtaccaga agggtggctg cggcgggttt ggcaagggca atttctccga gctgttcaag   1260
   tccattgagg actatgagaa atcccttgag gtcaagcaat ctgttgtagc tcagaaatcc   1320
   tag                                                                 1323
<210> 8
   <211> 440
   <212> PRT
   <213> Avena sativa
<400> 8
   Met Pro Pro Thr Pro Ala Thr Ala Thr Gly Ala Ala Ala Ala Ala Val
   1               5                   10                  15
   Thr Pro Glu His Ala Ala Arg Ser Phe Pro Arg Val Val Arg Val Asn
               20                  25                  30
   Pro Arg Ser Asp Arg Phe Pro Val Leu Ser Phe His His Val Glu Leu
           35                  40                  45
   Trp Cys Ala Asp Ala Ala Ser Ala Ala Gly Arg Phe Ser Phe Ala Leu
       50                  55                  60
   Gly Ala Pro Leu Ala Ala Arg Ser Asp Leu Ser Thr Gly Asn Ser Ala
   65                  70                  75                  80
   His Ala Ser Leu Leu Leu Arg Ser Gly Ala Leu Ala Phe Leu Phe Thr
                   85                  90                  95
   Ala Pro Tyr Ala Pro Pro Pro Gln Glu Ala Ala Thr Ala Ala Ala Thr
               100                 105                 110
   Ala Ser Ile Pro Ser Phe Ser Ala Asp Ala Ala Arg Thr Phe Ala Ala
           115                 120                 125
   Ala His Gly Leu Ala Val Arg Ser Val Gly Val Arg Val Ala Asp Ala
       130                 135                 140
   Ala Glu Ala Phe Arg Val Ser Val Ala Gly Gly Ala Arg Pro Ala Phe
   145                 150                 155                 160
   Ala Pro Ala Asp Leu Gly His Gly Phe Gly Leu Ala Glu Val Glu Leu
                   165                 170                 175
   Tyr Gly Asp Val Val Leu Arg Phe Val Ser Tyr Pro Asp Glu Thr Asp
               180                 185                 190
   Leu Pro Phe Leu Pro Gly Phe Glu Arg Val Ser Ser Pro Gly Ala Val
           195                 200                 205
   Asp Tyr Gly Leu Thr Arg Phe Asp His Val Val Gly Asn Val Pro Glu
       210                 215                 220
   Met Ala Pro Val Ile Asp Tyr Met Lys Gly Phe Leu Gly Phe His Glu
   225                 230                 235                 240
   Phe Ala Glu Phe Thr Ala Glu Asp Val Gly Thr Thr Glu Ser Gly Leu
                   245                 250                 255
   Asn Ser Val Val Leu Ala Asn Asn Ser Glu Ala Val Leu Leu Pro Leu
               260                 265                 270
   Asn Glu Pro Val His Gly Thr Lys Arg Arg Ser Gln Ile Gln Thr Tyr
           275                 280                 285
   Leu Glu Tyr His Gly Gly Pro Gly Val Gln His Ile Ala Leu Ala Ser
       290                 295                 300
   Asn Asp Val Leu Arg Thr Leu Arg Glu Met Arg Ala Arg Thr Pro Met
   305                 310                 315                 320
   Gly Gly Phe Glu Phe Met Ala Pro Pro Gln Ala Lys Tyr Tyr Glu Gly
                   325                 330                 335
   Val Arg Arg Ile Ala Gly Asp Val Leu Ser Glu Glu Gln Ile Lys Glu
               340                 345                 350
   Cys Gln Glu Leu Gly Val Leu Val Asp Arg Asp Asp Gln Gly Val Leu
           355                 360                 365
   Leu Gln Ile Phe Thr Lys Pro Val Gly Asp Arg Pro Thr Phe Phe Leu
       370                 375                 380
   Glu Met Ile Gln Arg Ile Gly Cys Met Glu Lys Asp Glu Val Gly Gln
   385                 390                 395                 400
   Glu Tyr Gln Lys Gly Gly Cys Gly Gly Phe Gly Lys Gly Asn Phe Ser
                   405                 410                 415
   Glu Leu Phe Lys Ser Ile Glu Asp Tyr Glu Lys Ser Leu Glu Val Lys
               420                 425                 430
   Gln Ser Val Val Ala Gln Lys Ser
           435                 440
<210> 9
   <211> 1077
   <212> DNA
   <213> Pseudomonas fluorescens
<400> 9
   atggcagatc tatacgaaaa cccaatgggc ctgatgggct ttgaattcat cgaattcgcg      60
   tcgccgacgc cgggtaccct ggagccgatc ttcgagatca tgggcttcac caaagtcgcg     120
   acccaccgtt ccaagaacgt gcacctgtac cgccagggcg agatcaacct gatcctcaac     180
   aacgagccca acagcatcgc ctcctacttt gcggccgaac acggcccgtc ggtgtgcggc     240
   atggcgttcc gcgtgaagga ctcgcaaaag gcctacaacc gcgccctgga actcggcgcc    300
   cagccgatcc atattgacac cgggccgatg gaattgaacc tgccggcgat caagggcatc    360
   ggcggcgcgc cgttgtacct gatcgaccgt ttcggcgaag gcagctcgat ctacgacatc    420
   gacttcgtgt acctcgaagg tgtggagcgc aatccggtcg gtgcaggtct caaagtcatc    480
   gaccacctga cccacaacgt ctatcgcggc cgcatggtct actgggccaa cttctacgag    540
   aaattgttca acttccgtga agcgcgttac ttcgatatca agggcgagta caccggcctg    600
   acttccaagg ccatgagtgc gccggacggc atgatccgca tcccgctgaa cgaagagtcg    660
   tccaagggcg cggggcagat cgaagagttc ctgatgcagt tcaacggcga aggcatccag    720
   cacgtggcgt tcctcaccga cgacctggtc aagacctggg acgcgttgaa gaaaatcggc    780
   atgcgcttca tgaccgcgcc gccagacact tattacgaaa tgctcgaagg ccgcctgcct    840
   gaccacggcg agccggtgga tcaactgcag gcacgcggta tcctgctgga cggatcttcc    900
   gtggaaggcg acaaacgcct gctgctgcag atcttctcgg aaaccctgat gggcccggtg    960
   ttcttcgaat tcatccagcg caagggcgac gatgggtttg gcgagggcaa cttcaaggcg   1020
   ctgttcgagt ccatcgaacg tgaccaggtg cgtcgtggtg tattgaccgc cgattaa      1077
<210> 10
   <211> 358
   <212> PRT
   <213> Pseudomonas fluorescens
<400> 10
   Met Ala Asp Leu Tyr Glu Asn Pro Met Gly Leu Met Gly Phe Glu Phe
   1               5                   10                  15
   Ile Glu Phe Ala Ser Pro Thr Pro Gly Thr Leu Glu Pro Ile Phe Glu
               20                  25                  30
   Ile Met Gly Phe Thr Lys Val Ala Thr His Arg Ser Lys Asn Val His
           35                  40                  45
   Leu Tyr Arg Gln Gly Glu Ile Asn Leu Ile Leu Asn Asn Glu Pro Asn
       50                  55                  60
   Ser Ile Ala Ser Tyr Phe Ala Ala Glu His Gly Pro Ser Val Cys Gly
   65                  70                  75                  80
   Met Ala Phe Arg Val Lys Asp Ser Gln Lys Ala Tyr Asn Arg Ala Leu
                   85                  90                  95
   Glu Leu Gly Ala Gln Pro Ile His Ile Asp Thr Gly Pro Met Glu Leu
               100                 105                 110
   Asn Leu Pro Ala Ile Lys Gly Ile Gly Gly Ala Pro Leu Tyr Leu Ile
           115                 120                 125
   Asp Arg Phe Gly Glu Gly Ser Ser Ile Tyr Asp Ile Asp Phe Val Tyr
       130                 135                 140
   Leu Glu Gly Val Glu Arg Asn Pro Val Gly Ala Gly Leu Lys Val Ile
   145                 150                 155                 160
   Asp His Leu Thr His Asn Val Tyr Arg Gly Arg Met Val Tyr Trp Ala
                   165                 170                 175
   Asn Phe Tyr Glu Lys Leu Phe Asn Phe Arg Glu Ala Arg Tyr Phe Asp
               180                 185                 190
   Ile Lys Gly Glu Tyr Thr Gly Leu Thr Ser Lys Ala Met Ser Ala Pro
           195                 200                 205
   Asp Gly Met Ile Arg Ile Pro Leu Asn Glu Glu Ser Ser Lys Gly Ala
       210                 215                 220
   Gly Gln Ile Glu Glu Phe Leu Met Gln Phe Asn Gly Glu Gly Ile Gln
   225                 230                 235                 240
   His Val Ala Phe Leu Thr Asp Asp Leu Val Lys Thr Trp Asp Ala Leu
                   245                 250                 255
   Lys Lys Ile Gly Met Arg Phe Met Thr Ala Pro Pro Asp Thr Tyr Tyr
               260                 265                 270
   Glu Met Leu Glu Gly Arg Leu Pro Asp His Gly Glu Pro Val Asp Gln
           275                 280                 285
   Leu Gln Ala Arg Gly Ile Leu Leu Asp Gly Ser Ser Val Glu Gly Asp
       290                 295                 300
   Lys Arg Leu Leu Leu Gln Ile Phe Ser Glu Thr Leu Met Gly Pro Val
   305                 310                 315                 320
   Phe Phe Glu Phe Ile Gln Arg Lys Gly Asp Asp Gly Phe Gly Glu Gly
                   325                 330                 335
   Asn Phe Lys Ala Leu Phe Glu Ser Ile Glu Arg Asp Gln Val Arg Arg
               340                 345                 350
   Gly Val Leu Thr Ala Asp
           355
<210> 13
   <211> 1053
   <212> DNA
   <213> Synechococcus sp.
<400> 13
   atgaacccgt ccattcgaat tgtccaaggg atccaccacc tgcacttcta cctttgggat      60
   ctgccccgtt ggcgggaaca cttttgtcgg gtttggggct tccgggtggc aagcgacgcc     120
   ggcaacaccc tggagctgga gcagggatcc ctgcgcttgc gcctgtctca gccggcacgg     180
   gcgggggacg aggtggaccg ccatttgcag cggcatgggc cgggggtggt ggatgtggcc     240
   ttggcggtgg gagagcagga gctaccggcc ttggcggagc tgttgcgggg ccgaggcgcc     300
   caactggcgt ggatcccggc agcagcggcg ctctgcctcc acacccccta cgggatccgg     360
   cattctctga tccctggccc cttggatgcc gcccctgccg aagcgggcct gttttcccac     420
   tgggatcacg tggtgttgaa cgtggagcag ggatccctgc aggcggcagc cgactggtat     480
   gggcgggtgc tgggctggcg gcggctgtac cgctacagca tcggcaccgc cacctccggc     540
   ctggaaagcg tggtggtggg ggatccggaa gcggggatcc aatgggccat caacgagccc     600
   acctgtgccg cttcccagat tcaggagttt ttgcatgccc atggcggccc gggcattcag     660
   cacgcggcgc tgcacagctc agacattgtt gccagcctgc gccggttgcg gcagggggga     720
   gtggactttt tgcaagtggc gccgcagtac tacaccagcc tggaaaggga gctggggttg     780
   gcgctccgtt ctgcccttgg gcaggccatc tcctggcaag acctggtgga gcagcagatc     840
   cttctggatg ctaccctgcc cgcttctgat ggccaggatc gcccccttct gctgcagacc     900
   tttacccagc ccctctttgg tcggcccacc tttttctttg aagtcattca acggctaggc     960
   ggggccacgg gctttggcga ggccaatttt caggctttgt tcgaggccct ggaacggcaa    1020
   cagcgacagc gacaccaggc gctgacccct tag                                 1053
<210> 14
   <211> 350
   <212> PRT
   <213> Synechococcus sp.
<400> 14
   Met Asn Pro Ser Ile Arg Ile Val Gln Gly Ile His His Leu His Phe
   1               5                   10                  15
   Tyr Leu Trp Asp Leu Pro Arg Trp Arg Glu His Phe Cys Arg Val Trp
               20                  25                  30
   Gly Phe Arg Val Ala Ser Asp Ala Gly Asn Thr Leu Glu Leu Glu Gln
           35                  40                  45
   Gly Ser Leu Arg Leu Arg Leu Ser Gln Pro Ala Arg Ala Gly Asp Glu
       50                  55                  60
   Val Asp Arg His Leu Gln Arg His Gly Pro Gly Val Val Asp Val Ala
   65                  70                  75                  80
   Leu Ala Val Gly Glu Gln Glu Leu Pro Ala Leu Ala Glu Leu Leu Arg
                   85                  90                  95
   Gly Arg Gly Ala Gln Leu Ala Trp Ile Pro Ala Ala Ala Ala Leu Cys
               100                 105                 110
   Leu His Thr Pro Tyr Gly Ile Arg His Ser Leu Ile Pro Gly Pro Leu
           115                 120                 125
   Asp Ala Ala Pro Ala Glu Ala Gly Leu Phe Ser His Trp Asp His Val
       130                 135                 140
   Val Leu Asn Val Glu Gln Gly Ser Leu Gln Ala Ala Ala Asp Trp Tyr
   145                 150                 155                 160
   Gly Arg Val Leu Gly Trp Arg Arg Leu Tyr Arg Tyr Ser Ile Gly Thr
                   165                 170                 175
   Ala Thr Ser Gly Leu Glu Ser Val Val Val Gly Asp Pro Glu Ala Gly
               180                 185                 190
   Ile Gln Trp Ala Ile Asn Glu Pro Thr Cys Ala Ala Ser Gln Ile Gln
           195                 200                 205
   Glu Phe Leu His Ala His Gly Gly Pro Gly Ile Gln His Ala Ala Leu
       210                 215                 220
   His Ser Ser Asp Ile Val Ala Ser Leu Arg Arg Leu Arg Gln Gly Gly
   225                 230                 235                 240
   Val Asp Phe Leu Gln Val Ala Pro Gln Tyr Tyr Thr Ser Leu Glu Arg
                   245                 250                 255
   Glu Leu Gly Leu Ala Leu Arg Ser Ala Leu Gly Gln Ala Ile Ser Trp
               260                 265                 270
   Gln Asp Leu Val Glu Gln Gln Ile Leu Leu Asp Ala Thr Leu Pro Ala
           275                 280                 285
   Ser Asp Gly Gln Asp Arg Pro Leu Leu Leu Gln Thr Phe Thr Gln Pro
       290                 295                 300
   Leu Phe Gly Arg Pro Thr Phe Phe Phe Glu Val Ile Gln Arg Leu Gly
   305                 310                 315                 320
   Gly Ala Thr Gly Phe Gly Glu Ala Asn Phe Gln Ala Leu Phe Glu Ala
                   325                 330                 335
   Leu Glu Arg Gln Gln Arg Gln Arg His Gln Ala Leu Thr Pro
               340                 345                 350
<210> 15
   <211> 1149
   <212> DNA
   <213> Blepharisma japonicum
<400> 15
   atgacttatt acgacaagca agaaacgcgt ccagatcttg gcgaattcta tggtttccat      60
   cacgttcgtt tttacgtctc caactcagag caagccgctt cgttctacac atctcgcttt     120
   gggttttctc cggttgccta tgaaggattg gaaacaggaa accaaaaatt ctgtaccaat     180
   gtcgtccgaa gcaaccatgt agtcatcgct tttacctcag ctctcactcc tgaagacaat     240
   gaagtgaacc gtcacgttgg caagcatagt gatggagttc aagacattgc ctttagtgta     300
   agtgacgcaa gagggatgta tgagaaagcg atagctaaag gctgtaaaag cttccgtgag     360
   ccacaggttt tacaagatca atttggatct gttataatag cgtctctcca gacttatgga     420
   gacactgttc acacattagt ccaaaatgtc gactatacag gacccttttt gcctggcttc      480
   agagcaatca caaaagatga tccattaaac tctgcctttc ctcaggtaaa ttatgacatt     540
   attgatcatg ttgtaggaaa tcagcctggt ggcgatatga ctcctacagt agaatggtat     600
   gagaaatatc tagaatttca tcgatattgg tctgctgatg agtctgtaat ccataccgat     660
   tattcagcat taaggtctgt tgtggttgct gattgggatg aagtgatcaa aatgcctatt     720
   aatgagcctg ctgatggact tagaaaaagt caaatccaag aatatgtcga atattatggt    780
   ggagcaggcg tacaacatat tgccttaaaa gtcaatgata ttatttcagt aataagcacc    840
   ttaagggcta gaggtgtgga attcttagaa gttcctccta aatattatga tagcttaaga    900
   aaaagacttg cgcattctgc ggtacaaatt gaagaagact taaaaagaat tgaagacctt    960
   catattttgg ttgactttga cgaccgtggg tatttacttc agattttcac aaaaccagta   1020
   gaagacagac ctactctgtt ttatgaaatt attcaaagac ataataacaa tggattcgga   1080
   attggaaatt ttaaagccct atttgaatca ttggaacaag agcaagaaag aagaggtaat   1140
   ttgatctaa                                                           1149
<210> 16
   <211> 382
   <212> PRT
   <213> Blepharisma japonicum
<400> 16
   Met Thr Tyr Tyr Asp Lys Gln Glu Thr Arg Pro Asp Leu Gly Glu Phe
   1               5                   10                  15
   Tyr Gly Phe His His Val Arg Phe Tyr Val Ser Asn Ser Glu Gln Ala
               20                  25                  30
   Ala Ser Phe Tyr Thr Ser Arg Phe Gly Phe Ser Pro Val Ala Tyr Glu
           35                  40                  45
   Gly Leu Glu Thr Gly Asn Gln Lys Phe Cys Thr Asn Val Val Arg Ser
       50                  55                  60
   Asn His Val Val Ile Ala Phe Thr Ser Ala Leu Thr Pro Glu Asp Asn
   65                  70                  75                  80
   Glu Val Asn Arg His Val Gly Lys His Ser Asp Gly Val Gln Asp Ile
                   85                  90                  95
   Ala Phe Ser Val Ser Asp Ala Arg Gly Met Tyr Glu Lys Ala Ile Ala
               100                 105                 110
   Lys Gly Cys Lys Ser Phe Arg Glu Pro Gln Val Leu Gln Asp Gln Phe
           115                 120                 125
   Gly Ser Val Ile Ile Ala Ser Leu Gln Thr Tyr Gly Asp Thr Val His
       130                 135                 140
   Thr Leu Val Gln Asn Val Asp Tyr Thr Gly Pro Phe Leu Pro Gly Phe
   145                 150                 155                 160
   Arg Ala Ile Thr Lys Asp Asp Pro Leu Asn Ser Ala Phe Pro Gln Val
                   165                 170                 175
   Asn Tyr Asp Ile Ile Asp His Val Val Gly Asn Gln Pro Gly Gly Asp
               180                 185                 190
   Met Thr Pro Thr Val Glu Trp Tyr Glu Lys Tyr Leu Glu Phe His Arg
           195                 200                 205
   Tyr Trp Ser Ala Asp Glu Ser Val Ile His Thr Asp Tyr Ser Ala Leu
       210                 215                 220
   Arg Ser Val Val Val Ala Asp Trp Asp Glu Val Ile Lys Met Pro Ile
   225                 230                 235                 240
   Asn Glu Pro Ala Asp Gly Leu Arg Lys Ser Gln Ile Gln Glu Tyr Val
                   245                 250                 255
   Glu Tyr Tyr Gly Gly Ala Gly Val Gln His Ile Ala Leu Lys Val Asn
               260                 265                 270
   Asp Ile Ile Ser Val Ile Ser Thr Leu Arg Ala Arg Gly Val Glu Phe
           275                 280                 285
   Leu Glu Val Pro Pro Lys Tyr Tyr Asp Ser Leu Arg Lys Arg Leu Ala
       290                 295                 300
   His Ser Ala Val Gln Ile Glu Glu Asp Leu Lys Arg Ile Glu Asp Leu
   305                 310                 315                 320
   His Ile Leu Val Asp Phe Asp Asp Arg Gly Tyr Leu Leu Gln Ile Phe
                   325                 330                 335
   Thr Lys Pro Val Glu Asp Arg Pro Thr Leu Phe Tyr Glu Ile Ile Gln
               340                 345                 350
   Arg His Asn Asn Asn Gly Phe Gly Ile Gly Asn Phe Lys Ala Leu Phe
           355                 360                 365
   Glu Ser Leu Glu Gln Glu Gln Glu Arg Arg Gly Asn Leu Ile
              370                 375                 380
<210> 17
   <211> 1206
   <212> DNA
   <213> Rhodococcus sp.
<400> 17
   atgacgatcg agcagactct caccgacaag gaacgcctgg caggtctcga cctcggccag      60
   ctcgagcagt tggtcgggct cgtcgagtac gacggcaccc gcgacccgtt cccggtcagc     120
   ggctgggatg ccgtcgtctg ggtggtcggc aacgccaccc agaccgccca ctacttccag     180
   tccgcgttcg ggatgaccct cgtcgcctac tccggaccca ccaccggcaa ccgcgaccac     240
   cacagcttcg tcctcgaatc cggggccgtc cgcttcgtca tcaaaggcgc cgtgaacccg     300
   gacagccccc tgatcgacca ccaccgcacc cacggcgacg gcgtcgtcga catcgccctc     360
   gccgtccccg acgtcgacaa gtgcatcgcc cacgcccgcg cccagggcgc caccgtcctc     420
   gacgaacccc acgacgtgac cgacgaccac ggcaccgtcc gcctcgccgc gatcgccacc     480
   tacggcgaca cccgccacac cctcgtcgac cgcagccact acaccggccc ctacctgccc     540
   ggctacaccg cccgcacctc cggccacacc aaacgggacg gggcacccaa gcgcctgttc     600
   caggccctcg accacgtcgt cggcaacgtc gaactcggca agatggacca ctgggtcgac     660
   ttctacaacc gggtcatggg ctttacgaac atggccgagt tcgtcggcga ggacatcgcc     720
   accgactact ccgcgctgat gagcaaggtc gtctccaacg gcaaccaccg ggtcaagttc     780
   cccctcaacg aacccgccct cgccaagaaa cgctcgcaga tcgacgaata cctcgacttc     840
   taccgcggcc ccggcgccca gcacctggcc ctggccacca atgacatcct caccgccgtc     900
   gaccagctga ccgccgaggg cgtcgagttc ctggccaccc ccgactccta ctacgaggac     960
   cccgaactgc gggcccggat cggcaacgtc cgcgccccca tcgccgaact gcagaaacgc    1020
   ggcatcctcg tcgaccgcga cgaagacggc tacctgctgc agatcttcac caaacccctc    1080
   gtcgaccggc ccaccgtgtt cttcgaactc atcgaacgcc acggctccct cggcttcggc    1140
   atcggcaact tcaaagccct cttcgaggcc atcgaacgcg aacaagccgc ccgcggaaac    1200
   ttctga                                                               1206
<210> 18
   <211> 401
   <212> PRT
   <213> Rhodococcus sp.
<400> 18
   Met Thr Ile Glu Gln Thr Leu Thr Asp Lys Glu Arg Leu Ala Gly Leu
   1               5                   10                  15
   Asp Leu Gly Gln Leu Glu Gln Leu Val Gly Leu Val Glu Tyr Asp Gly
               20                  25                  30
   Thr Arg Asp Pro Phe Pro Val Ser Gly Trp Asp Ala Val Val Trp Val
           35                  40                  45
   Val Gly Asn Ala Thr Gln Thr Ala His Tyr Phe Gln Ser Ala Phe Gly
       50                  55                  60
   Met Thr Leu Val Ala Tyr Ser Gly Pro Thr Thr Gly Asn Arg Asp His
   65                  70                  75                  80
   His Ser Phe Val Leu Glu Ser Gly Ala Val Arg Phe Val Ile Lys Gly
                   85                  90                  95
   Ala Val Asn Pro Asp Ser Pro Leu Ile Asp His His Arg Thr His Gly
               100                 105                 110
   Asp Gly Val Val Asp Ile Ala Leu Ala Val Pro Asp Val Asp Lys Cys
           115                 120                 125
   Ile Ala His Ala Arg Ala Gln Gly Ala Thr Val Leu Asp Glu Pro His
       130                 135                 140
   Asp Val Thr Asp Asp His Gly Thr Val Arg Leu Ala Ala Ile Ala Thr
   145                 150                 155                 160
   Tyr Gly Asp Thr Arg His Thr Leu Val Asp Arg Ser His Tyr Thr Gly
                   165                 170                 175
   Pro Tyr Leu Pro Gly Tyr Thr Ala Arg Thr Ser Gly His Thr Lys Arg
               180                 185                 190
   Asp Gly Ala Pro Lys Arg Leu Phe Gln Ala Leu Asp His Val Val Gly
           195                 200                 205
   Asn Val Glu Leu Gly Lys Met Asp His Trp Val Asp Phe Tyr Asn Arg
       210                 215                 220
   Val Met Gly Phe Thr Asn Met Ala Glu Phe Val Gly Glu Asp Ile Ala
   225                 230                 235                 240
   Thr Asp Tyr Ser Ala Leu Met Ser Lys Val Val Ser Asn Gly Asn His
   245 250 255
   Arg Val Lys Phe Pro Leu Asn Glu Pro Ala Leu Ala Lys Lys Arg Ser
   260 265 270
   Gln Ile Asp Glu Tyr Leu Asp Phe Tyr Arg Gly Pro Gly Ala Gln His
   275 280 285
   Leu Ala Leu Ala Thr Asn Asp Ile Leu Thr Ala Val Asp Gln Leu Thr
   290 295 300
   Ala Glu Gly Val Glu Phe Leu Ala Thr Pro Asp Ser Tyr Tyr Glu Asp
   305 310 315 320
   Pro Glu Leu Arg Ala Arg Ile Gly Asn Val Arg Ala Pro Ile Ala Glu
   325 330 335
   Leu Gln Lys Arg Gly Ile Leu Val Asp Arg Asp Glu Asp Gly Tyr Leu
   340 345 350
   Leu Gln Ile Phe Thr Lys Pro Leu Val Asp Arg Pro Thr Val Phe Phe
   355 360 365
   Glu Leu Ile Glu Arg His Gly Ser Leu Gly Phe Gly Ile Gly Asn Phe
   370 375 380
   Lys Ala Leu Phe Glu Ala Ile Glu Arg Glu Gln Ala Ala Arg Gly Asn
   385 390 395 400
   Phe
<210> 19
   <211> 1107
   <212> DNA
   <213> Picrophilus torridus
<400> 19
   atgtatggca aaaatttaat ctcagaacta agggaaaagg agatctttaa acgattacat 60
   cacgtggaat tttacgttag cagtgccaaa acatggtcat atttcatgaa caggggtctt 120
   ggatttaaaa cagtggcata tgccggtcca gaaaccggga taagggacaa gatatcctat 180
   gttatgtccc agggcactgc aaggatatct tttacatcat caatgaatga tgatagctat 240
   atatcgaatc atgttaaaaa acacggggat ggcgtaaagg atatagcact tgaggtcgat 300
   gatctggacg aggcaaaaag cctgatagaa aagtatggaa caaaggtttc aaaaataaat 360
   gaaataaagg atggaaatgg aaagataaga actgcagaga taaaaacgta cggtgaaacc 420
   gttcatacat taatagaaac cggggattac aatggcgtat tcatgcccgg ttatgaggaa 480
   tctgaaataa attcaaaaaa cactgggata aaaaagatcg atcatatagt tggaaatgtc 540
   tatgagggcg agatggatag ctgggttaat ttttacatag aaaaacttgg ctttgagcat 600
   ttaataacct ttgatgataa agatataaga actgattaca gcgcattaag atcaaaggtt 660
   gtaaaataca atgacgatat cgtatttcca ataaatgagc ctgcaaaggg cttaagaaaa 720
   tcacagatag aggaatatct tgactattac aggtctgagg gcgttcagca catagcactg 780
   ttaactgatg atataataaa aactgtatcc atgatggagg aaaacggcat agaattttta 840
   aaaacaccag gatcatacta tgaatcccta tcatcaagga taggctcaat agacgaggat 900
   ttaaatgaaa tagagaaaca taacatactt gtggatcgtg atgagaacgg atacctatta 960
   cagatcttca caaagcctgt tactgacagg ccaacgttct tctttgaggt catacagaga 1020
   aagggtgcaa ggtcattcgg caacggtaac tttaaggcac tttttgaggc gatagaaagg 1080
   gagcaggcaa agagaggaaa cctatga 1107
<210> 20
   <211> 368
   <212> PRT
   <213> Picrophilus torridus
<400> 20
   Met Tyr Gly Lys Asn Leu Ile Ser Glu Leu Arg Glu Lys Glu Ile Phe
   1 5 10 15
   Lys Arg Leu His His Val Glu Phe Tyr Val Ser Ser Ala Lys Thr Trp
   20 25 30
   Ser Tyr Phe Met Asn Arg Gly Leu Gly Phe Lys Thr Val Ala Tyr Ala
   35 40 45
   Gly Pro Glu Thr Gly Ile Arg Asp Lys Ile Ser Tyr Val Met Ser Gln
   50 55 60
   Gly Thr Ala Arg Ile Ser Phe Thr Ser Ser Met Asn Asp Asp Ser Tyr
   65 70 75 80
   Ile Ser Asn His Val Lys Lys His Gly Asp Gly Val Lys Asp Ile Ala
   85 90 95
   Leu Glu Val Asp Asp Leu Asp Glu Ala Lys Ser Leu Ile Glu Lys Tyr
   100 105 110
   Gly Thr Lys Val Ser Lys Ile Asn Glu Ile Lys Asp Gly Asn Gly Lys
   115 120 125
   Ile Arg Thr Ala Glu Ile Lys Thr Tyr Gly Glu Thr Val His Thr Leu
   130 135 140
   Ile Glu Thr Gly Asp Tyr Asn Gly Val Phe Met Pro Gly Tyr Glu Glu
   145 150 155 160
   Ser Glu Ile Asn Ser Lys Asn Thr Gly Ile Lys Lys Ile Asp His Ile
   165 170 175
   Val Gly Asn Val Tyr Glu Gly Glu Met Asp Ser Trp Val Asn Phe Tyr
   180 185 190
   Ile Glu Lys Leu Gly Phe Glu His Leu Ile Thr Phe Asp Asp Lys Asp
   195 200 205
   Ile Arg Thr Asp Tyr Ser Ala Leu Arg Ser Lys Val Val Lys Tyr Asn
   210 215 220
   Asp Asp Ile Val Phe Pro Ile Asn Glu Pro Ala Lys Gly Leu Arg Lys
   225 230 235 240
   Ser Gln Ile Glu Glu Tyr Leu Asp Tyr Tyr Arg Ser Glu Gly Val Gln
   245 250 255
   His Ile Ala Leu Leu Thr Asp Asp Ile Ile Lys Thr Val Ser Met Met
   260 265 270
   Glu Glu Asn Gly Ile Glu Phe Leu Lys Thr Pro Gly Ser Tyr Tyr Glu
   275 280 285
   Ser Leu Ser Ser Arg Ile Gly Ser Ile Asp Glu Asp Leu Asn Glu Ile
   290 295 300
   Glu Lys His Asn Ile Leu Val Asp Arg Asp Glu Asn Gly Tyr Leu Leu
   305 310 315 320
   Gln Ile Phe Thr Lys Pro Val Thr Asp Arg Pro Thr Phe Phe Phe Glu
                   325                 330                 335
   Val Ile Gln Arg Lys Gly Ala Arg Ser Phe Gly Asn Gly Asn Phe Lys
               340                 345                 350
   Ala Leu Phe Glu Ala Ile Glu Arg Glu Gln Ala Lys Arg Gly Asn Leu
           355                 360                 365
<210> 21
   <211> 1164
   <212> DNA
   <213> Kordia algicida
<400> 21
   atggcagcag aaataaaaaa cttaaaagat ttacaaaata cagaatacgg actcaaaaaa      60
   ttatttgacg aagcagaaga ctttcttcca cttttaggaa cagactacgt agaattatac     120
   gtcgggaacg ccaaacaatc ggcacatttc tacaaaacgg cttttggttt tcaatcagaa     180
   gcttacgcag gattggaaac aggattaacc gacagagttt catacgtatt aaaacaagat     240
   aaaattcgct tggtcttaac aacaccatta ggaaaaggtg gcgaaatcaa tgagcatatc     300
   gatttacacg gcgatggcgt aaaagtagta gcactttggg tagaagatgc tacaaaagcc     360
   tttgaagaaa cgaccaaaag aggcgcaaaa ccgtacatgg aaccaacaaa agaagaagat     420
   gaaaacggat atgtaattcg ctcaggaatc tatacgtacg gagaaacggt tcatgttttt     480
   gtagaacgta aaaactataa cggagtcttt ttaccaggat atcaaagatg ggaatctcac     540
   tacaatccgg agccagttgg cttaaaattc atcgatcaca tggtaggaaa tgtaggttgg     600
   ggagaaatga aagaatggtg tgaattctac gcgaaagtaa tgggatttgc gcaaattatc     660
   tcctttacag atgatgatat ttctaccgat tttactgcgt tgatgagtaa agtaatgagt     720
   aatggaaatg gtagaatcaa atttccaatc aatgaacccg cagaaggaaa aaagaaatcg     780
   caaattgaag aatatctaga cttttacaat ggttcaggag tacaacatat tgcggttgct     840
   acagacaata ttattgatac ggtttcgcaa atgcgcgaac gtggagtaga attcttatac     900
   gttccagata catattatga tgacttgtta gaacgtgttg gcgacatcga tgaagatgta     960
   gaagaactca aaaaacacgg aatcttaatt gatcgtgatg aagaaggata cttattgcag    1020
   ttatttacca aaaccattgt agacagacca acaatgttct ttgaagtcat tcagcgtaaa    1080
   ggcgcacaat catttggagt aggaaacttt aaagctttat ttgaagcgat agaaagagaa    1140
   caagctgctc gcggaacatt gtaa                                           1164
<210> 22
   <211> 387
   <212> PRT
   <213> Kordia algicida
<400> 22
   Met Ala Ala Glu Ile Lys Asn Leu Lys Asp Leu Gln Asn Thr Glu Tyr
   1               5                   10                  15
   Gly Leu Lys Lys Leu Phe Asp Glu Ala Glu Asp Phe Leu Pro Leu Leu
               20                  25                  30
   Gly Thr Asp Tyr Val Glu Leu Tyr Val Gly Asn Ala Lys Gln Ser Ala
           35                  40                  45
   His Phe Tyr Lys Thr Ala Phe Gly Phe Gln Ser Glu Ala Tyr Ala Gly
       50                  55                  60
   Leu Glu Thr Gly Leu Thr Asp Arg Val Ser Tyr Val Leu Lys Gln Asp
   65                  70                  75                  80
   Lys Ile Arg Leu Val Leu Thr Thr Pro Leu Gly Lys Gly Gly Glu Ile
                   85                  90                  95
   Asn Glu His Ile Asp Leu His Gly Asp Gly Val Lys Val Val Ala Leu
               100                 105                 110
   Trp Val Glu Asp Ala Thr Lys Ala Phe Glu Glu Thr Thr Lys Arg Gly
           115                 120                 125
   Ala Lys Pro Tyr Met Glu Pro Thr Lys Glu Glu Asp Glu Asn Gly Tyr
       130                 135                 140
   Val Ile Arg Ser Gly Ile Tyr Thr Tyr Gly Glu Thr Val His Val Phe
   145                 150                 155                 160
   Val Glu Arg Lys Asn Tyr Asn Gly Val Phe Leu Pro Gly Tyr Gln Arg
                   165                 170                 175
   Trp Glu Ser His Tyr Asn Pro Glu Pro Val Gly Leu Lys Phe Ile Asp
               180                 185                 190
   His Met Val Gly Asn Val Gly Trp Gly Glu Met Lys Glu Trp Cys Glu
           195                 200                 205
   Phe Tyr Ala Lys Val Met Gly Phe Ala Gln Ile Ile Ser Phe Thr Asp
       210                 215                 220
   Asp Asp Ile Ser Thr Asp Phe Thr Ala Leu Met Ser Lys Val Met Ser
   225                 230                 235                 240
   Asn Gly Asn Gly Arg Ile Lys Phe Pro Ile Asn Glu Pro Ala Glu Gly
                   245                 250                 255
   Lys Lys Lys Ser Gln Ile Glu Glu Tyr Leu Asp Phe Tyr Asn Gly Ser
               260                 265                 270
   Gly Val Gln His Ile Ala Val Ala Thr Asp Asn Ile Ile Asp Thr Val
           275                 280                 285
   Ser Gln Met Arg Glu Arg Gly Val Glu Phe Leu Tyr Val Pro Asp Thr
       290                 295                 300
   Tyr Tyr Asp Asp Leu Leu Glu Arg Val Gly Asp Ile Asp Glu Asp Val
   305                 310                 315                 320
   Glu Glu Leu Lys Lys His Gly Ile Leu Ile Asp Arg Asp Glu Glu Gly
                   325                 330                 335
   Tyr Leu Leu Gln Leu Phe Thr Lys Thr Ile Val Asp Arg Pro Thr Met
               340                 345                 350
   Phe Phe Glu Val Ile Gln Arg Lys Gly Ala Gln Ser Phe Gly Val Gly
           355                 360                 365
   Asn Phe Lys Ala Leu Phe Glu Ala Ile Glu Arg Glu Gln Ala Ala Arg
       370                 375                 380
   Gly Thr Leu
   385
<210> 23
   <211> 1323
   <212> DNA
   <213> Sorghum bicolor
<400> 23
   atgcccccga cccccaccac agccgccgca accggcgccg ccgtggcggc ggcatcagcg     60
   gagcaggcgg cgttccgcct cgtgggccac cgcaacttcg tccgcgtgaa cccgcgctcc    120
   gaccgcttcc acacgctcgc gttccaccac gtggagctct ggtgcgccga cgcggcctcc    180
   gccgcgggcc gcttctcctt cgggctcggc gcgccgctcg ccgcgcggtc cgacctctcc 240
   acggggaaca ccgcgcacgc gtccctgctg ctccgctcgg gcgccctcgc gttcctcttc 300
   acggcgccct acgcgcacgg cgccgacgcc gccacggcct cgctgccctc cttctccgcc 360
   gccgaggcgc ggcgcttcgc ggccgaccac ggcctcgcgg tgcgcgccgt cgcgctccgc 420
   gtggccgacg cggaggacgc cttccgcgcc agcgtcgcgg ccggcgcgcg cccggcgttc 480
   gagcccgtcg agctcggcct cggcttccgc ctcgccgaag tcgagctcta cggcgacgtc 540
   gtgctccggt acgtgagcta cccggacgac gcggacgcgt ccttcctgcc ggggttcgtg 600
   ggcgtgacca gccccggcgc ggcggactac gggctgagga ggttcgacca catcgtcggc 660
   aacgtgccgg agctggcgcc ggcggccgcc tacttcgctg gcttcacggg gttccacgag 720
   ttcgccgagt tcacggcgga ggacgtgggc accacggaga gcgggctcaa ctcgatggtg 780
   ctcgccaaca acgcggagaa cgtgctgctc ccactcaacg agccggtgca cggcaccaag 840
   cgccgcagcc agatacagac gtacttggac caccacggcg gccccggcgt gcagcacatg 900
   gcgctggcca gcgacgacgt gctcaggacg ctgagggaga tgcaagcgcg ctcggccatg 960
   ggcggcttcg agttcatggc gcctccggcg cccgaatact atgacggcgt gaggcggcgc 1020
   gccggggacg tgctcacgga ggcacagatt aaggagtgtc aggaactagg ggtgctggtg 1080
   gacagagatg accagggcgt gctgctccag atcttcacca agccagtggg ggacaggcca 1140
   acgttgttct tggagatcat tcaaaggatc gggtgcatgg agaaggatga gaaggggcaa 1200
   gaataccaga agggtggctg tggcgggttt ggcaagggaa acttctccca gctgttcaaa 1260
   tccattgagg attatgagaa gtcccttgaa gctaagcaag ctgcagcagc tcagggatcc 1320
   tag 1323
<210> 24
   <211> 440
   <212> PRT
   <213> Sorghum bicolor
<400> 24
   Met Pro Pro Thr Pro Thr Thr Ala Ala Ala Thr Gly Ala Ala Val Ala
   1 5 10 15
   Ala Ala Ser Ala Glu Gln Ala Ala Phe Arg Leu Val Gly His Arg Asn
   20 25 30
   Phe Val Arg Val Asn Pro Arg Ser Asp Arg Phe His Thr Leu Ala Phe
   35 40 45
   His His Val Glu Leu Trp Cys Ala Asp Ala Ala Ser Ala Ala Gly Arg
       50                  55                  60
   Phe Ser Phe Gly Leu Gly Ala Pro Leu Ala Ala Arg Ser Asp Leu Ser
   65                  70                  75                  80
   Thr Gly Asn Thr Ala His Ala Ser Leu Leu Leu Arg Ser Gly Ala Leu
                   85                  90                  95
   Ala Phe Leu Phe Thr Ala Pro Tyr Ala His Gly Ala Asp Ala Ala Thr
               100                 105                 110
   Ala Ser Leu Pro Ser Phe Ser Ala Ala Glu Ala Arg Arg Phe Ala Ala
           115                 120                 125
   Asp His Gly Leu Ala Val Arg Ala Val Ala Leu Arg Val Ala Asp Ala
       130                 135                 140
   Glu Asp Ala Phe Arg Ala Ser Val Ala Ala Gly Ala Arg Pro Ala Phe
   145                 150                 155                 160
   Glu Pro Val Glu Leu Gly Leu Gly Phe Arg Leu Ala Glu Val Glu Leu
                   165                 170                 175
   Tyr Gly Asp Val Val Leu Arg Tyr Val Ser Tyr Pro Asp Asp Ala Asp
               180                 185                 190
   Ala Ser Phe Leu Pro Gly Phe Val Gly Val Thr Ser Pro Gly Ala Ala
           195                 200                 205
   Asp Tyr Gly Leu Arg Arg Phe Asp His Ile Val Gly Asn Val Pro Glu
       210                 215                 220
   Leu Ala Pro Ala Ala Ala Tyr Phe Ala Gly Phe Thr Gly Phe His Glu
   225                 230                 235                 240
   Phe Ala Glu Phe Thr Ala Glu Asp Val Gly Thr Thr Glu Ser Gly Leu
                   245                 250                 255
   Asn Ser Met Val Leu Ala Asn Asn Ala Glu Asn Val Leu Leu Pro Leu
               260                 265                 270
   Asn Glu Pro Val His Gly Thr Lys Arg Arg Ser Gln Ile Gln Thr Tyr
   275 280 285
   Leu Asp His His Gly Gly Pro Gly Val Gln His Met Ala Leu Ala Ser
   290 295 300
   Asp Asp Val Leu Arg Thr Leu Arg Glu Met Gln Ala Arg Ser Ala Met
   305 310 315 320
   Gly Gly Phe Glu Phe Met Ala Pro Pro Ala Pro Glu Tyr Tyr Asp Gly
   325 330 335
   Val Arg Arg Arg Ala Gly Asp Val Leu Thr Glu Ala Gln Ile Lys Glu
   340 345 350
   Cys Gln Glu Leu Gly Val Leu Val Asp Arg Asp Asp Gln Gly Val Leu
   355 360 365
   Leu Gln Ile Phe Thr Lys Pro Val Gly Asp Arg Pro Thr Leu Phe Leu
   370 375 380
   Glu Ile Ile Gln Arg Ile Gly Cys Met Glu Lys Asp Glu Lys Gly Gln
   385 390 395 400
   Glu Tyr Gln Lys Gly Gly Cys Gly Gly Phe Gly Lys Gly Asn Phe Ser
   405 410 415
   Gln Leu Phe Lys Ser Ile Glu Asp Tyr Glu Lys Ser Leu Glu Ala Lys
   420 425 430
   Gln Ala Ala Ala Ala Gln Gly Ser
   435 440
<210> 25
   <211> 1311
   <212> DNA
   <213> Triticum aestivum
<400> 25
   atgccgccca cccccaccac ccccgcagcc accggcgccg gcgctgccgc cgcggtgacg 60
   ccggagcacg cgcggccgcg ccgaatggtc cgcttcaacc cgcgcagcga ccgcttccac 120
   acgctctcct tccaccacgt cgagttctgg tgcgcggacg ccgcctccgc cgccggccgc 180
   ttcgccttcg cgctcggcgc gccgctcgcc gccaggtccg acctctccac ggggaactcc 240
   gtgcacgcct cccagctgct ccgctcgggc aacctcgcct tcctcttcac cgcgccctac 300
   gcgaacggct gcgacgccgc caccgcctcc ctgccctcct tctccgccga cgccgcgcgc     360
   cggttctccg cggaccacgg gctcgcagtg cgctccatag cactgcgcgt cgcagacgcc     420
   gcagaggcct tccgcgccag cgtcgacgga ggcgcgcgcc cggccttcag ccccgtggac     480
   ctcggccgcg gcttcggctt cgcggaggtc gagctctacg gcgacgtcgt gctccgcttc     540
   gtcagtcacc cggatgacac ggacgtgccc ttcttgccgg ggttcgaggg cgtgagcaac     600
   ccggatgccg tggactacgg cctgacgcgg ttcgaccacg tcgtcggcaa cgtcccggag     660
   cttgcccccg ccgccgcata cgtcgccggg ttcgcggggt tccacgagtt cgccgagttc     720
   acgacggagg acgtgggcac ggccgagagc gggctcaact cgatggtgct cgccaacaac     780
   tcggagggcg tgctgctgcc gctcaacgag ccggtgcacg gcaccaagcg ccggagccag     840
   atacagacgt tcctggaaca ccacggcggc tcgggcgtgc agcacatcgc ggtggccagc     900
   agcgacgtgc tcaggacgct cagggagatg cgtgcgcgct ccgccatggg cggcttcgac     960
   ttcctgccac cccgctgccg aaagtactac gaaggcgtgc ggcgcatcgc cggggatgtg    1020
   ctctcggagg cgcagatcaa ggaatgccag gagctggggg tgctcgtcga cagggacgac    1080
   caaggggtgt tgctacaaat cttcacaaag ccagtggggg acaggccaac gctgttcctg    1140
   gagatgatcc aaaggatcgg gtgcatggag aaggacgaga gaggggaaga gtaccagaag    1200
   ggtggctgcg gcgggttcgg caaaggcaac ttctccgagc tgttcaagtc cattgaagat    1260
   tacgagaagt cccttgaagc caagcaatct gctgcagttc agggatcata g             1311
<210> 26
   <211> 436
   <212> PRT
   <213> Triticum aestivum
<400> 26
   Met Pro Pro Thr Pro Thr Thr Pro Ala Ala Thr Gly Ala Gly Ala Ala
   1               5                   10                  15
   Ala Ala Val Thr Pro Glu His Ala Arg Pro Arg Arg Met Val Arg Phe
               20                  25                  30
   Asn Pro Arg Ser Asp Arg Phe His Thr Leu Ser Phe His His Val Glu
           35                  40                  45
   Phe Trp Cys Ala Asp Ala Ala Ser Ala Ala Gly Arg Phe Ala Phe Ala
       50                  55                  60
   Leu Gly Ala Pro Leu Ala Ala Arg Ser Asp Leu Ser Thr Gly Asn Ser
   65                  70                  75                  80
   Val His Ala Ser Gln Leu Leu Arg Ser Gly Asn Leu Ala Phe Leu Phe
                   85                  90                  95
   Thr Ala Pro Tyr Ala Asn Gly Cys Asp Ala Ala Thr Ala Ser Leu Pro
               100                 105                 110
   Ser Phe Ser Ala Asp Ala Ala Arg Arg Phe Ser Ala Asp His Gly Leu
           115                 120                 125
   Ala Val Arg Ser Ile Ala Leu Arg Val Ala Asp Ala Ala Glu Ala Phe
       130                 135                 140
   Arg Ala Ser Val Asp Gly Gly Ala Arg Pro Ala Phe Ser Pro Val Asp
   145                 150                 155                 160
   Leu Gly Arg Gly Phe Gly Phe Ala Glu Val Glu Leu Tyr Gly Asp Val
                   165                 170                 175
   Val Leu Arg Phe Val Ser His Pro Asp Asp Thr Asp Val Pro Phe Leu
               180                 185                 190
   Pro Gly Phe Glu Gly Val Ser Asn Pro Asp Ala Val Asp Tyr Gly Leu
           195                 200                 205
   Thr Arg Phe Asp His Val Val Gly Asn Val Pro Glu Leu Ala Pro Ala
       210                 215                 220
   Ala Ala Tyr Val Ala Gly Phe Ala Gly Phe His Glu Phe Ala Glu Phe
   225                 230                 235                 240
   Thr Thr Glu Asp Val Gly Thr Ala Glu Ser Gly Leu Asn Ser Met Val
                   245                 250                 255
   Leu Ala Asn Asn Ser Glu Gly Val Leu Leu Pro Leu Asn Glu Pro Val
               260                 265                 270
   His Gly Thr Lys Arg Arg Ser Gln Ile Gln Thr Phe Leu Glu His His
           275                 280                 285
   Gly Gly Ser Gly Val Gln His Ile Ala Val Ala Ser Ser Asp Val Leu
       290                 295                 300
   Arg Thr Leu Arg Glu Met Arg Ala Arg Ser Ala Met Gly Gly Phe Asp
   305                 310                 315                 320
   Phe Leu Pro Pro Arg Cys Arg Lys Tyr Tyr Glu Gly Val Arg Arg Ile
                   325                 330                 335
   Ala Gly Asp Val Leu Ser Glu Ala Gln Ile Lys Glu Cys Gln Glu Leu
               340                 345                 350
   Gly Val Leu Val Asp Arg Asp Asp Gln Gly Val Leu Leu Gln Ile Phe
           355                 360                 365
   Thr Lys Pro Val Gly Asp Arg Pro Thr Leu Phe Leu Glu Met Ile Gln
       370                 375                 380
   Arg Ile Gly Cys Met Glu Lys Asp Glu Arg Gly Glu Glu Tyr Gln Lys
   385                 390                 395                 400
   Gly Gly Cys Gly Gly Phe Gly Lys Gly Asn Phe Ser Glu Leu Phe Lys
                   405                 410                 415
   Ser Ile Glu Asp Tyr Glu Lys Ser Leu Glu Ala Lys Gln Ser Ala Ala
               420                 425                 430
   Val Gln Gly Ser
           435
<210> 27
   <211> 1305
   <212> DNA
   <213> Hordeum vulgare
<400> 27
   atgccgccca cccccaccac ccccgcggct accggcgccg ccgccgcggt gacgccggag      60
   cacgcgcgac cgcaccgaat ggtccgcttc aacccgcgca gcgaccgctt ccacacgctc     120
   tccttccacc acgtcgagtt ctggtgcgcg gacgccgcct ccgccgccgg ccgcttcgcg     180
   ttcgcgctcg gcgcgccgct cgccgccagg tccgacctct ccacggggaa ctccgcgcac     240
   gcctcccagc tgctccgctc gggctccctc gccttcctct tcaccgcgcc ctacgccaac     300
   ggctgcgacg ccgccaccgc ctccctgccc tccttctccg ccgacgccgc gcgccggttc     360
   tccgccgacc acgggatcgc ggtgcgctcc gtagcgctgc gcgtcgcaga cgccgccgag     420
   gccttccgcg ccagtcgtcg acggggcgcg cgcccggcct tcgcccccgt ggacctcggc     480
   cgcggcttcg cgttcgcgga ggtcgagctc tacggcgacg tcgtgctccg cttcgtcagc     540
   cacccggacg gcacggacgt gcccttcttg ccggggttcg agggcgtaac caacccggac    600
   gccgtggact acggcctgac gcggttcgac cacgtcgtcg gcaacgtccc ggagcttgcc    660
   cccgccgcag cctacatcgc cgggttcacg gggttccacg agttcgccga gttcacggcg    720
   gaggacgtgg gcacgaccga gagcgggctc aactcggtgg tgctcgccaa caactcggag    780
   ggcgtgctgc tgccgctcaa cgagccggtg cacggcacca agcgccggag ccagatacag    840
   acgttcctgg aacaccacgg cggcccgggc gtgcagcaca tcgcggtggc cagcagtgac    900
   gtgctcagga cgctcaggaa gatgcgtgcg cgctccgcca tgggcggctt cgacttcctg    960
   ccacccccgc tgccgaagta ctacgaaggc gtgcgacgcc ttgccgggga tgtcctctcg   1020
   gaggcgcaga tcaaggaatg ccaggagctg ggtgtgctcg tcgataggga cgaccaaggg   1080
   gtgttgctcc aaatcttcac caagccagta ggggacaggc cgaccttgtt cctggagatg   1140
   atccagagga tcgggtgcat ggagaaggac gagagagggg aagagtacca gaagggtggc   1200
   tgcggcgggt tcggcaaagg caacttctcc gagctgttca agtccattga agattacgag   1260
   aagtcccttg aagccaagca atctgctgca gttcagggat catag                   1305
<210> 28
   <211> 434
   <212> PRT
   <213> Hordeum vulgare
<400> 28
   Met Pro Pro Thr Pro Thr Thr Pro Ala Ala Thr Gly Ala Ala Ala Ala
   1               5                   10                  15
   Val Thr Pro Glu His Ala Arg Pro His Arg Met Val Arg Phe Asn Pro
               20                  25                  30
   Arg Ser Asp Arg Phe His Thr Leu Ser Phe His His Val Glu Phe Trp
           35                  40                  45
   Cys Ala Asp Ala Ala Ser Ala Ala Gly Arg Phe Ala Phe Ala Leu Gly
       50                  55                  60
   Ala Pro Leu Ala Ala Arg Ser Asp Leu Ser Thr Gly Asn Ser Ala His
   65                  70                  75                  80
   Ala Ser Gln Leu Leu Arg Ser Gly Ser Leu Ala Phe Leu Phe Thr Ala
                   85                  90                  95
   Pro Tyr Ala Asn Gly Cys Asp Ala Ala Thr Ala Ser Leu Pro Ser Phe
               100                 105                 110
   Ser Ala Asp Ala Ala Arg Arg Phe Ser Ala Asp His Gly Ile Ala Val
           115                 120                 125
   Arg Ser Val Ala Leu Arg Val Ala Asp Ala Ala Glu Ala Phe Arg Ala
       130                 135                 140
   Ser Arg Arg Arg Gly Ala Arg Pro Ala Phe Ala Pro Val Asp Leu Gly
   145                 150                 155                 160
   Arg Gly Phe Ala Phe Ala Glu Val Glu Leu Tyr Gly Asp Val Val Leu
                   165                 170                 175
   Arg Phe Val Ser His Pro Asp Gly Thr Asp Val Pro Phe Leu Pro Gly
               180                 185                 190
   Phe Glu Gly Val Thr Asn Pro Asp Ala Val Asp Tyr Gly Leu Thr Arg
           195                 200                 205
   Phe Asp His Val Val Gly Asn Val Pro Glu Leu Ala Pro Ala Ala Ala
       210                 215                 220
   Tyr Ile Ala Gly Phe Thr Gly Phe His Glu Phe Ala Glu Phe Thr Ala
   225                 230                 235                 240
   Glu Asp Val Gly Thr Thr Glu Ser Gly Leu Asn Ser Val Val Leu Ala
                   245                 250                 255
   Asn Asn Ser Glu Gly Val Leu Leu Pro Leu Asn Glu Pro Val His Gly
               260                 265                 270
   Thr Lys Arg Arg Ser Gln Ile Gln Thr Phe Leu Glu His His Gly Gly
           275                 280                 285
   Pro Gly Val Gln His Ile Ala Val Ala Ser Ser Asp Val Leu Arg Thr
       290                 295                 300
   Leu Arg Lys Met Arg Ala Arg Ser Ala Met Gly Gly Phe Asp Phe Leu
   305                 310                 315                 320
   Pro Pro Pro Leu Pro Lys Tyr Tyr Glu Gly Val Arg Arg Leu Ala Gly
                   325                 330                 335
   Asp Val Leu Ser Glu Ala Gln Ile Lys Glu Cys Gln Glu Leu Gly Val
               340                 345                 350
   Leu Val Asp Arg Asp Asp Gln Gly Val Leu Leu Gln Ile Phe Thr Lys
           355                 360                 365
   Pro Val Gly Asp Arg Pro Thr Leu Phe Leu Glu Met Ile Gln Arg Ile
       370                 375                 380
   Gly Cys Met Glu Lys Asp Glu Arg Gly Glu Glu Tyr Gln Lys Gly Gly
   385                 390                 395                 400
   Cys Gly Gly Phe Gly Lys Gly Asn Phe Ser Glu Leu Phe Lys Ser Ile
                   405                 410                 415
   Glu Asp Tyr Glu Lys Ser Leu Glu Ala Lys Gln Ser Ala Ala Val Gln
               420                 425                 430
   Gly Ser
<210> 29
   <211> 1209
   <212> DNA
   <213> Rhodococcus sp.
<400> 29
   atgactaccg ccgacattcg cctgacgccc cgcgaggtgg ccgcacatct ggagaccgac     60
   gagctccggc agttggtcgg gctcgtcgaa cacgacgacg cgtcggatcc gtttcccgtg    120
   gtcgcgatgg atgccgtggt gttcgtgtgc ggcaacgcga cgcagagcac gcagtacttc    180
   gtctccacgt ggggcatgac cctcgtcgcc tacgccgggc cggagaccgg tcagcgctcg    240
   cacaagtcct tcgtcctcga gtcggggtcg gcacggttcg tgctgcacgg cgccgtcgat    300
   ccgaagagcc cgctcgcgga ccatcaccgg gcgcacggcg acggcgtggt ggacctggcg    360
   atggaagttc tcgacgtcga ccgctgcatc gcgcatgcac gctcgcaggg ggccaccatt    420
   ctcgaggagc cgcgcgacgt cacggatcag ttcggcaccg tgcggctcgc ggcgatcgcc    480
   acgtacggca gcacccggca caccatcgtc gaccgaagcc gatacgacgg cccctacctc    540
   cccggattcg tcgcgcgctc cagcggtttc gcggcgcgac cgggtaaacc cccgcgattg    600
   ttccaggcgc tcgaccacgc cgtcggcaac gtcgagatgg gccggatgga tcactgggtc    660
   cggttctaca accgcgtcat gggcttcacg aacatggccg aattcgtcgg cgacgacatc    720
   gccacggagt actcggcgct gatgtcgaag gtcgtggcga acggcaatca ccgggtgaag     780
   ttcccgctca acgaacccgc ggtgggaaag aagaagtcgc agatcgacga atatctcgag     840
   ttctacggtg agccgggctg ccagcatctg gccctcgcga ccggagacat cctcgcgacg     900
   gtggacgcgt tgcgggccga gggtgtcgaa ttcctgaaca cacccgacgc gtactacgag     960
   gacccacagc tgcgcgcccg gatcggcagg gtgcgggtgc cggtggagga actgcagaag    1020
   cgcggaatcc tcgtcgaccg cgacgaggac ggatacctcc tgcagatctt caccaaaccg    1080
   ctcggcgacc ggccgaccgt gttcttcgag gtgatcgaac ggcacggttc gctcgggttc    1140
   ggggcgggta acttccaggc cctgttcgaa tccatcgagc gtgagcaggc ggcgcgcggc    1200
   aatctgtga                                                            1209
<210> 30
   <211> 402
   <212> PRT
   <213> Rhodococcus sp.
<400> 30
   Met Thr Thr Ala Asp Ile Arg Leu Thr Pro Arg Glu Val Ala Ala His
   1               5                   10                  15
   Leu Glu Thr Asp Glu Leu Arg Gln Leu Val Gly Leu Val Glu His Asp
               20                  25                  30
   Asp Ala Ser Asp Pro Phe Pro Val Val Ala Met Asp Ala Val Val Phe
           35                  40                  45
   Val Cys Gly Asn Ala Thr Gln Ser Thr Gln Tyr Phe Val Ser Thr Trp
       50                  55                  60
   Gly Met Thr Leu Val Ala Tyr Ala Gly Pro Glu Thr Gly Gln Arg Ser
   65                  70                  75                  80
   His Lys Ser Phe Val Leu Glu Ser Gly Ser Ala Arg Phe Val Leu His
                   85                  90                  95
   Gly Ala Val Asp Pro Lys Ser Pro Leu Ala Asp His His Arg Ala His
               100                 105                 110
   Gly Asp Gly Val Val Asp Leu Ala Met Glu Val Leu Asp Val Asp Arg
           115                 120                 125
   Cys Ile Ala His Ala Arg Ser Gln Gly Ala Thr Ile Leu Glu Glu Pro
       130                 135                 140
   Arg Asp Val Thr Asp Gln Phe Gly Thr Val Arg Leu Ala Ala Ile Ala
   145                 150                 155                 160
   Thr Tyr Gly Ser Thr Arg His Thr Ile Val Asp Arg Ser Arg Tyr Asp
                   165                 170                 175
   Gly Pro Tyr Leu Pro Gly Phe Val Ala Arg Ser Ser Gly Phe Ala Ala
               180                 185                 190
   Arg Pro Gly Lys Pro Pro Arg Leu Phe Gln Ala Leu Asp His Ala Val
           195                 200                 205
   Gly Asn Val Glu Met Gly Arg Met Asp His Trp Val Arg Phe Tyr Asn
       210                 215                 220
   Arg Val Met Gly Phe Thr Asn Met Ala Glu Phe Val Gly Asp Asp Ile
   225                 230                 235                 240
   Ala Thr Glu Tyr Ser Ala Leu Met Ser Lys Val Val Ala Asn Gly Asn
                   245                 250                 255
   His Arg Val Lys Phe Pro Leu Asn Glu Pro Ala Val Gly Lys Lys Lys
               260                 265                 270
   Ser Gln Ile Asp Glu Tyr Leu Glu Phe Tyr Gly Glu Pro Gly Cys Gln
           275                 280                 285
   His Leu Ala Leu Ala Thr Gly Asp Ile Leu Ala Thr Val Asp Ala Leu
       290                 295                 300
   Arg Ala Glu Gly Val Glu Phe Leu Asn Thr Pro Asp Ala Tyr Tyr Glu
   305                 310                 315                 320
   Asp Pro Gln Leu Arg Ala Arg Ile Gly Arg Val Arg Val Pro Val Glu
                   325                 330                 335
   Glu Leu Gln Lys Arg Gly Ile Leu Val Asp Arg Asp Glu Asp Gly Tyr
               340                 345                 350
   Leu Leu Gln Ile Phe Thr Lys Pro Leu Gly Asp Arg Pro Thr Val Phe
           355                 360                 365
   Phe Glu Val Ile Glu Arg His Gly Ser Leu Gly Phe Gly Ala Gly Asn
       370                 375                 380
   Phe Gln Ala Leu Phe Glu Ser Ile Glu Arg Glu Gln Ala Ala Arg Gly
   385                 390                 395                 400
   Asn Leu
<210> 31
   <211> 1353
   <212> DNA
   <213> Artificial sequence
<220>
   <223> Nucleic acid sequence encoding Arabidopsis HPPD plus containing at the 5' end a nucleic acid encoding an Ala and 6 His amino acids
<220>
   <221> misc_feature
   <222> (4)..(6)
   <223> sequence coding for an Ala
<220>
   <221> misc_feature
   <222> (7)..(24)
   <223> sequence coding for an His Tag containing 6 His
<400> 31
   atggctcatc accatcacca tcaccaaaac gccgccgttt cagagaatca aaaccatgat     60
   gacggcgctg cgtcgtcgcc gggattcaag ctcgtcggat tttccaagtt cgtaagaaag    120
   aatccaaagt ctgataaatt caaggttaag cgcttccatc acatcgagtt ctggtgcggc    180
   gacgcaacca acgtcgctcg tcgcttctcc tggggtctgg ggatgagatt ctccgccaaa    240
   tccgatcttt ccaccggaaa catggttcac gcctcttacc tactcacctc cggtgacctc    300
   cgattccttt tcactgctcc ttactctccg tctctctccg ccggagagat taaaccgaca    360
   accacagctt ctatcccaag tttcgatcac ggctcttgtc gttccttctt ctcgtcacat    420
   ggtctcggtg ttagagccgt tgcgattgaa gtagaagacg cagagtcagc tttctccatc    480
   agtgtagcta atggcgctat tccttcgtcg cctcctatcg tcctcaatga agcagttacg    540
   atcgctgagg ttaaactata cggcgatgtt gttctccgat atgttagtta caaagcagaa    600
   gataccgaaa aatccgaatt cttgccaggg ttcgagcgtg tagaggatgc gtcgtcgttc    660
   ccattggatt atggtatccg gcggcttgac cacgccgtgg gaaacgttcc tgagcttggt    720
   ccggctttaa cttatgtagc ggggttcact ggttttcacc aattcgcaga gttcacagca    780
   gacgacgttg gaaccgccga gagcggttta aattcagcgg tcctggctag caatgatgaa     840
   atggttcttc taccgattaa cgagccagtg cacggaacaa agaggaagag tcagattcag     900
   acgtatttgg aacataacga aggcgcaggg ctacaacatc tggctctgat gagtgaagac     960
   atattcagga ccctgagaga gatgaggaag aggagcagta ttggaggatt cgacttcatg    1020
   ccttctcctc cgcctactta ctaccagaat ctcaagaaac gggtcggcga cgtgctcagc    1080
   gatgatcaga tcaaggagtg tgaggaatta gggattcttg tagacagaga tgatcaaggg    1140
   acgttgcttc aaatcttcac aaaaccacta ggtgacaggc cgacgatatt tatagagata    1200
   atccagagag taggatgcat gatgaaagat gaggaaggga aggcttacca gagtggagga    1260
   tgtggtggtt ttggcaaagg caatttctct gagctcttca agtccattga agaatacgaa    1320
   aagactcttg aagccaaaca gttagtggga tga                                 1353
<210> 32
   <211> 450
   <212> PRT
   <213> Artificial sequence
<220>
   <223> encoded by SEQ ID No. 31
<220>
   <221> MISC_FEATURE
   <222> (2)..(2)
   <223> Ala
<220>
   <221> MISC_FEATURE
   <222> (3)..(8)
   <223> His Tag made of 6 His
<400> 32
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
                   85                  90                  95
   Ser Gly Asp Leu Arg Phe Leu Phe Thr Ala Pro Tyr Ser Pro Ser Leu
               100                 105                 110
   Ser Ala Gly Glu Ile Lys Pro Thr Thr Thr Ala Ser Ile Pro Ser Phe
           115                 120                 125
   Asp His Gly Ser Cys Arg Ser Phe Phe Ser Ser His Gly Leu Gly Val
       130                 135                 140
   Arg Ala Val Ala Ile Glu Val Glu Asp Ala Glu Ser Ala Phe Ser Ile
   145                 150                 155                 160
   Ser Val Ala Asn Gly Ala Ile Pro Ser Ser Pro Pro Ile Val Leu Asn
                   165                 170                 175
   Glu Ala Val Thr Ile Ala Glu Val Lys Leu Tyr Gly Asp Val Val Leu
               180                 185                 190
   Arg Tyr Val Ser Tyr Lys Ala Glu Asp Thr Glu Lys Ser Glu Phe Leu
           195                 200                 205
   Pro Gly Phe Glu Arg Val Glu Asp Ala Ser Ser Phe Pro Leu Asp Tyr
       210                 215                 220
   Gly Ile Arg Arg Leu Asp His Ala Val Gly Asn Val Pro Glu Leu Gly
   225                 230                 235                 240
   Pro Ala Leu Thr Tyr Val Ala Gly Phe Thr Gly Phe His Gln Phe Ala
                   245                 250                 255
   Glu Phe Thr Ala Asp Asp Val Gly Thr Ala Glu Ser Gly Leu Asn Ser
               260                 265                 270
   Ala Val Leu Ala Ser Asn Asp Glu Met Val Leu Leu Pro Ile Asn Glu
           275                 280                 285
   Pro Val His Gly Thr Lys Arg Lys Ser Gln Ile Gln Thr Tyr Leu Glu
       290                 295                 300
   His Asn Glu Gly Ala Gly Leu Gln His Leu Ala Leu Met Ser Glu Asp
   305                 310                 315                 320
   Ile Phe Arg Thr Leu Arg Glu Met Arg Lys Arg Ser Ser Ile Gly Gly
                   325                 330                 335
   Phe Asp Phe Met Pro Ser Pro Pro Pro Thr Tyr Tyr Gln Asn Leu Lys
               340                 345                 350
   Lys Arg Val Gly Asp Val Leu Ser Asp Asp Gln Ile Lys Glu Cys Glu
           355                 360                 365
   Glu Leu Gly Ile Leu Val Asp Arg Asp Asp Gln Gly Thr Leu Leu Gln
       370                 375                 380
   Ile Phe Thr Lys Pro Leu Gly Asp Arg Pro Thr Ile Phe Ile Glu Ile
   385                 390                 395                 400
   Ile Gln Arg Val Gly Cys Met Met Lys Asp Glu Glu Gly Lys Ala Tyr
                   405                 410                 415
   Gln Ser Gly Gly Cys Gly Gly Phe Gly Lys Gly Asn Phe Ser Glu Leu
               420                 425                 430
   Phe Lys Ser Ile Glu Glu Tyr Glu Lys Thr Leu Glu Ala Lys Gln Leu
           435                 440                 445
   Val Gly
       450
<210> 33
   <211> 1353
   <212> DNA
   <213> Artificial sequence
<220>
   <223> nucleotide sequence encoding mutant HPPD polypeptide
<220>
   <221> misc_feature
   <222> (4)..(6)
   <223> sequence coding for Ala
<220>
   <221> misc_feature
   <222> (7)..(24)
   <223> sequence coding an His Tag containing 6 His
<400> 33
   atggctcatc accatcacca tcaccaaaac gccgccgttt cagagaatca aaaccatgat      60
   gacggcgctg cgtcgtcgcc gggattcaag ctcgtcggat tttccaagtt cgtaagaaag     120
   aatccaaagt ctgataaatt caaggttaag cgcttccatc acatcgagtt ctggtgcggc     180
   gacgcaacca acgtcgctcg tcgcttctcc tggggtctgg ggatgagatt ctccgccaaa     240
   tccgatcttt ccaccggaaa catggttcac gcctcttacc tactcacctc cggtgacctc     300
   cgattccttt tcactgctcc ttactctccg tctctctccg ccggagagat taaaccgaca     360
   accacagctt ctatcccaag tttcgatcac ggctcttgtc gttccttctt ctcgtcacat     420
   ggtctcggtg ttagagccgt tgcgattgaa gtagaagacg cagagtcagc tttctccatc     480
   agtgtagcta atggcgctat tccttcgtcg cctcctatcg tcctcaatga agcagttacg     540
   atcgctgagg ttaaactata cggcgatgtt gttctccgat atgttagtta caaagcagaa     600
   gataccgaaa aatccgaatt cttgccaggg ttcgagcgtg tagaggatgc gtcgtcgttc     660
   ccattggatt atggtatccg gcggcttgac cacgccgtgg gaaacgttcc tgagcttggt     720
   ccggctttaa cttatgtagc ggggttcact ggttttcacc aattcgcaga gttcacagca     780
   gacgacgttg gaaccgccga gagcggttta aatgcggcgg tcctggctag caatgatgaa     840
   atggttcttc taccgattaa cgagccagtg cacggaacaa agaggaagag tcagattcag     900
   acgtatttgg aacataacga aggcgcaggg ctacaacatc tggctctgat gagtgaagac     960
   atattcagga ccctgagaga gatgaggaag aggagcagta ttggaggatt cgacttcatg    1020
   ccttctcctc cgcctactta ctaccagaat ctcaagaaac gggtcggcga cgtgctcagc    1080
   gatgatcaga tcaaggagtg tgaggaatta gggattcttg tagacagaga tgatcaaggg    1140
   acgttgcttc aaatcttcac aaaaccacta ggtgacaggc cgacgatatt tatagagata    1200
   atccagagag taggatgcat gatgaaagat gaggaaggga aggcttacca gagtggagga    1260
   tgtggtggtt ttggcaaagg caatttctct gagctcttca agtccattga agaatacgaa    1320
   aagactcttg aagccaaaca gttagtggga tga                                 1353
<210> 34
   <211> 450
   <212> PRT
   <213> Artificial sequence
<220>
   <223> encoded by SEQ ID No. 33
<220>
   <221> MISC_FEATURE
   <222> (2)..(2)
   <223> Ala
<220>
   <221> MISC_FEATURE
   <222> (3)..(8)
   <223> His Tag made of 6 His
<400> 34
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
                   85                  90                  95
   Ser Gly Asp Leu Arg Phe Leu Phe Thr Ala Pro Tyr Ser Pro Ser Leu
               100                 105                 110
   Ser Ala Gly Glu Ile Lys Pro Thr Thr Thr Ala Ser Ile Pro Ser Phe
           115                 120                 125
   Asp His Gly Ser Cys Arg Ser Phe Phe Ser Ser His Gly Leu Gly Val
       130                 135                 140
   Arg Ala Val Ala Ile Glu Val Glu Asp Ala Glu Ser Ala Phe Ser Ile
   145                 150                 155                 160
   Ser Val Ala Asn Gly Ala Ile Pro Ser Ser Pro Pro Ile Val Leu Asn
                   165                 170                 175
   Glu Ala Val Thr Ile Ala Glu Val Lys Leu Tyr Gly Asp Val Val Leu
               180                 185                 190
   Arg Tyr Val Ser Tyr Lys Ala Glu Asp Thr Glu Lys Ser Glu Phe Leu
           195                 200                 205
   Pro Gly Phe Glu Arg Val Glu Asp Ala Ser Ser Phe Pro Leu Asp Tyr
       210                 215                 220
   Gly Ile Arg Arg Leu Asp His Ala Val Gly Asn Val Pro Glu Leu Gly
   225                 230                 235                 240
   Pro Ala Leu Thr Tyr Val Ala Gly Phe Thr Gly Phe His Gln Phe Ala
                   245                 250                 255
   Glu Phe Thr Ala Asp Asp Val Gly Thr Ala Glu Ser Gly Leu Asn Ala
               260                 265                 270
   Ala Val Leu Ala Ser Asn Asp Glu Met Val Leu Leu Pro Ile Asn Glu
           275                 280                 285
   Pro Val His Gly Thr Lys Arg Lys Ser Gln Ile Gln Thr Tyr Leu Glu
       290                 295                 300
   His Asn Glu Gly Ala Gly Leu Gln His Leu Ala Leu Met Ser Glu Asp
   305                 310                 315                 320
   Ile Phe Arg Thr Leu Arg Glu Met Arg Lys Arg Ser Ser Ile Gly Gly
                   325                 330                 335
   Phe Asp Phe Met Pro Ser Pro Pro Pro Thr Tyr Tyr Gln Asn Leu Lys
               340                 345                 350
   Lys Arg Val Gly Asp Val Leu Ser Asp Asp Gln Ile Lys Glu Cys Glu
           355                 360                 365
   Glu Leu Gly Ile Leu Val Asp Arg Asp Asp Gln Gly Thr Leu Leu Gln
       370                 375                 380
   Ile Phe Thr Lys Pro Leu Gly Asp Arg Pro Thr Ile Phe Ile Glu Ile
   385                 390                 395                 400
   Ile Gln Arg Val Gly Cys Met Met Lys Asp Glu Glu Gly Lys Ala Tyr
                   405                 410                 415
   Gln Ser Gly Gly Cys Gly Gly Phe Gly Lys Gly Asn Phe Ser Glu Leu
               420                 425                 430
   Phe Lys Ser Ile Glu Glu Tyr Glu Lys Thr Leu Glu Ala Lys Gln Leu
           435                 440                 445
   Val Gly
       450
<210> 35
   <211> 1353
   <212> DNA
   <213> Artificial sequence
<220>
   <223> nucleotide sequence encoding mutant HPPD polypeptide
<220>
   <221> misc_feature
   <222> (4)..(6)
   <223> sequence coding for Ala
<220>
   <221> misc_feature
   <222> (7)..(24)
   <223> sequence coding for an His Tag containing 6 His
<400> 35
   atggctcatc accatcacca tcaccaaaac gccgccgttt cagagaatca aaaccatgat     60
   gacggcgctg cgtcgtcgcc gggattcaag ctcgtcggat tttccaagtt cgtaagaaag    120
   aatccaaagt ctgataaatt caaggttaag cgcttccatc acatcgagtt ctggtgcggc    180
   gacgcaacca acgtcgctcg tcgcttctcc tggggtctgg ggatgagatt ctccgccaaa    240
   tccgatcttt ccaccggaaa catggttcac gcctcttacc tactcacctc cggtgacctc    300
   cgattccttt tcactgctcc ttactctccg tctctctccg ccggagagat taaaccgaca    360
   accacagctt ctatcccaag tttcgatcac ggctcttgtc gttccttctt ctcgtcacat    420
   ggtctcggtg ttagagccgt tgcgattgaa gtagaagacg cagagtcagc tttctccatc    480
   agtgtagcta atggcgctat tccttcgtcg cctcctatcg tcctcaatga agcagttacg    540
   atcgctgagg ttaaactata cggcgatgtt gttctccgat atgttagtta caaagcagaa    600
   gataccgaaa aatccgaatt cttgccaggg ttcgagcgtg tagaggatgc gtcgtcgttc    660
   ccattggatt atggtatccg gcggcttgac cacgccgtgg gaaacgttcc tgagcttggt    720
   ccggctttaa cttatgtagc ggggttcact ggttttcacc aattcgcaga gttcacagca    780
   gacgacgttg gaaccgccga gagcggttta aattcagcgg tcctggctag caatgatgaa    840
   atggttcttc taccgattgc cgagccagtg cacggaacaa agaggaagag tcagattcag    900
   acgtatttgg aacataacga aggcgcaggg ctacaacatc tggctctgat gagtgaagac    960
   atattcagga ccctgagaga gatgaggaag aggagcagta ttggaggatt cgacttcatg   1020
   ccttctcctc cgcctactta ctaccagaat ctcaagaaac gggtcggcga cgtgctcagc   1080
   gatgatcaga tcaaggagtg tgaggaatta gggattcttg tagacagaga tgatcaaggg   1140
   acgttgcttc aaatcttcac aaaaccacta ggtgacaggc cgacgatatt tatagagata   1200
   atccagagag taggatgcat gatgaaagat gaggaaggga aggcttacca gagtggagga   1260
   tgtggtggtt ttggcaaagg caatttctct gagctcttca agtccattga agaatacgaa   1320
   aagactcttg aagccaaaca gttagtggga tga                                1353
<210> 36
   <211> 450
   <212> PRT
   <213> Artificial sequence
<220>
   <223> encoded by SEQ ID No. 35
<220>
   <221> MISC_FEATURE
   <222> (2)..(2)
   <223> Ala
<220>
   <221> MISC_FEATURE
   <222> (3)..(8)
   <223> His Tag made of 6 His
<400> 36
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
   85 90 95
   Ser Gly Asp Leu Arg Phe Leu Phe Thr Ala Pro Tyr Ser Pro Ser Leu
   100 105 110
   Ser Ala Gly Glu Ile Lys Pro Thr Thr Thr Ala Ser Ile Pro Ser Phe
   115 120 125
   Asp His Gly Ser Cys Arg Ser Phe Phe Ser Ser His Gly Leu Gly Val
   130 135 140
   Arg Ala Val Ala Ile Glu Val Glu Asp Ala Glu Ser Ala Phe Ser Ile
   145 150 155 160
   Ser Val Ala Asn Gly Ala Ile Pro Ser Ser Pro Pro Ile Val Leu Asn
   165 170 175
   Glu Ala Val Thr Ile Ala Glu Val Lys Leu Tyr Gly Asp Val Val Leu
   180 185 190
   Arg Tyr Val Ser Tyr Lys Ala Glu Asp Thr Glu Lys Ser Glu Phe Leu
   195 200 205
   Pro Gly Phe Glu Arg Val Glu Asp Ala Ser Ser Phe Pro Leu Asp Tyr
   210 215 220
   Gly Ile Arg Arg Leu Asp His Ala Val Gly Asn Val Pro Glu Leu Gly
   225 230 235 240
   Pro Ala Leu Thr Tyr Val Ala Gly Phe Thr Gly Phe His Gln Phe Ala
   245 250 255
   Glu Phe Thr Ala Asp Asp Val Gly Thr Ala Glu Ser Gly Leu Asn Ser
   260 265 270
   Ala Val Leu Ala Ser Asn Asp Glu Met Val Leu Leu Pro Ile Ala Glu
   275 280 285
   Pro Val His Gly Thr Lys Arg Lys Ser Gln Ile Gln Thr Tyr Leu Glu
   290 295 300
   His Asn Glu Gly Ala Gly Leu Gln His Leu Ala Leu Met Ser Glu Asp
   305 310 315 320
   Ile Phe Arg Thr Leu Arg Glu Met Arg Lys Arg Ser Ser Ile Gly Gly
                   325                 330                 335
   Phe Asp Phe Met Pro Ser Pro Pro Pro Thr Tyr Tyr Gln Asn Leu Lys
               340                 345                 350
   Lys Arg Val Gly Asp Val Leu Ser Asp Asp Gln Ile Lys Glu Cys Glu
           355                 360                 365
   Glu Leu Gly Ile Leu Val Asp Arg Asp Asp Gln Gly Thr Leu Leu Gln
       370                 375                 380
   Ile Phe Thr Lys Pro Leu Gly Asp Arg Pro Thr Ile Phe Ile Glu Ile
   385                 390                 395                 400
   Ile Gln Arg Val Gly Cys Met Met Lys Asp Glu Glu Gly Lys Ala Tyr
                   405                 410                 415
   Gln Ser Gly Gly Cys Gly Gly Phe Gly Lys Gly Asn Phe Ser Glu Leu
               420                 425                 430
   Phe Lys Ser Ile Glu Glu Tyr Glu Lys Thr Leu Glu Ala Lys Gln Leu
           435                 440                 445
   Val Gly
       450
<210> 37
   <211> 1353
   <212> DNA
   <213> Artificial sequence
<220>
   <223> nucleotide sequence encoding mutant HPPD polypeptide
<220>
   <221> misc_feature
   <222> (4)..(6)
   <223> sequence coding for Ala
<220>
   <221> misc_feature
   <222> (7)..(24)
   <223> sequence coding for an His Tag containing 6 His
<400> 37
   atggctcatc accatcacca tcaccaaaac gccgccgttt cagagaatca aaaccatgat     60
   gacggcgctg cgtcgtcgcc gggattcaag ctcgtcggat tttccaagtt cgtaagaaag    120
   aatccaaagt ctgataaatt caaggttaag cgcttccatc acatcgagtt ctggtgcggc 180
   gacgcaacca acgtcgctcg tcgcttctcc tggggtctgg ggatgagatt ctccgccaaa 240
   tccgatcttt ccaccggaaa catggttcac gcctcttacc tactcacctc cggtgacctc 300
   cgattccttt tcactgctcc ttactctccg tctctctccg ccggagagat taaaccgaca 360
   accacagctt ctatcccaag tttcgatcac ggctcttgtc gttccttctt ctcgtcacat 420
   ggtctcggtg ttagagccgt tgcgattgaa gtagaagacg cagagtcagc tttctccatc 480
   agtgtagcta atggcgctat tccttcgtcg cctcctatcg tcctcaatga agcagttacg 540
   atcgctgagg ttaaactata cggcgatgtt gttctccgat atgttagtta caaagcagaa 600
   gataccgaaa aatccgaatt cttgccaggg ttcgagcgtg tagaggatgc gtcgtcgttc 660
   ccattggatt atggtatccg gcggcttgac cacgccgtgg gaaacgttcc tgagcttggt 720
   ccggctttaa cttatgtagc ggggttcact ggttttcacc aattcgcatc tttcacagca 780
   gacgacgttg gaaccgccga gagcggttta aattcagcgg tcctggctag caatgatgaa 840
   atggttcttc taccgattaa cgagccagtg cacggaacaa agaggaagag tcagattcag 900
   acgtatttgg aacataacga aggcgcaggg ctacaacatc tggctctgat gagtgaagac 960
   atattcagga ccctgagaga gatgaggaag aggagcagta ttggaggatt cgacttcatg 1020
   ccttctcctc cgcctactta ctaccagaat ctcaagaaac gggtcggcga cgtgctcagc 1080
   gatgatcaga tcaaggagtg tgaggaatta gggattcttg tagacagaga tgatcaaggg 1140
   acgttgcttc aaatcttcac aaaaccacta ggtgacaggc cgacgatatt tatagagata 1200
   atccagagag taggatgcat gatgaaagat gaggaaggga aggcttacca gagtggagga 1260
   tgtggtggtt ttggcaaagg caatttctct gagctcttca agtccattga agaatacgaa 1320
   aagactcttg aagccaaaca gttagtggga tga 1353
<210> 38
   <211> 450
   <212> PRT
   <213> Artificial sequence
<220>
   <223> encoded by SEQ ID No. 37
<220>
   <221> MISC_FEATURE
   <222> (2)..(2)
   <223> Ala
<220>
   <221> MISC_FEATURE
   <222> (3)..(8)
   <223> His Tag made of 6 His
<400> 38
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
                   85                  90                  95
   Ser Gly Asp Leu Arg Phe Leu Phe Thr Ala Pro Tyr Ser Pro Ser Leu
               100                 105                 110
   Ser Ala Gly Glu Ile Lys Pro Thr Thr Thr Ala Ser Ile Pro Ser Phe
           115                 120                 125
   Asp His Gly Ser Cys Arg Ser Phe Phe Ser Ser His Gly Leu Gly Val
       130                 135                 140
   Arg Ala Val Ala Ile Glu Val Glu Asp Ala Glu Ser Ala Phe Ser Ile
   145                 150                 155                 160
   Ser Val Ala Asn Gly Ala Ile Pro Ser Ser Pro Pro Ile Val Leu Asn
                   165                 170                 175
   Glu Ala Val Thr Ile Ala Glu Val Lys Leu Tyr Gly Asp Val Val Leu
               180                 185                 190
   Arg Tyr Val Ser Tyr Lys Ala Glu Asp Thr Glu Lys Ser Glu Phe Leu
           195                 200                 205
   Pro Gly Phe Glu Arg Val Glu Asp Ala Ser Ser Phe Pro Leu Asp Tyr
   210 215 220
   Gly Ile Arg Arg Leu Asp His Ala Val Gly Asn Val Pro Glu Leu Gly
   225 230 235 240
   Pro Ala Leu Thr Tyr Val Ala Gly Phe Thr Gly Phe His Gln Phe Ala
   245 250 255
   Ser Phe Thr Ala Asp Asp Val Gly Thr Ala Glu Ser Gly Leu Asn Ser
   260 265 270
   Ala Val Leu Ala Ser Asn Asp Glu Met Val Leu Leu Pro Ile Asn Glu
   275 280 285
   Pro Val His Gly Thr Lys Arg Lys Ser Gln Ile Gln Thr Tyr Leu Glu
   290 295 300
   His Asn Glu Gly Ala Gly Leu Gln His Leu Ala Leu Met Ser Glu Asp
   305 310 315 320
   Ile Phe Arg Thr Leu Arg Glu Met Arg Lys Arg Ser Ser Ile Gly Gly
   325 330 335
   Phe Asp Phe Met Pro Ser Pro Pro Pro Thr Tyr Tyr Gln Asn Leu Lys
   340 345 350
   Lys Arg Val Gly Asp Val Leu Ser Asp Asp Gln Ile Lys Glu Cys Glu
   355 360 365
   Glu Leu Gly Ile Leu Val Asp Arg Asp Asp Gln Gly Thr Leu Leu Gln
   370 375 380
   Ile Phe Thr Lys Pro Leu Gly Asp Arg Pro Thr Ile Phe Ile Glu Ile
   385 390 395 400
   Ile Gln Arg Val Gly Cys Met Met Lys Asp Glu Glu Gly Lys Ala Tyr
   405 410 415
   Gln Ser Gly Gly Cys Gly Gly Phe Gly Lys Gly Asn Phe Ser Glu Leu
   420 425 430
   Phe Lys Ser Ile Glu Glu Tyr Glu Lys Thr Leu Glu Ala Lys Gln Leu
   435 440 445
   Val Gly
   450
<210> 39
   <211> 1353
   <212> DNA
   <213> Artificial sequence
<220>
   <223> nucleotide sequence encoding mutant HPPD polypeptide
<220>
   <221> misc_feature
   <222> (4)..(6)
   <223> sequence coding for Ala
<220>
   <221> misc_feature
   <222> (7)..(24)
   <223> sequence coding for an His Tag containing 6 His
<400> 39
   atggctcatc accatcacca tcaccaaaac gccgccgttt cagagaatca aaaccatgat      60
   gacggcgctg cgtcgtcgcc gggattcaag ctcgtcggat tttccaagtt cgtaagaaag     120
   aatccaaagt ctgataaatt caaggttaag cgcttccatc acatcgagtt ctggtgcggc     180
   gacgcaacca acgtcgctcg tcgcttctcc tggggtctgg ggatgagatt ctccgccaaa     240
   tccgatcttt ccaccggaaa catggttcac gcctcttacc tactcacctc cggtgacctc     300
   cgattccttt tcactgctcc ttactctccg tctctctccg ccggagagat taaaccgaca     360
   accacagctt ctatcccaag tttcgatcac ggctcttgtc gttccttctt ctcgtcacat     420
   ggtctcggtg ttagagccgt tgcgattgaa gtagaagacg cagagtcagc tttctccatc     480
   agtgtagcta atggcgctat tccttcgtcg cctcctatcg tcctcaatga agcagttacg     540
   atcgctgagg ttaaactata cggcgatgtt gttctccgat atgttagtta caaagcagaa     600
   gataccgaaa aatccgaatt cttgccaggg ttcgagcgtg tagaggatgc gtcgtcgttc     660
   ccattggatt atggtatccg gcggcttgac cacgccgtgg gaaacgttcc tgagcttggt     720
   ccggctttaa cttatgtagc ggggttcact ggttttcacc aattcgcaga gttcacagca     780
   gacgacgttg gaaccgccga gagcggttta aattcagcgg cgctggctag caatgatgaa     840
   atggttcttc taccgattaa cgagccagtg cacggaacaa agaggaagag tcagattcag     900
   acgtatttgg aacataacga aggcgcaggg ctacaacatc tggctctgat gagtgaagac     960
   atattcagga ccctgagaga gatgaggaag aggagcagta ttggaggatt cgacttcatg    1020
   ccttctcctc cgcctactta ctaccagaat ctcaagaaac gggtcggcga cgtgctcagc    1080
   gatgatcaga tcaaggagtg tgaggaatta gggattcttg tagacagaga tgatcaaggg    1140
   acgttgcttc aaatcttcac aaaaccacta ggtgacaggc cgacgatatt tatagagata    1200
   atccagagag taggatgcat gatgaaagat gaggaaggga aggcttacca gagtggagga    1260
   tgtggtggtt ttggcaaagg caatttctct gagctcttca agtccattga agaatacgaa    1320
   aagactcttg aagccaaaca gttagtggga tga                                 1353
<210> 40
   <211> 450
   <212> PRT
   <213> Artificial sequence
<220>
   <223> encoded by SEQ ID No. 39
<220>
   <221> MISC_FEATURE
   <222> (2)..(2)
   <223> Ala
<220>
   <221> MISC_FEATURE
   <222> (3)..(8)
   <223> His Tag made of 6 His
<400> 40
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
                   85                  90                  95
   Ser Gly Asp Leu Arg Phe Leu Phe Thr Ala Pro Tyr Ser Pro Ser Leu
               100                 105                 110
   Ser Ala Gly Glu Ile Lys Pro Thr Thr Thr Ala Ser Ile Pro Ser Phe
           115                 120                 125
   Asp His Gly Ser Cys Arg Ser Phe Phe Ser Ser His Gly Leu Gly Val
       130                 135                 140
   Arg Ala Val Ala Ile Glu Val Glu Asp Ala Glu Ser Ala Phe Ser Ile
   145                 150                 155                 160
   Ser Val Ala Asn Gly Ala Ile Pro Ser Ser Pro Pro Ile Val Leu Asn
                   165                 170                 175
   Glu Ala Val Thr Ile Ala Glu Val Lys Leu Tyr Gly Asp Val Val Leu
               180                 185                 190
   Arg Tyr Val Ser Tyr Lys Ala Glu Asp Thr Glu Lys Ser Glu Phe Leu
           195                 200                 205
   Pro Gly Phe Glu Arg Val Glu Asp Ala Ser Ser Phe Pro Leu Asp Tyr
       210                 215                 220
   Gly Ile Arg Arg Leu Asp His Ala Val Gly Asn Val Pro Glu Leu Gly
   225                 230                 235                 240
   Pro Ala Leu Thr Tyr Val Ala Gly Phe Thr Gly Phe His Gln Phe Ala
                   245                 250                 255
   Glu Phe Thr Ala Asp Asp Val Gly Thr Ala Glu Ser Gly Leu Asn Ser
               260                 265                 270
   Ala Ala Leu Ala Ser Asn Asp Glu Met Val Leu Leu Pro Ile Asn Glu
           275                 280                 285
   Pro Val His Gly Thr Lys Arg Lys Ser Gln Ile Gln Thr Tyr Leu Glu
       290                 295                 300
   His Asn Glu Gly Ala Gly Leu Gln His Leu Ala Leu Met Ser Glu Asp
   305                 310                 315                 320
   Ile Phe Arg Thr Leu Arg Glu Met Arg Lys Arg Ser Ser Ile Gly Gly
                   325                 330                 335
   Phe Asp Phe Met Pro Ser Pro Pro Pro Thr Tyr Tyr Gln Asn Leu Lys
                   340                 345                 350
   Lys Arg Val Gly Asp Val Leu Ser Asp Asp Gln Ile Lys Glu Cys Glu
           355                 360                 365
   Glu Leu Gly Ile Leu Val Asp Arg Asp Asp Gln Gly Thr Leu Leu Gln
       370                 375                 380
   Ile Phe Thr Lys Pro Leu Gly Asp Arg Pro Thr Ile Phe Ile Glu Ile
   385                 390                 395                 400
   Ile Gln Arg Val Gly Cys Met Met Lys Asp Glu Glu Gly Lys Ala Tyr
                   405                 410                 415
   Gln Ser Gly Gly Cys Gly Gly Phe Gly Lys Gly Asn Phe Ser Glu Leu
               420                 425                 430
   Phe Lys Ser Ile Glu Glu Tyr Glu Lys Thr Leu Glu Ala Lys Gln Leu
           435                 440                 445
   Val Gly
       450
<210> 41
   <211> 1353
   <212> DNA
   <213> Artificial sequence
<220>
   <223> nucleotide sequebc encoding mutant HPPD polypeptide
<220>
   <221> misc_feature
   <222> (4)..(6)
   <223> sequence coding for Ala
<220>
   <221> misc_feature
   <222> (7)..(24)
   <223> sequence coding for an His Tag containing 6 His
<400> 41
   atggctcatc accatcacca tcaccaaaac gccgccgttt cagagaatca aaaccatgat      60
   gacggcgctg cgtcgtcgcc gggattcaag ctcgtcggat tttccaagtt cgtaagaaag     120
   aatccaaagt ctgataaatt caaggttaag cgcttccatc acatcgagtt ctggtgcggc     180
   gacgcaacca acgtcgctcg tcgcttctcc tggggtctgg ggatgagatt ctccgccaaa     240
   tccgatcttt ccaccggaaa catggttcac gcctcttacc tactcacctc cggtgacctc 300
   cgattccttt tcactgctcc ttactctccg tctctctccg ccggagagat taaaccgaca 360
   accacagctt ctatcccaag tttcgatcac ggctcttgtc gttccttctt ctcgtcacat 420
   ggtctcggtg ttagagccgt tgcgattgaa gtagaagacg cagagtcagc tttctccatc 480
   agtgtagcta atggcgctat tccttcgtcg cctcctatcg tcctcaatga agcagttacg 540
   atcgctgagg ttaaactata cggcgatgtt gttctccgat atgttagtta caaagcagaa 600
   gataccgaaa aatccgaatt cttgccaggg ttcgagcgtg tagaggatgc gtcgtcgttc 660
   ccattggatt atggtatccg gcggcttgac cacgccgtgg gaaacgttcc tgagcttggt 720
   ccggctttaa cttatgtagc ggggttcact ggttttcacc aattcgcaga gttcacagca 780
   gacgacgttg gaaccgccga gagcggttta aattcagcgg tcctggctag caatgatgaa 840
   atggttcttc tagcgattaa cgagccagtg cacggaacaa agaggaagag tcagattcag 900
   acgtatttgg aacataacga aggcgcaggg ctacaacatc tggctctgat gagtgaagac 960
   atattcagga ccctgagaga gatgaggaag aggagcagta ttggaggatt cgacttcatg 1020
   ccttctcctc cgcctactta ctaccagaat ctcaagaaac gggtcggcga cgtgctcagc 1080
   gatgatcaga tcaaggagtg tgaggaatta gggattcttg tagacagaga tgatcaaggg 1140
   acgttgcttc aaatcttcac aaaaccacta ggtgacaggc cgacgatatt tatagagata 1200
   atccagagag taggatgcat gatgaaagat gaggaaggga aggcttacca gagtggagga 1260
   tgtggtggtt ttggcaaagg caatttctct gagctcttca agtccattga agaatacgaa 1320
   aagactcttg aagccaaaca gttagtggga tga 1353
<210> 42
   <211> 450
   <212> PRT
   <213> Artificial sequence
<220>
   <223> encoded by SEQ ID No. 41
<220>
   <221> MISC_FEATURE
   <222> (2)..(2)
   <223> Ala
<220>
   <221> MISC_FEATURE
   <222> (3)..(8)
   <223> His Tag made of 6 His
<400> 42
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
                   85                  90                  95
   Ser Gly Asp Leu Arg Phe Leu Phe Thr Ala Pro Tyr Ser Pro Ser Leu
               100                 105                 110
   Ser Ala Gly Glu Ile Lys Pro Thr Thr Thr Ala Ser Ile Pro Ser Phe
           115                 120                 125
   Asp His Gly Ser Cys Arg Ser Phe Phe Ser Ser His Gly Leu Gly Val
       130                 135                 140
   Arg Ala Val Ala Ile Glu Val Glu Asp Ala Glu Ser Ala Phe Ser Ile
   145                 150                 155                 160
   Ser Val Ala Asn Gly Ala Ile Pro Ser Ser Pro Pro Ile Val Leu Asn
                   165                 170                 175
   Glu Ala Val Thr Ile Ala Glu Val Lys Leu Tyr Gly Asp Val Val Leu
               180                 185                 190
   Arg Tyr Val Ser Tyr Lys Ala Glu Asp Thr Glu Lys Ser Glu Phe Leu
           195                 200                 205
   Pro Gly Phe Glu Arg Val Glu Asp Ala Ser Ser Phe Pro Leu Asp Tyr
       210                 215                 220
   Gly Ile Arg Arg Leu Asp His Ala Val Gly Asn Val Pro Glu Leu Gly
   225                 230                 235                 240
   Pro Ala Leu Thr Tyr Val Ala Gly Phe Thr Gly Phe His Gln Phe Ala
                   245                 250                 255
   Glu Phe Thr Ala Asp Asp Val Gly Thr Ala Glu Ser Gly Leu Asn Ser
               260                 265                 270
   Ala Val Leu Ala Ser Asn Asp Glu Met Val Leu Leu Ala Ile Asn Glu
           275                 280                 285
   Pro Val His Gly Thr Lys Arg Lys Ser Gln Ile Gln Thr Tyr Leu Glu
       290                 295                 300
   His Asn Glu Gly Ala Gly Leu Gln His Leu Ala Leu Met Ser Glu Asp
   305                 310                 315                 320
   Ile Phe Arg Thr Leu Arg Glu Met Arg Lys Arg Ser Ser Ile Gly Gly
                   325                 330                 335
   Phe Asp Phe Met Pro Ser Pro Pro Pro Thr Tyr Tyr Gln Asn Leu Lys
               340                 345                 350
   Lys Arg Val Gly Asp Val Leu Ser Asp Asp Gln Ile Lys Glu Cys Glu
           355                 360                 365
   Glu Leu Gly Ile Leu Val Asp Arg Asp Asp Gln Gly Thr Leu Leu Gln
       370                 375                 380
   Ile Phe Thr Lys Pro Leu Gly Asp Arg Pro Thr Ile Phe Ile Glu Ile
   385                 390                 395                 400
   Ile Gln Arg Val Gly Cys Met Met Lys Asp Glu Glu Gly Lys Ala Tyr
                   405                 410                 415
   Gln Ser Gly Gly Cys Gly Gly Phe Gly Lys Gly Asn Phe Ser Glu Leu
               420                 425                 430
   Phe Lys Ser Ile Glu Glu Tyr Glu Lys Thr Leu Glu Ala Lys Gln Leu
           435                 440                 445
   Val Gly
       450
<210> 43
   <211> 1353
   <212> DNA
   <213> Artificial sequence
<220>
   <223> nucleotide sequence encoding mutant HPPD polypeptide
<220>
   <221> misc_feature
   <222> (4)..(6)
   <223> sequence coding for Ala
<220>
   <221> misc_feature
   <222> (7)..(24)
   <223> sequence coding for an His Tag containing 6 His
<400> 43
   atggctcatc accatcacca tcaccaaaac gccgccgttt cagagaatca aaaccatgat      60
   gacggcgctg cgtcgtcgcc gggattcaag ctcgtcggat tttccaagtt cgtaagaaag     120
   aatccaaagt ctgataaatt caaggttaag cgcttccatc acatcgagtt ctggtgcggc     180
   gacgcaacca acgtcgctcg tcgcttctcc tggggtctgg ggatgagatt ctccgccaaa     240
   tccgatcttt ccaccggaaa catggttcac gcctcttacc tactcacctc cggtgacctc     300
   cgattccttt tcactgctcc ttactctccg tctctctccg ccggagagat taaaccgaca     360
   accacagctt ctatcccaag tttcgatcac ggctcttgtc gttccttctt ctcgtcacat     420
   ggtctcggtg ttagagccgt tgcgattgaa gtagaagacg cagagtcagc tttctccatc     480
   agtgtagcta atggcgctat tccttcgtcg cctcctatcg tcctcaatga agcagttacg     540
   atcgctgagg ttaaactata cggcgatgtt gttctccgat atgttagtta caaagcagaa     600
   gataccgaaa aatccgaatt cttgccaggg ttcgagcgtg tagaggatgc gtcgtcgttc     660
   ccattggatt atggtatccg gcggcttgac cacgccgtgg gaaacgttcc tgagcttggt     720
   ccggctttaa cttatgtagc ggggttcact ggttttcacc aattcgcaga gttcacagca     780
   gacgacgttg gaaccgccga gagcggttta aattcagcgg tcctggctag caatgatgaa     840
   atggttcttc taccgattaa cgagccagtg cacggaacaa agaggaagag tcagattcag     900
   acgtatttgg aacataacga aggcgcaggg ctacaacatc tggctctgat gagtgaagac     960
   atattcagga ccctgagaga gatgaggaag aggagcagta ttggaggatt cgacttcatg    1020
   ccttctcctc cgcctactta ctaccagaat ctcaagaaac gggtcggcga cgtgctcagc    1080
   gatgatcaga tcaaggagtg tgaggaatta gggattatgg tagacagaga tgatcaaggg    1140
   acgttgcttc aaatcttcac aaaaccacta ggtgacaggc cgacgatatt tatagagata    1200
   atccagagag taggatgcat gatgaaagat gaggaaggga aggcttacca gagtggagga   1260
   tgtggtggtt ttggcaaagg caatttctct gagctcttca agtccattga agaatacgaa   1320
   aagactcttg aagccaaaca gttagtggga tga                                1353
<210> 44
   <211> 450
   <212> PRT
   <213> Artificial sequence
<220>
   <223> encoded by SEQ ID No. 43
<220>
   <221> MISC_FEATURE
   <222> (2)..(2)
   <223> Ala
<220>
   <221> MISC_FEATURE
   <222> (3)..(8)
   <223> His Tag made of 6 His
<400> 44
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
                   85                  90                  95
   Ser Gly Asp Leu Arg Phe Leu Phe Thr Ala Pro Tyr Ser Pro Ser Leu
               100                 105                 110
   Ser Ala Gly Glu Ile Lys Pro Thr Thr Thr Ala Ser Ile Pro Ser Phe
           115                 120                 125
   Asp His Gly Ser Cys Arg Ser Phe Phe Ser Ser His Gly Leu Gly Val
       130                 135                 140
   Arg Ala Val Ala Ile Glu Val Glu Asp Ala Glu Ser Ala Phe Ser Ile
   145                 150                 155                 160
   Ser Val Ala Asn Gly Ala Ile Pro Ser Ser Pro Pro Ile Val Leu Asn
                   165                 170                 175
   Glu Ala Val Thr Ile Ala Glu Val Lys Leu Tyr Gly Asp Val Val Leu
               180                 185                 190
   Arg Tyr Val Ser Tyr Lys Ala Glu Asp Thr Glu Lys Ser Glu Phe Leu
           195                 200                 205
   Pro Gly Phe Glu Arg Val Glu Asp Ala Ser Ser Phe Pro Leu Asp Tyr
       210                 215                 220
   Gly Ile Arg Arg Leu Asp His Ala Val Gly Asn Val Pro Glu Leu Gly
   225                 230                 235                 240
   Pro Ala Leu Thr Tyr Val Ala Gly Phe Thr Gly Phe His Gln Phe Ala
                   245                 250                 255
   Glu Phe Thr Ala Asp Asp Val Gly Thr Ala Glu Ser Gly Leu Asn Ser
               260                 265                 270
   Ala Val Leu Ala Ser Asn Asp Glu Met Val Leu Leu Pro Ile Asn Glu
           275                 280                 285
   Pro Val His Gly Thr Lys Arg Lys Ser Gln Ile Gln Thr Tyr Leu Glu
       290                 295                 300
   His Asn Glu Gly Ala Gly Leu Gln His Leu Ala Leu Met Ser Glu Asp
   305                 310                 315                 320
   Ile Phe Arg Thr Leu Arg Glu Met Arg Lys Arg Ser Ser Ile Gly Gly
                   325                 330                 335
   Phe Asp Phe Met Pro Ser Pro Pro Pro Thr Tyr Tyr Gln Asn Leu Lys
               340                 345                 350
   Lys Arg Val Gly Asp Val Leu Ser Asp Asp Gln Ile Lys Glu Cys Glu
           355                 360                 365
   Glu Leu Gly Ile Met Val Asp Arg Asp Asp Gln Gly Thr Leu Leu Gln
       370                 375                 380
   Ile Phe Thr Lys Pro Leu Gly Asp Arg Pro Thr Ile Phe Ile Glu Ile
   385                 390                 395                 400
   Ile Gln Arg Val Gly Cys Met Met Lys Asp Glu Glu Gly Lys Ala Tyr
                   405                 410                 415
   Gln Ser Gly Gly Cys Gly Gly Phe Gly Lys Gly Asn Phe Ser Glu Leu
               420                 425                 430
   Phe Lys Ser Ile Glu Glu Tyr Glu Lys Thr Leu Glu Ala Lys Gln Leu
           435                 440                 445
   Val Gly
       450
<210> 45
   <211> 1353
   <212> DNA
   <213> Artificial sequence
<220>
   <223> nucleotide sequence encoding mutant HPPD polypeptide
<220>
   <221> misc_feature
   <222> (4)..(6)
   <223> sequence coding for Ala
<220>
   <221> misc_feature
   <222> (7)..(24)
   <223> sequence coding for an His Tag containing 6 His
<400> 45
   atggctcatc accatcacca tcaccaaaac gccgccgttt cagagaatca aaaccatgat 60
   gacggcgctg cgtcgtcgcc gggattcaag ctcgtcggat tttccaagtt cgtaagaaag 120
   aatccaaagt ctgataaatt caaggttaag cgcttccatc acatcgagtt ctggtgcggc 180
   gacgcaacca acgtcgctcg tcgcttctcc tggggtctgg ggatgagatt ctccgccaaa 240
   tccgatcttt ccaccggaaa catggttcac gcctcttacc tactcacctc cggtgacctc 300
   cgattccttt tcactgctcc ttactctccg tctctctccg ccggagagat taaaccgaca 360
   accacagctt ctatcccaag tttcgatcac ggctcttgtc gttccttctt ctcgtcacat 420
   ggtctcggtg ttagagccgt tgcgattgaa gtagaagacg cagagtcagc tttctccatc    480
   agtgtagcta atggcgctat tccttcgtcg cctcctatcg tcctcaatga agcagttacg    540
   atcgctgagg ttaaactata cggcgatgtt gttctccgat atgttagtta caaagcagaa    600
   gataccgaaa aatccgaatt cttgccaggg ttcgagcgtg tagaggatgc gtcgtcgttc    660
   ccattggatt atggtatccg gcggcttgac cacgccgtgg gaaacgttcc tgagcttggt    720
   ccggctttaa cttatgtagc ggggttcact ggttttcacc aattcgcaga gttcacagca    780
   gacgacgttg gaaccgccga gagcggttta aattcagcgg tcctggctag caatgatgaa    840
   atggttcttc taccgattaa cgagccagtg cacggaacaa agaggaagag tcagattcag    900
   acgtatttgg aacataacga aggcgcaggg ctacaacatc tggctctgat gagtgaagac    960
   atattcagga ccctgagaga gatgaggaag aggagcagta ttggaggatt cgacttcatg   1020
   ccttctcctc cgcctactta ctaccagaat ctcaagaaac gggtcggcga cgtgctcagc   1080
   gatgatcaga tcaaggagtg tgaggaatta gggattcttg tagacagaga tgatcaaggg   1140
   acgttgcttc aaatcttcac aaaaccacta ggtgacaggc cgacgatatt tatagagata   1200
   atccagagag taggatgcat gatgaaagat gaggaaggga aggcttacca gagtggagga   1260
   tgtggtggtt ttggcattgg caatttctct gagctcttca agtccattga agaatacgaa   1320
   aagactcttg aagccaaaca gttagtggga tga                                1353
<210> 46
   <211> 450
   <212> PRT
   <213> Artificial sequence
<220>
   <223> encoded by SEQ ID No. 45
<220>
   <221> MISC_FEATURE
   <222> (2)..(2)
   <223> Ala
<220>
   <221> MISC_FEATURE
   <222> (3)..(8)
   <223> His Tag made of 6 His
<400> 46
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
                   85                  90                  95
   Ser Gly Asp Leu Arg Phe Leu Phe Thr Ala Pro Tyr Ser Pro Ser Leu
               100                 105                 110
   Ser Ala Gly Glu Ile Lys Pro Thr Thr Thr Ala Ser Ile Pro Ser Phe
           115                 120                 125
   Asp His Gly Ser Cys Arg Ser Phe Phe Ser Ser His Gly Leu Gly Val
       130                 135                 140
   Arg Ala Val Ala Ile Glu Val Glu Asp Ala Glu Ser Ala Phe Ser Ile
   145                 150                 155                 160
   Ser Val Ala Asn Gly Ala Ile Pro Ser Ser Pro Pro Ile Val Leu Asn
                   165                 170                 175
   Glu Ala Val Thr Ile Ala Glu Val Lys Leu Tyr Gly Asp Val Val Leu
               180                 185                 190
   Arg Tyr Val Ser Tyr Lys Ala Glu Asp Thr Glu Lys Ser Glu Phe Leu
           195                 200                 205
   Pro Gly Phe Glu Arg Val Glu Asp Ala Ser Ser Phe Pro Leu Asp Tyr
       210                 215                 220
   Gly Ile Arg Arg Leu Asp His Ala Val Gly Asn Val Pro Glu Leu Gly
   225                 230                 235                 240
   Pro Ala Leu Thr Tyr Val Ala Gly Phe Thr Gly Phe His Gln Phe Ala
                   245                 250                 255
   Glu Phe Thr Ala Asp Asp Val Gly Thr Ala Glu Ser Gly Leu Asn Ser
               260                 265                 270
   Ala Val Leu Ala Ser Asn Asp Glu Met Val Leu Leu Pro Ile Asn Glu
           275                 280                 285
   Pro Val His Gly Thr Lys Arg Lys Ser Gln Ile Gln Thr Tyr Leu Glu
       290                 295                 300
   His Asn Glu Gly Ala Gly Leu Gln His Leu Ala Leu Met Ser Glu Asp
   305                 310                 315                 320
   Ile Phe Arg Thr Leu Arg Glu Met Arg Lys Arg Ser Ser Ile Gly Gly
                   325                 330                 335
   Phe Asp Phe Met Pro Ser Pro Pro Pro Thr Tyr Tyr Gln Asn Leu Lys
               340                 345                 350
   Lys Arg Val Gly Asp Val Leu Ser Asp Asp Gln Ile Lys Glu Cys Glu
           355                 360                 365
   Glu Leu Gly Ile Leu Val Asp Arg Asp Asp Gln Gly Thr Leu Leu Gln
       370                 375                 380
   Ile Phe Thr Lys Pro Leu Gly Asp Arg Pro Thr Ile Phe Ile Glu Ile
   385                 390                 395                 400
   Ile Gln Arg Val Gly Cys Met Met Lys Asp Glu Glu Gly Lys Ala Tyr
                   405                 410                 415
   Gln Ser Gly Gly Cys Gly Gly Phe Gly Ile Gly Asn Phe Ser Glu Leu
               420                 425                 430
   Phe Lys Ser Ile Glu Glu Tyr Glu Lys Thr Leu Glu Ala Lys Gln Leu
           435                 440                 445
   Val Gly
       450
<210> 47
   <211> 1353
   <212> DNA
   <213> Artificial sequence
<220>
   <223> nucleotide sequence encoding mutant HPPD polypeptide
<220>
   <221> misc_feature
   <222> (4)..(6)
   <223> sequence coding for Ala
<220>
   <221> misc_feature
   <222> (7)..(24)
   <223> sequence coding for an His Tag containing 6 His
<400> 47
   atggctcatc accatcacca tcaccaaaac gccgccgttt cagagaatca aaaccatgat      60
   gacggcgctg cgtcgtcgcc gggattcaag ctcgtcggat tttccaagtt cgtaagaaag     120
   aatccaaagt ctgataaatt caaggttaag cgcttccatc acatcgagtt ctggtgcggc     180
   gacgcaacca acgtcgctcg tcgcttctcc tggggtctgg ggatgagatt ctccgccaaa     240
   tccgatcttt ccaccggaaa catggttcac gcctcttacc tactcacctc cggtgacctc     300
   cgattccttt tcactgctcc ttactctccg tctctctccg ccggagagat taaaccgaca     360
   accacagctt ctatcccaag tttcgatcac ggctcttgtc gttccttctt ctcgtcacat     420
   ggtctcggtg ttagagccgt tgcgattgaa gtagaagacg cagagtcagc tttctccatc     480
   agtgtagcta atggcgctat tccttcgtcg cctcctatcg tcctcaatga agcagttacg     540
   atcgctgagg ttaaactata cggcgatgtt gttctccgat atgttagtta caaagcagaa     600
   gataccgaaa aatccgaatt cttgccaggg ttcgagcgtg tagaggatgc gtcgtcgttc     660
   ccattggatt atggtatccg gcggcttgac cacgccgtgg gaaacgttcc tgagcttggt     720
   ccggctttaa cttatgtagc ggggttcact ggttttcacc aattcgcaga gttcacagca     780
   gacgacgttg gaaccgccga gagcggttta aattcagcgg tcctggctag caatgatgaa     840
   atggttcttc taccgattaa cgagccagtg cacggaacaa agaggaagag tcagattcag     900
   acgtatttgg aacataacga aggcgcaggg ctacaacatc tggctctgat gagtgaagac     960
   atattcagga ccctgagaga gatgaggaag aggagcagta ttggaggatt cgacttcatg    1020
   ccttctcctc cgcctactta ctaccagaat ctcaagaaac gggtcggcga cgtgctcagc    1080
   gatgatcaga tcaaggagtg tgaggaatta gggattcttg tagacagaga tgatcaaggg    1140
   acgttgcttc aaatcttcac aaaaccacta ggtgacaggc cgacgatatt tatagagata    1200
   atccagagag taggatgcat gatgaaagat gaggaaggga aggcttacca gagtggagga    1260
   tgtggtggtt ttggccaggg caatttctct gagctcttca agtccattga agaatacgaa    1320
   aagactcttg aagccaaaca gttagtggga tga                                 1353
<210> 48
   <211> 450
   <212> PRT
   <213> Artificial sequence
<220>
   <223> encoded by SEQ ID No. 47
<220>
   <221> MISC_FEATURE
   <222> (2)..(2)
   <223> Ala
<220>
   <221> MISC_FEATURE
   <222> (3)..(8)
   <223> His Tag made of 6 His
<400> 48
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
                   85                  90                  95
   Ser Gly Asp Leu Arg Phe Leu Phe Thr Ala Pro Tyr Ser Pro Ser Leu
               100                 105                 110
   Ser Ala Gly Glu Ile Lys Pro Thr Thr Thr Ala Ser Ile Pro Ser Phe
           115                 120                 125
   Asp His Gly Ser Cys Arg Ser Phe Phe Ser Ser His Gly Leu Gly Val
       130                 135                 140
   Arg Ala Val Ala Ile Glu Val Glu Asp Ala Glu Ser Ala Phe Ser Ile
   145                 150                 155                 160
   Ser Val Ala Asn Gly Ala Ile Pro Ser Ser Pro Pro Ile Val Leu Asn
                   165                 170                 175
   Glu Ala Val Thr Ile Ala Glu Val Lys Leu Tyr Gly Asp Val Val Leu
               180                 185                 190
   Arg Tyr Val Ser Tyr Lys Ala Glu Asp Thr Glu Lys Ser Glu Phe Leu
           195                 200                 205
   Pro Gly Phe Glu Arg Val Glu Asp Ala Ser Ser Phe Pro Leu Asp Tyr
       210                 215                 220
   Gly Ile Arg Arg Leu Asp His Ala Val Gly Asn Val Pro Glu Leu Gly
   225                 230                 235                 240
   Pro Ala Leu Thr Tyr Val Ala Gly Phe Thr Gly Phe His Gln Phe Ala
                   245                 250                 255
   Glu Phe Thr Ala Asp Asp Val Gly Thr Ala Glu Ser Gly Leu Asn Ser
               260                 265                 270
   Ala Val Leu Ala Ser Asn Asp Glu Met Val Leu Leu Pro Ile Asn Glu
           275                 280                 285
   Pro Val His Gly Thr Lys Arg Lys Ser Gln Ile Gln Thr Tyr Leu Glu
       290                 295                 300
   His Asn Glu Gly Ala Gly Leu Gln His Leu Ala Leu Met Ser Glu Asp
   305                 310                 315                 320
   Ile Phe Arg Thr Leu Arg Glu Met Arg Lys Arg Ser Ser Ile Gly Gly
                   325                 330                 335
   Phe Asp Phe Met Pro Ser Pro Pro Pro Thr Tyr Tyr Gln Asn Leu Lys
               340                 345                 350
   Lys Arg Val Gly Asp Val Leu Ser Asp Asp Gln Ile Lys Glu Cys Glu
           355                 360                 365
   Glu Leu Gly Ile Leu Val Asp Arg Asp Asp Gln Gly Thr Leu Leu Gln
       370                 375                 380
   Ile Phe Thr Lys Pro Leu Gly Asp Arg Pro Thr Ile Phe Ile Glu Ile
   385                 390                 395                 400
   Ile Gln Arg Val Gly Cys Met Met Lys Asp Glu Glu Gly Lys Ala Tyr
                   405                 410                 415
   Gln Ser Gly Gly Cys Gly Gly Phe Gly Gln Gly Asn Phe Ser Glu Leu
               420                 425                 430
   Phe Lys Ser Ile Glu Glu Tyr Glu Lys Thr Leu Glu Ala Lys Gln Leu
           435                 440                 445
   Val Gly
       450
<210> 49
   <211> 1353
   <212> DNA
   <213> Artificial sequence
<220>
   <223> nucleotide sequence encoding mutant HPPD polypeptide
<220>
   <221> misc_feature
   <222> (4)..(6)
   <223> sequence coding for Ala
<220>
   <221> misc_feature
   <222> (7)..(24)
   <223> sequence coding for an His Tag containing 6 His
<400> 49
   atggctcatc accatcacca tcaccaaaac gccgccgttt cagagaatca aaaccatgat      60
   gacggcgctg cgtcgtcgcc gggattcaag ctcgtcggat tttccaagtt cgtaagaaag     120
   aatccaaagt ctgataaatt caaggttaag cgcttccatc acatcgagtt ctggtgcggc     180
   gacgcaacca acgtcgctcg tcgcttctcc tggggtctgg ggatgagatt ctccgccaaa     240
   tccgatcttt ccaccggaaa catggttcac gcctcttacc tactcacctc cggtgacctc     300
   cgattccttt tcactgctcc ttactctccg tctctctccg ccggagagat taaaccgaca     360
   accacagctt ctatcccaag tttcgatcac ggctcttgtc gttccttctt ctcgtcacat     420
   ggtctcggtg ttagagccgt tgcgattgaa gtagaagacg cagagtcagc tttctccatc     480
   agtgtagcta atggcgctat tccttcgtcg cctcctatcg tcctcaatga agcagttacg     540
   atcgctgagg ttaaactata cggcgatgtt gttctccgat atgttagtta caaagcagaa    600
   gataccgaaa aatccgaatt cttgccaggg ttcgagcgtg tagaggatgc gtcgtcgttc    660
   ccattggatt atggtatccg gcggcttgac cacgccgtgg gaaacgttcc tgagcttggt    720
   ccggctttaa cttatgtagc ggggttcact ggttttcacc aattcgcaga gttcacagca    780
   gacgacgttg gaaccgccga gagcggttta aattcagcgg tcctggctag caatgatgaa    840
   atggttcttc taccgattaa cgagccagtg cacggaacaa agaggaagag tcagattcag    900
   acgtatttgg aacataacga aggcgcaggg ctacaacatc tggctctgat gagtgaagac    960
   atattcagga ccctgagaga gatgaggaag aggagcagta ttggaggatt cgacttcatg   1020
   ccttctcctc cgcctactta ctaccagaat ctcaagaaac gggtcggcga cgtgctcagc   1080
   gatgatcaga tcaaggagtg tgaggaatta gggattcttg tagacagaga tgatcaaggg   1140
   acgttgcttc aaatcttcac aaaaccacta ggtgacaggc cgacgatatt tatagagata   1200
   atccagagag taggatgcat gatgaaagat gaggaaggga aggcttacca gagtggagga   1260
   tgtggtggtt ttggcgtggg caatttctct gagctcttca agtccattga agaatacgaa   1320
   aagactcttg aagccaaaca gttagtggga tga                                 1353
<210> 50
   <211> 450
   <212> PRT
   <213> Artificial sequence
<220>
   <223> encoded by SEQ ID No. 49
<220>
   <221> MISC_FEATURE
   <222> (2)..(2)
   <223> Ala
<220>
   <221> MISC_FEATURE
   <222> (3)..(8)
   <223> His Tag made of 6 His
<400> 50
   Met Ala His His His His His His Gln Asn Ala Ala Val Ser Glu Asn
   1               5                   10                  15
   Gln Asn His Asp Asp Gly Ala Ala Ser Ser Pro Gly Phe Lys Leu Val
               20                  25                  30
   Gly Phe Ser Lys Phe Val Arg Lys Asn Pro Lys Ser Asp Lys Phe Lys
           35                  40                  45
   Val Lys Arg Phe His His Ile Glu Phe Trp Cys Gly Asp Ala Thr Asn
       50                  55                  60
   Val Ala Arg Arg Phe Ser Trp Gly Leu Gly Met Arg Phe Ser Ala Lys
   65                  70                  75                  80
   Ser Asp Leu Ser Thr Gly Asn Met Val His Ala Ser Tyr Leu Leu Thr
                   85                  90                  95
   Ser Gly Asp Leu Arg Phe Leu Phe Thr Ala Pro Tyr Ser Pro Ser Leu
               100                 105                 110
   Ser Ala Gly Glu Ile Lys Pro Thr Thr Thr Ala Ser Ile Pro Ser Phe
           115                 120                 125
   Asp His Gly Ser Cys Arg Ser Phe Phe Ser Ser His Gly Leu Gly Val
       130                 135                 140
   Arg Ala Val Ala Ile Glu Val Glu Asp Ala Glu Ser Ala Phe Ser Ile
   145                 150                 155                 160
   Ser Val Ala Asn Gly Ala Ile Pro Ser Ser Pro Pro Ile Val Leu Asn
                   165                 170                 175
   Glu Ala Val Thr Ile Ala Glu Val Lys Leu Tyr Gly Asp Val Val Leu
               180                 185                 190
   Arg Tyr Val Ser Tyr Lys Ala Glu Asp Thr Glu Lys Ser Glu Phe Leu
           195                 200                 205
   Pro Gly Phe Glu Arg Val Glu Asp Ala Ser Ser Phe Pro Leu Asp Tyr
       210                 215                 220
   Gly Ile Arg Arg Leu Asp His Ala Val Gly Asn Val Pro Glu Leu Gly
   225                 230                 235                 240
   Pro Ala Leu Thr Tyr Val Ala Gly Phe Thr Gly Phe His Gln Phe Ala
                   245                 250                 255
   Glu Phe Thr Ala Asp Asp Val Gly Thr Ala Glu Ser Gly Leu Asn Ser
               260                 265                 270
   Ala Val Leu Ala Ser Asn Asp Glu Met Val Leu Leu Pro Ile Asn Glu
           275                 280                 285
   Pro Val His Gly Thr Lys Arg Lys Ser Gln Ile Gln Thr Tyr Leu Glu
       290                 295                 300
   His Asn Glu Gly Ala Gly Leu Gln His Leu Ala Leu Met Ser Glu Asp
   305                 310                 315                 320
   Ile Phe Arg Thr Leu Arg Glu Met Arg Lys Arg Ser Ser Ile Gly Gly
                   325                 330                 335
   Phe Asp Phe Met Pro Ser Pro Pro Pro Thr Tyr Tyr Gln Asn Leu Lys
               340                 345                 350
   Lys Arg Val Gly Asp Val Leu Ser Asp Asp Gln Ile Lys Glu Cys Glu
           355                 360                 365
   Glu Leu Gly Ile Leu Val Asp Arg Asp Asp Gln Gly Thr Leu Leu Gln
       370                 375                 380
   Ile Phe Thr Lys Pro Leu Gly Asp Arg Pro Thr Ile Phe Ile Glu Ile
   385                 390                 395                 400
   Ile Gln Arg Val Gly Cys Met Met Lys Asp Glu Glu Gly Lys Ala Tyr
                   405                 410                 415
   Gln Ser Gly Gly Cys Gly Gly Phe Gly Val Gly Asn Phe Ser Glu Leu
               420                 425                 430
   Phe Lys Ser Ile Glu Glu Tyr Glu Lys Thr Leu Glu Ala Lys Gln Leu
           435                 440                 445
   Val Gly
       450

## Description

The present invention relates to an isolated nucleic acid comprising a nucleotide sequence encoding a mutated HPPD protein, wherein said mutated HPPD protein has HPPD activity, wherein in said mutated HPPD protein an amino acid has been replaced so that the resulting amino acid sequence comprises at least one amino acid selected from certain amino acids at a specific position important for conferring an increased HPPD inhibitor tolerance. The present invention also relates to proteins encoded by the nucleic acid of the invention, to chimeric genes, plant cells comprising the nucleic acid of the invention operably linked to a plant-expressible promoter and optionally a transcription termination and polyadenylation region, plants essentially consisting of the plant cells of the invention and methods of obtaining transgenic plants.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

HPPD (hydroxyphenylpyruvate dioxygenase) proteins are enzymes which catalyse the reaction in which para-hydroxyphenylpyruvate (abbreviated herein as HPP), a tyrosine degradation product, is transformed into homogentisate (abbreviated herein as HG), the precursor in plants of tocopherol and plastoquinone (Crouch N.P. et al. (1997) Tetrahedron, 53, 20, 6993-7010, Fritze et al., (2004), Plant Physiology 134:1388-1400). Tocopherol acts as a membrane-associated antioxidant.

Plastoquinone, firstly acts as an electron carrier between PSII and the cytochrome b6/f complex and secondly, is a redox cofactor for phytoene desaturase, which is involved in the biosynthesis of carotenoids.

Up to now, more than 700 nucleic acid sequences from various organisms present in NCBI database were annotated as coding for a putative protein having an HPPD domain. But for most of these sequences, it has not been proven that the protein would have an HPPD enzymatic activity either in an *in vitro* assay or an in *in planta* approach, nor that such HPPD protein can confer herbicide tolerance to HPPD inhibitor herbicides when expressed in a plant. Several HPPD proteins and their primary sequences have been described in the state of the art, in particular the HPPDs of bacteria such as *Pseudomonas* (Rüetschi et al., Eur. J. Biochem., 205, 459-466, 1992, WO 96/38567), of plants such as *Arabidopsis* (WO 96/38567, Genebank AF047834), carrot (WO 96/38567, Genebank 87257), *Avena sativa* (WO 02/046387), wheat (WO 02/046387), *Brachiaria platyphylla* (WO 02/046387), *Cenchrus echinatus* (WO 02/046387), *Lolium rigidum* (WO 02/046387), *Festuca arundinacea* (WO 02/046387), *Setaria faberi* (WO 02/046387), *Eleusine indica* (WO 02/046387), *Sorghum* (WO 02/046387), *Coccicoides* (Genebank COITRP),of *Coptis japonica* (WO 06/132270), *Chlamydomonas reinhardtii* (ES 2275365), or of mammals such as mouse or pig.

Most plants synthesize tyrosine via arrogenate (Abou-Zeid et al. (1995), Applied Env Microb 41: 1298-1302; Bonner et al., (1995), Plant Cells Physiol. 36, 1013-1022; Byng et al., (1981), Phytochemistry 6: 1289-1292; Connely and Conn (1986), Z. Naturforsch 41 c: 69-78; Gaines et al., (1982), Plants 156: 233-240). In these plants, the HPP is derived only from the degradation of tyrosine. On the other hand, in organisms such as the yeast *Sacharomyces cerevisiae* or the bacterium *Escherichia coli,* HPP is a tyrosine precursor, and it is synthesized by the action of an enzyme, prephenate dehydrogenase (hereinafter referred to as PDH), which converts prephenate to HPP (Lingens et al., (1967) European J. Biochem 1: 363-374; Sampathkumar and Morrisson (1982), Bioch Biophys Acta 701: 204-211). In these organisms, the production of HPP is therefore directly connected to the aromatic amino acid biosynthetic pathway (shikimate pathway), and not to the tyrosine degradation pathway.

Inhibition of HPPD leads to uncoupling of photosynthesis, deficiency in accessory light-harvesting pigments and, most importantly, to destruction of chlorophyll by UV-radiation and reactive oxygen species (bleaching) due to the lack of photo protection normally provided by carotenoids (Norris et al. (1995), Plant Cell 7: 2139-2149). Bleaching of photosynthetically active tissues leads to growth inhibition and plant death.

At present, most commercially available HPPD inhibitor herbicides belong to one of these four chemical families:
1) the triketones, e.g. sulcotrione [i.e. 2-[2-chloro-4-(methylsulfonyl)benzoyl]-1,3-cyclohexanedione], mesotrione [i.e.2-[4-(methylsulfonyl)-2-nitrobenzoyl]-1,3-cyclohexanedione]; tembotrione [i.e.2-[2-chloro-4-(methylsulfonyl)-3-[(2,2,2,-tri-fluoroethoxy)methyl] benzoyl]-1,3-cyclo-hexanedione]; tefuryltrione [i.e. 2-[2-chloro-4-(methylsulfonyl)-3-[[(tetrahydro-2-furanyl)methoxy]methyl]benzoyl]-1,3 cyclohexanedione]]; bicyclopyrone [i.e. 4-hydroxy-3-[[2-[(2-methoxyethoxy)methyl]-6-(trifluoromethyl)-3-pyridinyl]carbonyl]bicyclo[3.2.1]oct-3-en-2-one] ; Benzobicyclon [i.e. 3-(2-chloro-4-mesylbenzoyl)-2-phenylthiobicyclo[3.2.1]oct-2-en-4-one]
2) The diketonitriles, e.g. 2-cyano-3-cyclopropyl-1-(2-methylsulphonyl-4-trifluoromethylphenyl)-propane-1,3-dione and 2-cyano-1-[4-(methylsulphonyl)-2-trifluoromethylphenyl]-3-(1-methylcyclopropyl)propane-1,3-dione;
3) the isoxazoles, e.g. isoxaflutole [i.e.(5-cyclopropyl-4-isoxazolyl)[2-(methylsulfonyl)-4-(trifluoromethyl)phenyl]methanone]. In plants, the isoxaflutole is rapidly metabolized in DKN, a diketonitrile compound which exhibits the HPPD inhibitor property; and
4) the pyrazolinates, e.g. topramezone [i.e.[3-(4,5-dihydro-3-isoxazolyl)-2-methyl-4-(methylsulfonyl) phenyl](5-hydroxy-1-methyl-1H-pyrazol-4-yl)methanone], and pyrasulfotole [(5-hydroxy-1,3-dimethylpyrazol-4-yl(2-mesyl-4-trifluaromethylphenyl)methanone]; pyrazofen [2-[4-(2,4-dichlorobenzoyl)-1,3-dimethylpyrazol-5-yloxy]acetophenone].

These HPPD-inhibiting herbicides can be used against grass and/or broad leaf weeds in crop plants that display metabolic tolerance, such as maize (Zea mays) in which they are rapidly degraded (Schulz et al., (1993). FEBS letters, 318, 162-166; Mitchell et al., (2001) Pest Management Science, Vol 57, 120-128; Garcia et al., (2000) Biochem., 39, 7501-7507; Pallett et al., (2001) Pest Management Science, Vol 57, 133-142). In order to extend the scope of these HPPD-inhibiting herbicides, several efforts have been developed in order to confer to plants, particularly plants without or with an underperforming metabolic tolerance, a tolerance level acceptable under agronomic field conditions.

Besides the attempt of by-passing HPPD-mediated production of homogentisate (US 6,812,010), overexpressing the sensitive enzyme so as to produce quantities of the target enzyme in the plant which are sufficient in relation to the herbicide has been performed (WO96/38567). Overexpression of HPPD resulted in better pre-emergence tolerance to the diketonitrile derivative (DKN) of isoxaflutole (IFT), but tolerance was not sufficient for tolerance to post-emergence treatment (Matringe et al., (2005), Pest Management Science 61: 269-276).

In WO 04/024928, the inventors have sought to increase the prenylquinone biosynthesis (e.g., synthesis of plastoquinones, tocopherols) in the cells of plants by increasing the flux of the HPP precursor into the cells of these plants. This has been done by connecting the synthesis of said precursor to the "shikimate" pathway by overexpression of a PDH enzyme. They have also noted that the transformation of plants with a gene encoding a PDH enzyme makes it possible to increase the tolerance of said plants to HPPD inhibitors.

Another strategy was to mutate the HPPD in order to obtain a target enzyme which, while retaining its properties of catalysing the transformation of HPP into homogentisate, is less sensitive to HPPD inhibitors than is the native HPPD before mutation.
This strategy has been successfully applied for the production of plants tolerant to 2-cyano-3-cyclopropyl-1-(2-methylsulphonyl-4-trifluoromethylphenyl)-propane-1,3-dione and to 2-cyano-1-[4-(methylsulphonyl)-2-trifluoromethylphenyl]-3-(1-methylcyclopropyl)propane-1,3-dione (EP496630), two HPPD-inhibiting herbicides belonging to the diketonitriles family (WO 99/24585). Pro215Leu, Gly336Glu, Gly336Ile, and more particularly Gly336Trp (positions of the mutated amino acid are indicated with reference to the *Pseudomonas* HPPD) were identified as mutations which are responsible for an increased tolerance to pre-emergence treatment with these diketonitrile herbicides without causing an alteration of the activity of the enzyme.

More recently, introduction of a *Pseudomonas* HPPD gene into the plastid genome of tobacco and soybean has shown to be more effective than nuclear transformation, conferring even tolerance to post-emergence application of isoxaflutole (Dufourmantel et al., 2007, Plant Biotechnol J.5(1):118-33 ).

In the patent application WO 2009/144079, a nucleic acid sequence encoding a mutated hydroxyphenylpyruvate dioxygenase (HPPD) at position 336 of the *Pseudomonas fluorescens* HPPD protein and its use for obtaining plants which are tolerant to HPPD inhibitor herbicides is disclosed.

In WO 2002/046387, several domains of HPPD proteins originated from plants have been identified that may be relevant to confer tolerance to various HPPD inhibitor herbicides but no *in planta* nor biochemical data have been shown to confirm the impact of the as described domain functions.

In WO 2008/150473, the combination of two distinct tolerance mechanisms - a modified *Avena sativa* gene coding for a mutant HPPD enzyme and a CYP450 Maize monooxygenase (nsf1 gene) - was exemplified in order to obtain an improved tolerance to HPPD inhibitor herbicides, but no data have been disclosed demonstrating the synergistic effects based on the combination of both proteins.

US 2010/0197503 suggests a number of mutations at different positions within or close to the active site of the HPPD taken from Avena sativa and examined some of them for their inhibition by certain HPPD inhibitors such as sulcotrione in vitro and in planta.

Despite these successes obtained for the development of plants showing tolerance to several HPPD inhibitors herbicides described above, it is still necessary to develop and/or improve the tolerance of plants to newer or to several different HPPD inhibitors, particularly HPPD inhibitors belonging to the classes of the triketones (e.g.sulcotrione, mesotrione, tembotrione, benzobicyclon and bicyclopyrone) and the pyrazolinates (e.g., topramezone and pyrasulfotole).

Accordingly, the present invention relates to an isolated nucleic acid comprising a nucleotide sequence encoding a mutated HPPD protein, wherein said mutated HPPD protein has HPPD activity,
wherein in said mutated HPPD protein an amino acid has been replaced so that the resulting amino acid sequence comprises at least one amino acid selected from: Ala, Asp, Glu, Phe, Gly, Lys, Gln, Arg, Ser, Thr, Val, Tyr, Ile, Leu or Met at a position in an HPPD protein, said position corresponding to position 250 of the amino acid sequence of SEQ ID No. 2;

Unless indicated otherwise, the specific definitions or specific features of certain embodiments can be introduced into any other embodiment of the present invention.

According to the present invention, a "nucleic acid" is understood as being a nucleotide sequence which can be of the DNA or RNA type, preferably of the DNA type, and in particular double-stranded, whether it be of natural or synthetic origin, in particular a DNA sequence in which the codons which encode the HPPD according to the invention have been optimized in accordance with the host organism in which it is to be expressed (e.g., by replacing codons with those codons more preferred or most preferred in codon usage tables of such host organism or the group to which such host organism belongs, compared to the original or source organism).

An "isolated nucleic acid/DNA/protein", as used in the present application, refers to a nucleic acid/DNA/protein which is not naturally-occurring (such as an artificial or synthetic DNA with a different nucleotide sequence than the naturally-occurring DNA, or a modified protein) or which is no longer in the natural environment wherein it was originally present, e.g., a DNA coding sequence associated with a heterologous regulatory element (such as a bacterial coding sequence operably-linked to a plant-expressible promoter) in a chimeric gene, a DNA transferred into another host cell, such as a transgenic plant cell.

The terminology relating to nucleic acid or protein "comprising" a certain nucleotide sequence or amino acid sequence, as used throughout the text, refers to a nucleic acid or protein including or containing at least the described sequence, so that other nucleotide or amino acid sequences can be included at the 5' (or N-terminal) and/or 3' (or C-terminal) end, e.g. (the nucleotide sequence of) a selectable marker protein, (the nucleotide sequence of) a transit peptide, and/or a 5' leader sequence or a 3' trailer sequence. Similarly, use of the term "comprise", "comprising" or "comprises" throughout the text and the claims of this application should be understood to imply the inclusion of a stated integer or step or group of integers or steps but not the exclusion of any other integer or step or group of integers or steps. The term "comprising" also includes the term "consisting of".

In accordance with the present invention, the term "mutated HPPD protein" which is interchangeably used with the term "mutant HPPD protein" denotes an HPPD protein having an amino acid sequence which does not occur in nature. As opposed to the term "isolated" referred to above, the term "mutated" cannot refer to the environment of the (amino acid or protein) sequence in question, such as it being isolated from its natural environment or being coupled to a heterologous (amino acid or protein) sequence, but only refers to the amino acid sequence defining said mutated HPPD protein which cannot be found anywhere in nature but arose from a unmutated or wild-type starting amino acid sequence. In other words, in arriving at the nucleic acid of the present invention encoding a mutated HPPD protein, a starting amino acid sequence of a naturally existing protein has to be taken and to be modified by man by replacing at least one amino acid as defined in the present application.

The sequence which encodes an original unmutated HPPD which will be mutated according to the invention can be of any origin. In particular, it can be of bacterial, plant or animal origin. Advantageous examples which may be cited are bacteria of the Pseudomonas sp. type, for example Pseudomonas fluorescens, or otherwise cyanobacteria of the Synechocystis genus. The sequence can also be of plant origin, in particular derived from dicotyledonous plants, umbelliferous plants, or otherwise monocotyledonous plants. Advantageous examples which may be cited are plants such as tobacco, Arabidopsis, Daucus carotta, Zea mays (corn), wheat, barley, Avena sativa, wheat, Brachiaria platyphylla, Cenchrus echinatus, Lolium rigidum, Festuca arundinacea, Setaria faberi, Eleusine indica, and Sorghum. The coding sequences, and the way of isolating and cloning them, are described in the previously cited references. In a particular embodiment of the invention, the HPPD is from a bacterial origin, particularly from Pseudomonas sp., more particularly from Pseudomonas fluorescens, Rhodococcus sp., Blepharisma japonicum, Synechococcus sp., Picrophilus torridus, Kordia algicida or from a plant origin, particularly from Arabidopsis thaliana or Avena sativa. The HPPD to make the mutation (s) in for the purpose of the invention, can be any naturally-occurring HPPD, or any active fragment thereof or any variant thereof wherein some amino acids (1 to 10 amino acids) have been replaced, added or deleted for cloning purposes, to make a transit peptide fusion, and the like, which retains HPPD activity, i.e. the property of catalysing the conversion of para-hydroxyphenylpyruvate to homogentisate.

The mutated HPPD protein according to the present invention has HPPD activity, i. e., as described above, catalyses the reaction in which para-hydroxyphenylpyruvate is transformed into homogentisate. Preferentially, the catalytic activity of the isolated mutated HPPD of the present invention, when tested in vitro, does not differ from that of the unmutated reference HPPD protein by more than 70%, preferably more than 50%, more preferably more than 30%, even more preferably more than 20% when assayed under identical conditions and in the absence of the HPPD inhibitor herbicides described above. The catalytic activity of an HPPD enzyme may be defined by various methods well-known in the art. WO 2009/144079 describes various suitable screening methods.

Initial screens may be performed with the nucleic acid encoding the mutated HPPD protein of the invention being expressed in bacteria.

### Colorimetric Screening Test for Active HPPD Enzymes:

A YT-broth-type culture medium with 1% agarose, 5mM L-Tyrosine and 42mM Succinate, which contains the selection agent for the vector pSE420 (Invitrogen, Karlsruhe, Germany) is poured into deep well plates. E.coli culture in the exponentional growth phase which contains the vector pSE420-HPPDx (HPPDx means any gene coding for a putative HPPD enzyme/protein) is applied to each well. After 16 hours at 37°C, the wells which do not contain the culture medium, those which have been seeded with an E. coil culture containing the empty vector pSE420 are transparent, or those which have been seeded with an E. coli culture containing a vector pSE420-HPPDx containing a gene coding for an inactive HPPD are transparent, while the weels seeded with an E. coli culture containing the vector pSE420-HPPDx coding for an active HPPD are brown. It has been previously demonstrated that this test refelects the HPPD activity, whatever the orgin of this activity is, and allows the identification of HPPD activities (US 6,768,044), i.e. at a qualitative level.

Further and more elaborate screens may be carried out in plant cells or plants expressing the mutated HPPD protein of the invention.

The same screenings may also be used when examining of whether a mutated HPPD protein is capable of modulating, such as decreasing or increasing, the tolerance of a plant to at least one HPPD herbicide inhibitor which will be referred to further below, with the difference that at least one of such an HPPD inhibitor is added. Examples of HPPD inhibitors to be used in those screenings include tembotrione, mesotrione, pyrasulfotole, bicyclopyrone, topramezone and sulcotrione. A screening method which is simple to implement is to determine the dose of HPPD inhibitor which fully inhibits the original unmutated HPPD, and which is lethal for the cells which express this unmutated HPPD, and to subject the mutated cells to this predetermined dose, and thereafter to isolate the mutated cells which have withstood this lethal dose, and then to isolate and to clone the gene which encodes the mutated HPPD.

Alternatively, at the quantitative level data like pl₅₀ (pl₅₀-value means the log value of the concentration of inhibitor necessary to inhibit 50% of the enzyme activity in molar concentration) can be obtained by employing the isolated and purified HPPD polypeptide, i.e. the mutated vs. the unmutated HPPD polypeptide and in presence or absence of the any respective HPPD inhibitor herbicide.

The terms "tolerance", "tolerant" or "less sensitive" denotes the lack of susceptibility of a plant expressing the mutated HPPD protein of the present invention to substances, particularly herbicides, which inhibit HPPD proteins, optionally in comparison with the plant's own HPPD protein or with any known HPPD protein. More specifically, said terms mean the relative levels of inherent tolerance of the HPPD screened according to a visible indicator phenotype of the strain or plant transformed with a nucleic acid comprising the gene coding for the respective HPPD protein in the presence of different concentrations of the various HPPD inhibitors. Dose responses and relative shifts in dose responses associated with these indicator phenotypes (formation of brown colour, growth inhibition, bleaching, herbicidal effect etc) are conveniently expressed in terms, for example, of GR50 (concentration for 50% reduction of growth) or MIC (minimum inhibitory concentration) values where increases in values correspond to increases in inherent tolerance of the expressed HPPD, in the normal manner based upon plant damage, meristematic bleaching symptoms etc. at a range of different concentrations of herbicides. These data can be expressed in terms of, for example, GR50 values derived from dose/response curves having "dose" plotted on the x-axis and "percentage kill", "herbicidal effect", "numbers of emerging green plants" etc. plotted on the y-axis where increased GR50 values correspond to increased levels of inherent tolerance of the expressed HPPD. Herbicides can suitably be applied pre-emergence or post emergence.

Likewise, tolerance level of the nucleic acid or gene encoding an HPPD protein according to the invention, or the mutated HPPD protein of the invention is screened via transgenesis, regeneration, breeding and spray testing of a test plant such as tobacco, or a crop plant such as soybean or cotton. In line with the results obtained by such screening, such plants are at least 2-4 times more tolerant to HPPD inhibitors like tembotrione, mesotrione, diketonitrile, and/or bicyclopyrone, pyrasulfotole, than plants that do not contain any exogenous gene encoding an HPPD protein, or than plants that contain a gene comprising an *Arabidopsis thaliana* HPPD-encoding DNA, under control of the same promoter as the nucleic acid encoding the mutated HPPD protein of the invention. Accordingly, the term "capable of increasing the tolerance of a plant to at least one herbicide acting on HPPD" denotes a tolerance increased in a plant by at least the factor of 2, alternatively at least the factor of 3 or 4 or even 5 or 6 as compared to a plant only expressing it's endogenous HPPD or a plant expressing an Arabidopsis thaliana HPPD. In this regard, the term "herbicide acting on HPPD" is not limited to substances which are known and/or used as herbicides but to any substances which inhibits the catalytic activity of HPPD proteins.

In an alternative embodiment of the nucleic acid encoding a mutated HPPD polypeptide comprising a mutation as defined above, the HPPD protein comprises
a) a His at a position in an HPPD protein, said position corresponding to position 226 of the amino acid sequence of SEQ ID No. 2;
b) a Ser at a position in an HPPD protein, said position corresponding to position 267 of the amino acid sequence of SEQ ID No. 2;
c) an Asn at a position in an HPPD protein, said position corresponding to position 282 of the amino acid sequence of SEQ ID No. 2;
d) a His at a position in an HPPD protein, said position corresponding to position 308 of the amino acid sequence of SEQ ID No. 2;
e) a Tyr at a position in an HPPD protein, said position corresponding to position 342 of the amino acid sequence of SEQ ID No. 2;
f) a Glu at a position in an HPPD protein, said position corresponding to position 394 of the amino acid sequence of SEQ ID No. 2;
g) a Gly at a position in an HPPD protein, said position corresponding to position 420 of the amino acid sequence of SEQ ID No. 2; and
h) an Asn at a position in an HPPD protein, said position corresponding to position 423 of the amino acid sequence of SEQ ID No. 2

In the mutated HPPD protein encoded by the nucleic acid of the invention at least one amino acid has been deleted or replaced as defined above.
The replacement or deletion can be effected in the nucleic acid sequence which encodes the original unmutated, i. e. naturally occurring HPPD as defined above by any means which is appropriate for replacing, in the said sequence, the codon which encodes the amino acid to be replaced with the codon which corresponds to the amino acid which is to replace it, or by deleting a codon, with the said codons being widely described in the literature and well known to the skilled person.
Several molecular biological methods can be used to achieve this replacement or deletion. A preferred method for preparing a mutated nucleic acid sequence according to the invention and the corresponding protein comprises carrying out site-directed mutagenesis on codons encoding one or more amino acids which are selected in advance. The methods for obtaining these site-directed mutations are well known to the skilled person and widely described in the literature (in particular: Directed Mutagenesis: A Practical Approach, 1991, Edited by M.J. McPHERSON, IRL PRESS), or are methods for which it is possible to employ commercial kits (for example the U. S. E. mutagenesis kit from PHARMACIA). After the site-directed mutagenesis, it is useful to select the cells which contain a mutated HPPD which is less sensitive to an HPPD inhibitor by using an appropriate screening aid. Appropriate screening methods to achieve this have been described above.

In accordance with the present invention, the term "said position corresponding to position X", X being any number to be found in the respective context in the present application, does not only include the respective position in the SEQ ID No. referred to afterwards but also includes any sequence encoding an HPPD protein, where, after alignment with the reference SEQ ID No., the respective position might have a different number but corresponds to that indicated for the reference SEQ ID No. Whereas HPPD sequences may be very diverse and may only show a low sequence identity of about 30%, HPPD proteins are characterized by a common three dimensional consensus structure which is achieved despite a low sequence identity. Due to specific positions being conserved within HPPD proteins, alignment of HPPD proteins can be effected by by applying various alignment tools in a senseful manner.

Methods of aligning nucleic acid or amino acid sequences and, accordingly, determining the sequence identity of two or more sequences, are well-known in the art. They include performing mathematical algorithms such as the algorithm of Myers and Miller (1988) CABIOS 4:11-17 or the local alignment algorithm of Smith and Waterman (1981) Adv. Appl. Math. 2:482-489; the global alignment algorithm of Needleman and Wunsch (1970) J. Mol. Biol. 48:443-453; the algorithm of Karlin and Altschul (1990) Proc. Natl. Acad. Sci USA 872264 and that of Brutlag et al. (Comp. App. Biosci. 6:237-245 (1990 )).
Such algorithms can be implemented in computer programs including but not limited to CLUSTALX, ALIGN, GAP, BESTFIT, BLAST, FASTDB and FASTA.
For example, when using BESTFIT (Wisconsin Sequence Analysis Package, Version 8 for Unix, Genetics Computer Group, University Research Park, 575 Science Drive, Madison, WI 53711)or any other sequence alignment program to determine whether a particular sequence is, for instance, 95% identical to a reference sequence, the parameters are set, of course, such that the percentage of identity is calculated over the full length of the reference nucleotide sequence and that gaps in homology of up to 5% of the total number of nucleotides in the reference sequence are allowed.
The identity between a first sequence and a second sequence, also referred to as a global sequence alignment, is determined using the FASTDB computer program based on the algorithm of Brutlag and colleagues (Comp. App. Biosci. 6:237-245 (1990)). In a sequence alignment the query and subject sequences are both DNA sequences. The result of said global sequence alignment is in percent identity. Preferred parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

The present invention is based on the results of a combination of a comparison of the amino acid sequences of HPPD proteins from various organisms and the analysis of the substrate binding and inhibitor binding site of selected HPPD proteins using X-ray crystallography. Using this combined approach, it was possible to determine key positions in HPPD proteins, where an amino acid can be replaced with one of a defined set of other amino acids in order to modulate HPPD catalytic activity and the affinity to at least one HPPD herbicide inhibitor to a plant expressing the mutated HPPD protein.

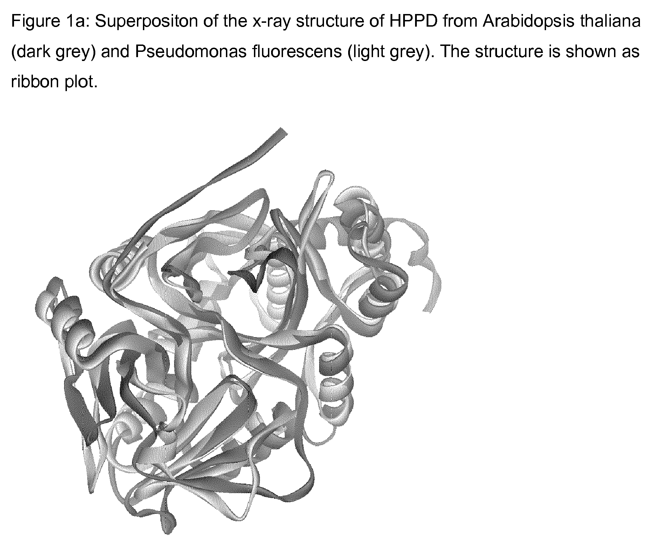

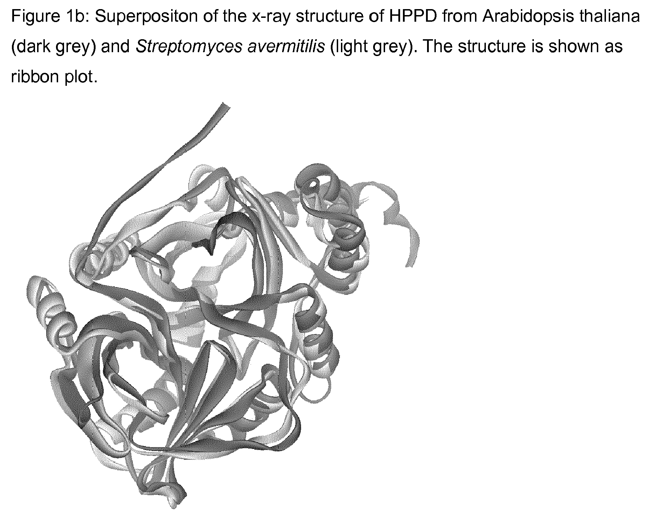

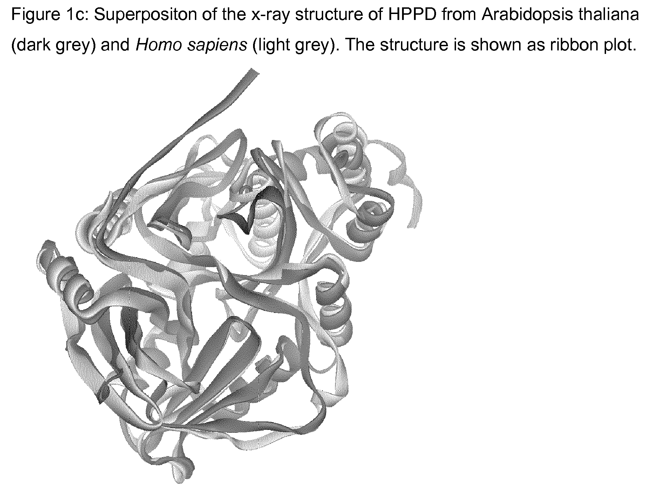

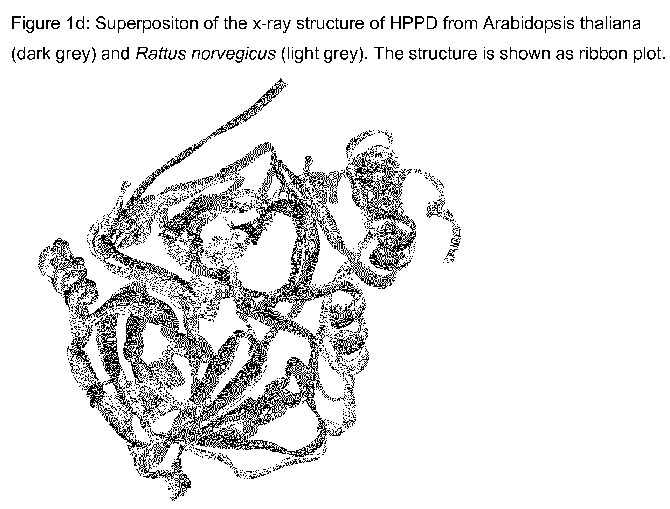

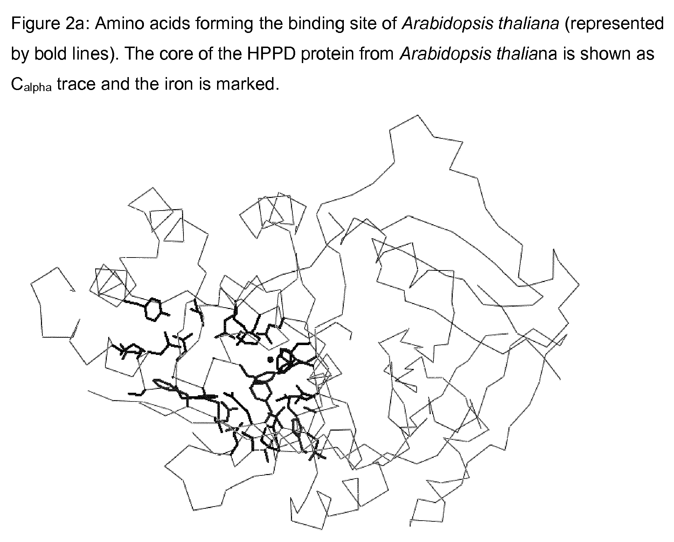

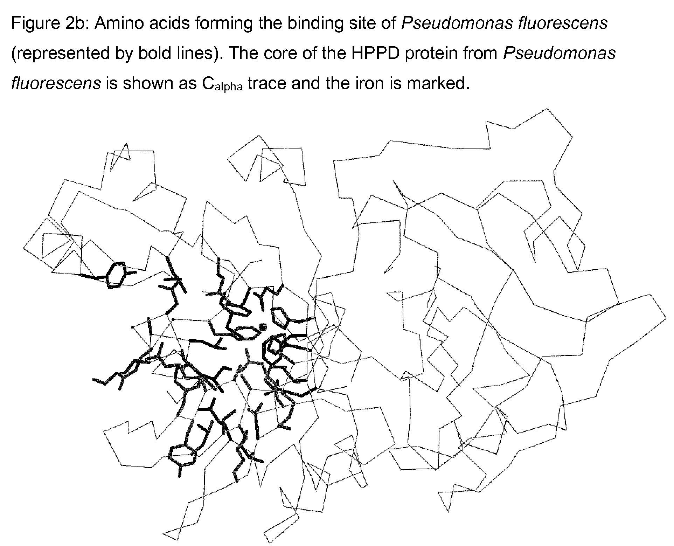

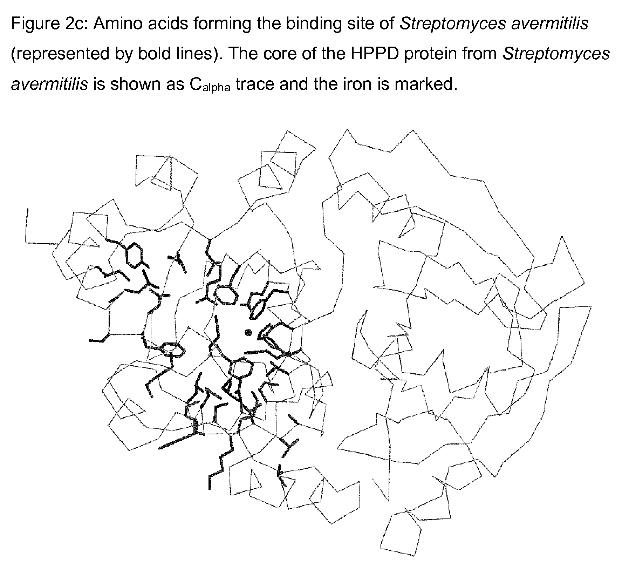

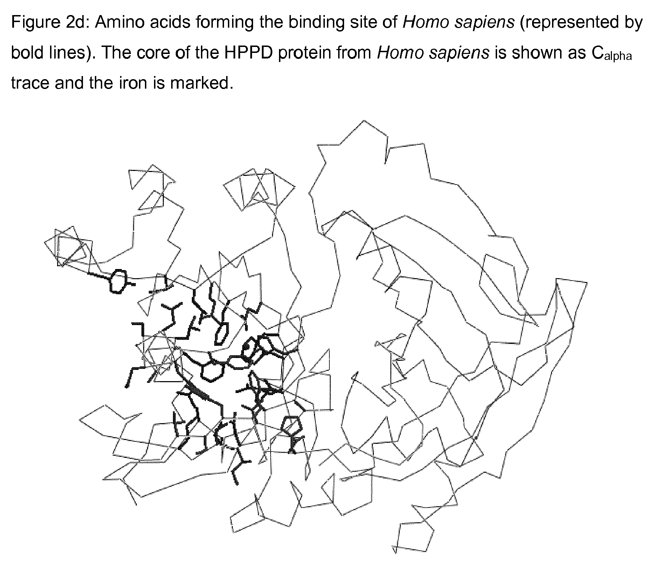

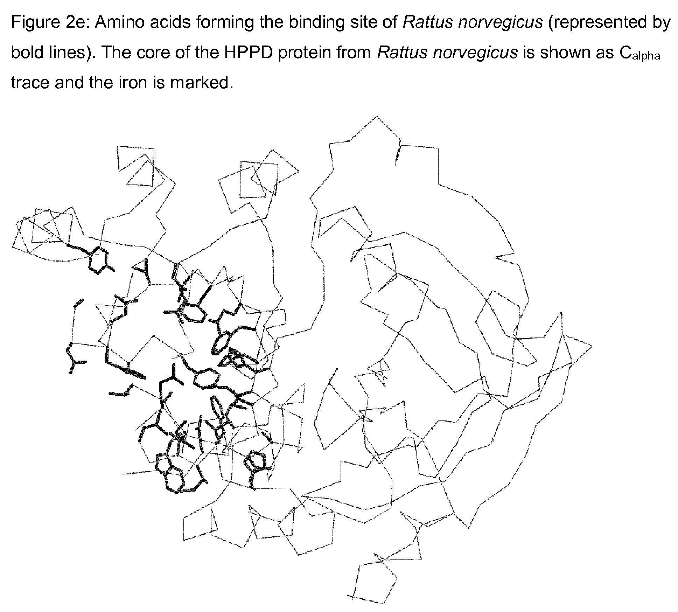

Superposition of the 3D structure of HPPD from *Arabidopsis thaliana* (1TFZ) (Yang et al., 2004, Biochemistry 43, 10414-10423) with the 3D structures of HPPD from other species such as *Pseudomonas fluorescens* (1CJX) (Serre et al., 1999, Structure Fold Des. 7, 977-988), *Streptomyces avermitilis* (1T47) (Brownlee et al., 2004, Biochemistry 43, 6370-6377), *Homo sapiens* (3ISQ) (PDB ID: 3isq Pilka et al., Structural Genomics Consortium (SGC). Crystal structure of human 4-Hydroxyphenylpyruvate dioxygenase), *Rattus norvegicus* (1SQI) (Yang et al., 2004, Biochemistry 43, 10414-10423) shows that they have the same folding and corresponding amino acids are at equivalent position in the 3D structure of the protein. Since the species with known 3D structures are very diverse in their amino acid sequence, it can be assumed that all HPPD sequences have the same basic folding even though the overall sequence identity is low. The sequence and the 3D structure of *Arabidopsis thaliana* has been used as reference structure in the present invention. Figure 1 shows the superposition of the structure of A.thaliana HPPD with the structure of (a) *Pseudomonas fluorescens,* (b) *Streptomyces avermitilis,* (c) *Homo sapiens* and (d) *Rattus norvegicus.* In order to define the binding site of the substrate and/or inhibitors, amino acids were selected which play a role in catalysis or inhibitor binding. This includes amino acids in the active site and amino acids from the C-terminal helix. The 3D arrangement is demonstrated in Figure 2 which displays the amino acids defined as binding site in case of (a) *Arabidopsis thaliana,* (b) *Pseudomonas fluorescens,* (c) *Streptomyces avermitilis,* (d) *Homo sapiens* and (e) *Rattus norvegicus.* The amino acid numbering of the *Pseudomonas Fluorescens structure* (1cjx) was changed into the numbering according to SEQ ID No. 10. The 36 amino acids defining the binding site including their position are listed in Table 1 for (a) *Arabidopsis thaliana,* (b) *Pseudomonas fluorescens,* (c) *Streptomyces avermitilis,* (d) *Homo sapiens* and (e) *Rattus norvegicus.*

**Table 1: Amino acids forming the binding site in A.thaliana, P.fluorescens, S.avermitilis, H.sapiens, R. norvegicus [not forming part of the invention]**

| *Arabidopsis thaliana* | | *Pseudomonas fluorescens* | | *Streptomyces avermitilis* | | *Homo sapiens* | | *Rattus norvegicus* | |
|---|---|---|---|---|---|---|---|---|---|
| Position | Amino Acid | Position | Amino Acid | Position | Amino Acid | Position | Amino Acid | Position | Amino Acid |
| 226 | H | 162 | H | 187 | H | 183 | H | 183 | H |
| 228 | V | 164 | T | 189 | V | 185 | V | 185 | V |
| 248 | H | 186 | R | 211 | T | 207 | H | 207 | H |
| 250 | F | 188 | A | 213 | M | 209 | F | 209 | F |
| 251 | A | 189 | R | 214 | K | 210 | W | 210 | W |
| 252 | E | 190 | Y | 215 | E | 211 | S | 211 | S |
| 253 | F | 191 | F | 216 | F | 212 | V | 212 | V |
| 265 | L | 200 | L | 228 | L | 224 | L | 224 | L |
| 267 | S | 202 | S | 230 | S | 226 | S | 226 | S |
| 268 | A | 203 | K | 231 | K | 227 | I | 227 | I |
| 269 | V | 204 | A | 232 | V | 228 | V | 228 | V |
| 270 | L | 205 | M | 233 | V | 229 | V | 229 | V |
| 271 | A | 206 | S | 234 | A | 230 | A | 230 | A |
| 280 | P | 215 | P | 243 | P | 239 | P | 239 | P |
| 282 | N | 217 | N | 245 | N | 241 | N | 241 | N |
| 293 | Q | 226 | Q | 255 | Q | 251 | Q | 251 | Q |
| 294 | I | 227 | I | 256 | I | 252 | I | 252 | I |
| 307 | Q | 240 | Q | 269 | Q | 265 | Q | 265 | Q |
| 308 | H | 241 | H | 270 | H | 266 | H | 266 | H |
| 335 | M | 264 | M | 293 | L | 289 | L | 289 | L |
| 342 | Y | 271 | Y | 299 | Y | 295 | Y | 295 | Y |
| 368 | L | 295 | L | 323 | L | 323 | L | 323 | L |

| Position | Amino Acid | Position | Amino Acid | Position | Amino Acid | Position | Amino Acid | Position | Amino Acid |
|---|---|---|---|---|---|---|---|---|---|
| 379 | Q | 310 | Q | 334 | Q | 334 | Q | 334 | Q |
| 381 | F | 312 | F | 336 | F | 336 | F | 336 | F |
| 392 | F | 321 | F | 347 | F | 347 | F | 347 | F |
| 394 | E | 323 | E | 349 | E | 349 | E | 349 | E |
| 419 | F | 333 | F | 359 | F | 359 | F | 359 | F |
| 420 | G | 334 | G | 360 | G | 360 | G | 360 | G |
| 421 | K | 335 | E | 361 | K | 361 | A | 361 | A |
| 422 | G | 336 | G | 362 | G | 362 | G | 362 | G |
| 423 | N | 337 | N | 363 | N | 363 | N | 363 | N |
| 424 | F | 338 | F | 364 | F | 364 | F | 364 | F |
| 425 | S | 339 | K | 365 | K | 365 | N | 365 | N |
| 426 | E | 340 | A | 366 | A | 366 | S | 366 | S |
| 427 | L | 341 | L | 367 | L | 367 | L | 367 | L |
| 431 | I | 345 | I | 371 | I | 371 | F | 371 | F |

An exemplary alignment of HPPD proteins is given in Table 2a for the HPPD proteins with known 3D structures. Table 2a gives the numbering of the amino acids of the Arabidopsis sequence and also the amino acids which are common within these HPPD sequences, with these amino acids being designated by an asterisk. On the basis of such an alignment and from the definition of the Arabidopsis amino acid by its position and its nature, it is easy to identify the position of the corresponding amino acid in another HPPD sequence. Figure 2 shows that this can be done with the alignment of sequences of different plant, mammalian and bacterial origin, demonstrating that this method of alignment, which is well known to a skilled person, can be generalized to any other sequence. An alignment of different HPPD sequences is also described in Patent Application WO 97/49816.

**Table 2a: Alignment of HPPD sequences with known crystal structures i.e. A.thaliana, P.fluorescens, S.avermitilis, H.sapiens, R. norvegicus [not forming part of the invention]**

| Pos. | 1TFZ | Pos. | 1CJX | Pos. | 1T47 | Pos. | 31SQ | Pos. | 1SQI | Common |
|---|---|---|---|---|---|---|---|---|---|---|
| | A. thaliana | P. fluorescens | | S. avermitilis | | H. Sapiens | | R. norvegicus | | amino acid |
| 1 | M | | - | | - | | - | | | |
| 2 | G | | - | | - | | - | | | |
| 3 | H | | - | | - | | - | | | |
| 4 | Q | | - | | - | | - | | | |
| 5 | N | | - | | - | | - | | | |
| 6 | A | | - | | - | | - | | | |
| 7 | A | | - | | - | | - | | | |
| 8 | V | | - | | - | | - | | | |
| 9 | S | | - | | - | | - | | | |
| 10 | E | | - | | - | | - | | | |
| 11 | N | | - | | - | | - | | | |
| 12 | Q | | - | | - | | - | | | |
| 13 | N | | - | | - | | - | | | |
| 14 | H | | - | 1 | M | | - | | | |

| Pos. | 1TFZ | Pos. | 1CJX | Pos. | 1T47 | Pos. | 3ISQ | Pos. | 1SQI | Common |
|---|---|---|---|---|---|---|---|---|---|---|
| | A. thaliana | P. fluorescens | | S. avermitilis | | H. Sapiens | | R. norvegicus | | amino acid |
| 15 | D | | - | 2 | T | | - | | | |
| 16 | D | | - | 3 | Q | | - | | | |
| 17 | G | | - | 4 | T | | - | | | |
| 18 | A | | - | 5 | T | | - | | | |
| 19 | A | | - | 6 | H | 8 | G | 8 | G | |
| 20 | S | | - | 7 | H | 9 | A | 9 | P | |
| 21 | S | | - | 8 | T | 10 | K | 10 | K | |
| 22 | P | | - | 9 | P | 11 | P | 11 | P | |
| 23 | G | | - | | - | | - | | | |
| 24 | F | | - | | - | | - | | | |
| 25 | K | | - | | - | | - | | | |
| 26 | L | | - | | - | | - | | | |
| 27 | V | | - | | - | | - | | | |
| 28 | G | | - | | - | | - | | | |
| 29 | F | | - | | - | | - | | | |
| 30 | S | | - | | - | | - | | | |
| 31 | K | | - | | - | | - | | | |
| 32 | F | | - | | - | | - | | | |
| 33 | V | | - | | - | | - | | | |
| 34 | R | | - | 10 | D | 12 | E | 12 | E | |
| 35 | K | 2 | A | 11 | T | 13 | R | 13 | R | |
| 36 | N | 3 | D | 12 | A | 14 | G | 14 | G | |
| 37 | P | 4 | L | 13 | R | 15 | R | 15 | R | |
| 38 | K | 5 | Y | 14 | Q | 16 | F | 16 | F | |
| 39 | S | 6 | E | 15 | A | 17 | L | 17 | L | |
| 40 | D | 7 | N | 16 | D | 18 | H | 18 | H | |
| 41 | K | 8 | P | 17 | P | | - | | | |
| 42 | F | 9 | M | 18 | F | | - | | | |
| 43 | K | 10 | G | 19 | P | | - | | | |
| 44 | V | 11 | L | 20 | V | | - | | | |
| 45 | K | 12 | M | 21 | K | | - | | | |
| 46 | R | 13 | G | 22 | G | | - | | | |
| 47 | F | 14 | F | 23 | M | 19 | F | 19 | F | |
| 48 | H | 15 | E | 24 | D | 20 | H | 20 | H | |
| 49 | H | 16 | F | 25 | A | 21 | S | 21 | S | |
| 50 | I | 17 | I | 26 | V | 22 | V | 22 | V | |
| 51 | E | 18 | E | 27 | V | 23 | T | 23 | T | |
| 52 | F | 19 | F | 28 | F | 24 | F | 24 | F | x |
| 53 | W | 20 | A | 29 | A | 25 | W | 25 | W | |
| 54 | C | 21 | S | 30 | V | 26 | V | 26 | V | |
| 55 | G | 22 | P | 31 | G | 27 | G | 27 | G | |
| 56 | D | 23 | T | 32 | N | 28 | N | 28 | N | |
| 57 | A | 24 | P | 33 | A | 29 | A | 29 | A | |
| 58 | T | 25 | G | 34 | K | 30 | K | 30 | K | |
| 59 | N | 26 | T | 35 | Q | 31 | Q | 31 | Q | |
| 60 | V | 27 | L | 36 | A | 32 | A | 32 | A | |
| 61 | A | 28 | E | 37 | A | 33 | A | 33 | A | |
| 62 | R | 29 | P | 38 | H | 34 | S | 34 | S | |
| 63 | R | 30 | I | 39 | Y | 35 | F | 35 | F | |
| 64 | F | 31 | F | 40 | Y | 36 | Y | 36 | Y | |
| 65 | S | 32 | E | 41 | S | 37 | C | 37 | C | |
| 66 | W | 33 | I | 42 | T | 38 | S | 38 | N | |
| 67 | G | 34 | M | 43 | A | 39 | K | 39 | K | |
| 68 | L | 35 | G | 44 | F | 40 | M | 40 | M | |
| 69 | G | 36 | F | 45 | G | 41 | G | 41 | G | |
| 70 | M | 37 | T | 46 | M | 42 | F | 42 | F | |
| 71 | R | 38 | K | 47 | Q | 43 | E | 43 | E | |
| 72 | F | 39 | V | 48 | L | 44 | P | 44 | P | |
| 73 | S | 40 | A | 49 | V | 45 | L | 45 | L | |
| 74 | A | 41 | T | 50 | A | 46 | A | 46 | A | |
| 75 | K | 42 | H | 51 | Y | 47 | Y | 47 | Y | |
| 76 | S | 43 | R | 52 | S | 48 | R | 48 | K | |
| 77 | D | 44 | S | 53 | G | 49 | G | 49 | G | |
| 78 | L | 45 | K | 54 | P | 50 | L | 50 | L | |
| 79 | S | 46 | N | 55 | E | 51 | E | 51 | E | |
| 80 | T | | - | 56 | N | 52 | T | 52 | T | |
| 81 | G | | - | 57 | G | 53 | G | 53 | G | |
| 82 | N | | - | 58 | S | 54 | S | 54 | S | |
| 83 | M | | - | 59 | R | 55 | R | 55 | R | |
| 84 | V | | - | 60 | E | 56 | E | 56 | E | |
| 85 | H | | - | 61 | T | 57 | V | 57 | V | |
| 86 | A | | - | 62 | A | 58 | V | 58 | V | |
| 87 | S | 47 | V | 63 | S | 59 | S | 59 | S | |
| 88 | Y | 48 | H | 64 | Y | 60 | H | 60 | H | |
| 89 | L | 49 | L | 65 | V | 61 | V | 61 | V | |
| 90 | L | 50 | Y | 66 | L | 62 | I | 62 | I | |
| 91 | T | 51 | R | 67 | T | 63 | K | 63 | K | |
| 92 | S | 52 | Q | 68 | N | 64 | Q | 64 | Q | |
| 93 | G | 53 | G | 69 | G | 65 | G | 65 | G | X |
| 94 | D | 54 | E | 70 | S | 66 | K | 66 | K | |
| 95 | L | 55 | I | 71 | A | 67 | I | 67 | I | |
| 96 | R | 56 | N | 72 | R | 68 | V | 68 | V | |
| 97 | F | 57 | L | 73 | F | 69 | F | 69 | F | |
| 98 | L | 58 | I | 74 | V | 70 | V | 70 | V | |
| 99 | F | 59 | L | 75 | L | 71 | L | 71 | L | |
| 100 | T | 60 | N | 76 | T | 72 | S | 72 | C | |
| 101 | A | 61 | N | 77 | S | 73 | S | 73 | S | |
| 102 | P | 62 | E | 78 | V | 74 | A | 74 | A | |
| 103 | Y | 63 | P | 79 | I | 75 | L | 75 | L | |
| 104 | S | 64 | N | 80 | K | 76 | N | 76 | N | |
| 105 | P | 65 | S | 81 | P | | - | | | |
| 106 | S | 66 | I | 82 | A | | - | | | |
| 107 | L | 67 | A | 83 | T | | - | | | |
| 108 | S | 68 | S | 84 | P | 77 | P | 77 | P | |
| 109 | A | | - | 85 | W | 78 | W | 78 | W | |
| 110 | G | | - | 86 | G | 79 | N | 79 | N | |
| 111 | E | | - | 87 | H | 80 | K | 80 | K | |
| 112 | I | | - | 88 | F | 81 | E | 81 | E | |
| 113 | K | | - | 89 | L | 82 | M | 82 | M | |
| 114 | P | | - | 90 | A | 83 | G | 83 | G | |
| 115 | T | | - | | - | | - | | | |
| 116 | T | | - | | - | | - | | | |
| 117 | T | | - | | - | | - | | | |
| 118 | A | | - | | - | | - | | | |
| 119 | S | | - | | - | | - | | | |
| 120 | I | | - | | - | | - | | | |
| 121 | P | | - | | - | | - | | | |
| 122 | S | | - | | - | | - | | | |
| 123 | F | | - | | - | | - | | | |
| 124 | D | | - | | - | | - | | | |
| 125 | H | | - | | - | | - | | | |
| 126 | G | | - | | - | | - | | | |
| 127 | S | | - | | - | | - | | | |
| 128 | C | | - | | - | | - | | | |
| 129 | R | | - | | - | | - | | | |
| 130 | S | 69 | Y | 91 | D | 84 | D | 84 | D | |
| 131 | F | 70 | F | 92 | H | 85 | H | 85 | H | |
| 132 | F | 71 | A | 93 | V | 86 | L | 86 | L | |
| 133 | S | 72 | A | 94 | A | 87 | V | 87 | V | |
| 134 | S | 73 | E | 95 | E | 88 | K | 88 | K | |
| 135 | H | 74 | H | 96 | H | 89 | H | 89 | H | X |
| 136 | G | 75 | G | 97 | G | 90 | G | 90 | G | X |
| 137 | L | 76 | P | 98 | D | 91 | D | 91 | D | |
| 138 | G | 77 | S | 99 | G | 92 | G | 92 | G | |
| 139 | V | 78 | V | 100 | V | 93 | V | 93 | V | X |
| 140 | R | 79 | C | 101 | V | 94 | K | 94 | K | |
| 141 | A | 80 | G | 102 | D | 95 | D | 95 | D | |
| 142 | V | 81 | M | 103 | L | 96 | I | 96 | I | |
| 143 | A | 82 | A | 104 | A | 97 | A | 97 | A | X |
| 144 | I | 83 | F | 105 | I | 98 | F | 98 | F | |
| 145 | E | 84 | R | 106 | E | 99 | E | 99 | E | |
| 146 | V | 85 | V | 107 | V | 100 | V | 100 | V | X |
| 147 | E | 86 | K | 108 | P | 101 | E | 101 | E | |
| 148 | D | 87 | D | 109 | D | 102 | D | 102 | D | X |
| 149 | A | 88 | S | 110 | A | 103 | C | 103 | C | |
| 150 | E | 89 | Q | 111 | R | 104 | D | 104 | E | |
| 151 | S | 90 | K | 112 | A | 105 | Y | 105 | H | |
| 152 | A | 91 | A | 113 | A | 106 | I | 106 | I | |
| 153 | F | 92 | Y | 114 | H | 107 | V | 107 | V | |
| 154 | S | 93 | N | 115 | A | 108 | Q | 108 | Q | |
| 155 | I | 94 | R | 116 | Y | 109 | K | 109 | K | |
| 156 | S | 95 | A | 117 | A | 110 | A | 110 | A | |
| 157 | V | 96 | L | 118 | I | 111 | R | 111 | R | |
| 158 | A | 97 | E | 119 | E | 112 | E | 112 | E | |
| 159 | N | 98 | L | 120 | H | 113 | R | 113 | R | |
| 160 | G | 99 | G | 121 | G | 114 | G | 114 | G | X |
| 161 | A | 100 | A | 122 | A | 115 | A | 115 | A | |
| 162 | I | 101 | Q | 123 | R | 116 | K | 116 | K | |
| 163 | P | | - | 124 | S | 117 | I | 117 | I | |
| 164 | S | | - | 125 | V | 118 | M | 118 | V | |
| 165 | S | | - | 126 | A | 119 | R | 119 | R | |
| 166 | P | | - | 127 | E | 120 | E | 120 | E | |
| 167 | P | 102 | P | 128 | P | 121 | P | 121 | P | X |
| 168 | I | 103 | I | 129 | Y | 122 | W | 122 | W | |
| 169 | V | 104 | H | 130 | E | 123 | V | 123 | V | |
| 170 | L | 105 | I | 131 | L | 124 | E | 124 | E | |
| 171 | N | 106 | D | 132 | K | 125 | Q | 125 | E | |
| 172 | E | 107 | T | 133 | D | 126 | D | 126 | D | |
| 173 | A | 108 | G | 134 | E | 127 | K | 127 | K | |
| 174 | V | 109 | P | 135 | H | 128 | F | 128 | F | |
| 175 | T | 110 | M | 136 | G | 129 | G | 129 | G | |
| 176 | I | 111 | E | 137 | T | 130 | K | 130 | K | |
| 177 | A | 112 | L | 138 | V | 131 | V | 131 | V | |
| 178 | E | 113 | N | 139 | V | 132 | K | 132 | K | |
| 179 | V | 114 | L | 140 | L | 133 | F | 133 | F | |
| 180 | K | 115 | P | 141 | A | 134 | A | 134 | A | |
| 181 | L | 116 | A | 142 | A | 135 | V | 135 | V | |
| 182 | Y | 117 | I | 143 | I | 136 | L | 136 | L | |
| 183 | G | 118 | K | 144 | A | 137 | Q | 137 | Q | |
| 184 | D | 119 | G | 145 | T | 138 | T | 138 | T | |
| 185 | V | 120 | I | 146 | Y | 139 | Y | 139 | Y | |
| 186 | V | 121 | G | 147 | G | 140 | G | 140 | G | |
| 187 | L | 122 | G | 148 | K | 141 | D | 141 | D | |
| 188 | R | 123 | A | 149 | T | 142 | T | 142 | T | |
| 189 | Y | 124 | P | 150 | R | 143 | T | 143 | T | |
| 190 | V | 125 | L | 151 | H | 144 | H | 144 | H | |
| 191 | S | 126 | Y | 152 | T | 145 | T | 145 | T | |
| 192 | Y | 127 | L | 153 | L | 146 | L | 146 | L | |
| 193 | K | 128 | I | 154 | V | 147 | V | 147 | V | |
| 194 | A | 129 | D | 155 | D | 148 | E | 148 | E | |
| 195 | E | 130 | R | 156 | R | 149 | K | 149 | K | |
| 196 | D | 131 | F | 157 | T | 150 | M | 150 | I | |
| 197 | T | 132 | G | 158 | G | 151 | N | 151 | N | |
| 198 | E | 133 | E | 159 | Y | 152 | Y | 152 | Y | |
| | - | 134 | G | | - | | - | | | |
| 199 | K | 135 | S | 160 | D | 153 | I | 153 | T | |
| 200 | S | 136 | S | 161 | G | 154 | G | 154 | G | |
| 201 | E | 137 | I | 162 | P | 155 | Q | 155 | R | |
| 202 | F | 138 | Y | 163 | Y | 156 | F | 156 | F | |
| 203 | L | 139 | D | 164 | L | 157 | L | 157 | L | |
| 204 | P | 140 | I | 165 | P | 158 | P | 158 | P | |
| 205 | G | 141 | D | 166 | G | 159 | G | 159 | G | |
| 206 | F | 142 | F | 167 | Y | 160 | Y | 160 | F | |
| 207 | E | 143 | V | 168 | V | 161 | E | 161 | E | |
| 208 | R | 144 | Y | 169 | A | 162 | A | 162 | A | |
| 209 | V | 145 | L | 170 | A | 163 | P | 163 | P | |
| 210 | E | 146 | E | 171 | A | 164 | A | 164 | T | |
| 211 | D | 147 | G | | - | 165 | F | 165 | Y | |
| | - | | - | | - | 166 | M | 166 | K | |
| | - | | - | | - | 167 | D | 167 | D | |
| | - | | - | 172 | P | 168 | P | 168 | T | |
| 212 | A | 148 | V | 173 | I | 169 | L | 169 | L | |
| 213 | S | 149 | E | 174 | V | 170 | L | 170 | L | |
| 214 | S | 150 | R | 175 | E | 171 | P | 171 | P | |
| 215 | F | 151 | N | 176 | P | 172 | K | 172 | K | |
| 216 | P | 152 | P | 177 | P | 173 | L | 173 | L | |
| 217 | L | 153 | V | 178 | A | 174 | P | 174 | P | |
| 218 | D | 154 | G | 179 | H | 175 | K | 175 | S | |
| 219 | Y | 155 | A | 180 | R | 176 | C | 176 | C | |
| 220 | G | 156 | G | 181 | T | 177 | S | 177 | N | |
| 221 | I | 157 | L | 182 | F | 178 | L | 178 | L | |
| 222 | R | 158 | K | 183 | Q | 179 | E | 179 | E | |
| 223 | R | 159 | V | 184 | A | 180 | M | 180 | I | |
| 224 | L | 160 | I | 185 | I | 181 | I | 181 | I | |
| 225 | D | 161 | D | 186 | D | 182 | D | 182 | D | x |
| 226 | H | 162 | H | 187 | H | 183 | H | 183 | H | x |
| 227 | A | 163 | L | 188 | C | 184 | I | 184 | I | |
| 228 | V | 164 | T | 189 | V | 185 | V | 185 | V | |
| 229 | G | 165 | H | 190 | G | 186 | G | 186 | G | |
| 230 | N | 166 | N | 191 | N | 187 | N | 187 | N | x |
| 231 | V | 167 | V | 192 | V | 188 | Q | 188 | Q | |
| 232 | P | 168 | Y | 193 | E | 189 | P | 189 | P | |
| | - | 169 | R | 194 | L | 190 | D | 190 | D | |
| | - | 170 | G | 195 | G | 191 | Q | 191 | Q | |
| 233 | E | 171 | R | 196 | R | 192 | E | 192 | E | |
| 234 | L | 172 | M | 197 | M | 193 | M | 193 | M | |
| 235 | G | 173 | V | 198 | N | 194 | V | 194 | E | |
| 236 | P | 174 | Y | 199 | E | 195 | S | 195 | S | |
| 237 | A | 175 | W | 200 | W | 196 | A | 196 | A | |
| 238 | L | 176 | A | 201 | V | 197 | S | 197 | S | |
| 239 | T | 177 | N | 202 | G | 198 | E | 198 | E | |
| 240 | Y | 178 | F | 203 | F | 199 | W | 199 | W | |
| 241 | V | 179 | Y | 204 | Y | 200 | Y | 200 | Y | |
| 242 | A | 180 | E | 205 | N | 201 | L | 201 | L | |
| 243 | G | 181 | K | 206 | K | 202 | K | 202 | K | |
| 244 | F | 182 | L | 207 | V | 203 | N | 203 | N | |
| 245 | T | 183 | F | 208 | M | 204 | L | 204 | L | |
| 246 | G | 184 | N | 209 | G | 205 | Q | 205 | Q | |
| 247 | F | 185 | F | 210 | F | 206 | F | 206 | F | x |
| 248 | H | 186 | R | 211 | T | 207 | H | 207 | H | |
| 249 | Q | 187 | E | 212 | N | 208 | R | 208 | R | |
| 250 | F | 188 | A | 213 | M | 209 | F | 209 | F | |
| 251 | A | 189 | R | 214 | K | 210 | W | 210 | W | |
| 252 | E | 190 | Y | 215 | E | 211 | S | 211 | S | |
| 253 | F | 191 | F | 216 | F | 212 | V | 212 | V | |
| 254 | T | 192 | D | 217 | V | 213 | D | 213 | D | |
| 255 | A | 193 | I | 218 | G | 214 | D | 214 | D | |
| 256 | D | 194 | K | 219 | D | 215 | T | 215 | T | |
| 257 | D | 195 | G | 220 | D | 216 | Q | 216 | Q | |
| 258 | V | 196 | E | 221 | I | 217 | V | 217 | V | |
| 259 | G | | - | 222 | A | 218 | H | 218 | H | |
| 260 | T | | - | 223 | T | 219 | T | 219 | T | |
| 261 | A | | - | 224 | E | 220 | E | 220 | E | |
| 262 | E | 197 | Y | 225 | Y | 221 | Y | 221 | Y | |
| 263 | S | 198 | T | 226 | S | 222 | S | 222 | S | |
| 264 | G | 199 | G | 227 | A | 223 | S | 223 | S | |
| 265 | L | 200 | L | 228 | L | 224 | L | 224 | L | x |
| 266 | N | 201 | T | 229 | M | 225 | R | 225 | R | |
| 267 | S | 202 | S | 230 | S | 226 | S | 226 | S | x |
| 268 | A | 203 | K | 231 | K | 227 | I | 227 | I | |
| 269 | V | 204 | A | 232 | V | 228 | V | 228 | V | |
| 270 | L | 205 | M | 233 | V | 229 | V | 229 | V | |
| 271 | A | 206 | S | 234 | A | 230 | A | 230 | A | x |
| 272 | S | 207 | A | 235 | D | 231 | N | 231 | N | |
| 273 | N | 208 | P | 236 | G | 232 | Y | 232 | Y | |
| 274 | D | 209 | D | 237 | T | 233 | E | 233 | E | |
| 275 | E | 210 | G | 238 | L | 234 | E | 234 | E | |
| 276 | M | 211 | M | 239 | K | 235 | S | 235 | S | |
| 277 | V | 212 | I | 240 | V | 236 | I | 236 | I | |
| 278 | L | 213 | R | 241 | K | 237 | K | 237 | K | |
| 279 | L | 214 | I | 242 | F | 238 | M | 238 | M | |
| 280 | P | 215 | P | 243 | P | 239 | P | 239 | P | x |
| 281 | I | 216 | L | 244 | I | 240 | I | 240 | I | |
| 282 | N | 217 | N | 245 | N | 241 | N | 241 | N | x |
| 283 | E | 218 | E | 246 | E | 242 | E | 242 | E | x |
| 284 | P | 219 | E | 247 | P | 243 | P | 243 | P | |
| 285 | V | 220 | S | 248 | A | 244 | A | 244 | A | |
| 286 | H | | - | 249 | L | 245 | P | 245 | P | |
| 287 | G | | - | 250 | A | 246 | G | 246 | G | |
| 288 | T | 221 | S | 251 | K | 247 | K | 247 | R | |
| 289 | K | 222 | K | 252 | K | 248 | K | 248 | K | x |
| 290 | R | 223 | G | | - | | - | | | |
| 291 | K | 224 | A | 253 | K | 249 | K | 249 | K | |
| 292 | S | 225 | G | 254 | S | 250 | S | 250 | S | |
| 293 | Q | 226 | Q | 255 | Q | 251 | Q | 251 | Q | x |
| 294 | I | 227 | I | 256 | I | 252 | I | 252 | I | x |
| 295 | Q | 228 | E | 257 | D | 253 | Q | 253 | Q | |
| 296 | T | 229 | E | 258 | E | 254 | E | 254 | E | |
| 297 | Y | 230 | F | 259 | Y | 255 | Y | 255 | Y | |
| 298 | L | 231 | L | 260 | L | 256 | V | 256 | V | |
| 299 | E | 232 | M | 261 | E | 257 | D | 257 | D | |
| 300 | H | 233 | Q | 262 | F | 258 | Y | 258 | Y | |
| 301 | N | 234 | F | 263 | Y | 259 | N | 259 | N | |
| 302 | E | 235 | N | 264 | G | 260 | G | 260 | G | |
| 303 | G | 236 | G | 265 | G | 261 | G | 261 | G | x |
| 304 | A | 237 | E | 266 | A | 262 | A | 262 | A | |
| 305 | G | 238 | G | 267 | G | 263 | G | 263 | G | x |
| 306 | L | 239 | I | 268 | V | 264 | V | 264 | V | |
| 307 | Q | 240 | Q | 269 | Q | 265 | Q | 265 | Q | x |
| 308 | H | 241 | H | 270 | H | 266 | H | 266 | H | x |
| 309 | L | 242 | V | 271 | I | 267 | I | 267 | I | |
| 310 | A | 243 | A | 272 | A | 268 | A | 268 | A | x |
| 311 | L | 244 | F | 273 | L | 269 | L | 269 | L | |
| 312 | M | 245 | L | 274 | N | 270 | K | 270 | R | |
| 313 | S | 246 | T | 275 | T | 271 | T | 271 | T | |
| 314 | E | 247 | D | 276 | G | 272 | E | 272 | E | |
| 315 | D | 248 | D | 277 | D | 273 | D | 273 | D | x |
| 316 | I | 249 | L | 278 | I | 274 | I | 274 | I | |
| 317 | F | 250 | V | 279 | V | 275 | I | 275 | I | |
| 318 | R | 251 | K | 280 | E | 276 | T | 276 | T | |
| 319 | T | 252 | T | 281 | T | 277 | A | 277 | T | |
| 320 | L | 253 | W | 282 | V | 278 | I | 278 | I | |
| 321 | R | 254 | D | 283 | R | 279 | R | 279 | R | |
| 322 | E | 255 | A | 284 | T | 280 | H | 280 | H | |
| 323 | M | 256 | L | 285 | M | 281 | L | 281 | L | |
| 324 | R | 257 | K | 286 | R | 282 | R | 282 | R | |
| 325 | K | 258 | K | 287 | A | 283 | E | 283 | E | |
| 326 | R | 259 | I | 288 | A | 284 | R | 284 | R | |
| 327 | S | | - | | - | | - | | | |
| 328 | S | | - | | - | | - | | | |
| 329 | I | | - | | - | | - | | | |
| 330 | G | | - | | - | | - | | | |
| 331 | G | 260 | G | 289 | G | 285 | G | 285 | G | x |
| 332 | F | 261 | M | 290 | V | 286 | L | 286 | M | |
| 333 | D | 262 | R | 291 | Q | 287 | E | 287 | E | |
| 334 | F | 263 | F | 292 | F | 288 | F | 288 | F | x |
| 335 | M | 264 | M | 293 | L | 289 | L | 289 | L | |
| 336 | P | 265 | T | 294 | D | 290 | S | 290 | A | |
| 337 | S | 266 | A | 295 | T | 291 | V | 291 | V | |
| 338 | P | 267 | P | 296 | P | 292 | P | 292 | P | x |
| 339 | P | 268 | P | | - | | - | | | |
| 340 | P | 269 | D | 297 | D | 293 | S | 293 | S | |
| 341 | T | 270 | T | 298 | S | 294 | T | 294 | S | |
| 342 | Y | 271 | Y | 299 | Y | 295 | Y | 295 | Y | x |
| 343 | Y | 272 | Y | 300 | Y | 296 | Y | 296 | Y | x |
| 344 | Q | 273 | E | 301 | D | 297 | K | 297 | R | |
| 345 | N | 274 | M | 302 | T | 298 | Q | 298 | L | |
| 346 | L | 275 | L | 303 | L | 299 | L | 299 | L | x |
| 347 | K | 276 | E | 304 | G | 300 | R | 300 | R | |
| 348 | K | 277 | G | 305 | E | 301 | E | 301 | E | |
| 349 | R | 278 | R | 306 | W | 302 | K | 302 | N | |
| 350 | V | 279 | L | 307 | V | 303 | L | 303 | L | |
| 351 | G | 280 | P | 308 | G | 304 | K | 304 | K | |
| 352 | D | 281 | D | 309 | D | 305 | T | 305 | T | |
| 353 | V | 282 | H | 310 | T | 306 | A | 306 | S | |
| 354 | L | 283 | G | 311 | R | 307 | K | 307 | K | |
| 355 | S | 284 | E | 312 | V | 308 | I | 308 | I | |
| 356 | D | 285 | P | 313 | P | 309 | K | 309 | Q | |
| 357 | D | 286 | V | 314 | V | 310 | V | 310 | V | |
| 358 | Q | 287 | D | | - | 311 | K | 311 | K | |
| | - | 288 | Q | | - | 312 | E | 312 | E | |
| | - | 289 | L | | - | 313 | N | 313 | N | |
| 359 | I | 290 | Q | | - | 314 | I | 314 | M | |
| 360 | K | 291 | A | 315 | D | 315 | D | 315 | D | |
| 361 | E | 292 | R | 316 | T | 316 | A | 316 | V | |
| 362 | C | 293 | G | 317 | L | 317 | L | 317 | L | |
| 363 | E | 294 | I | 318 | R | 318 | E | 318 | E | |
| 364 | E | 295 | L | 319 | E | 319 | E | 319 | E | |
| 365 | L | 296 | L | 320 | L | 320 | L | 320 | L | x |
| 366 | G | 297 | D | 321 | K | 321 | K | 321 | K | |
| 367 | I | 298 | G | 322 | I | 322 | I | 322 | I | |
| 368 | L | 299 | S | 323 | L | 323 | L | 323 | L | |
| 369 | V | 300 | S | 324 | A | 324 | V | 324 | V | |
| 370 | D | 301 | V | 325 | D | 325 | D | 325 | D | |
| 371 | R | 302 | E | 326 | R | 326 | Y | 326 | Y | |
| 372 | D | 303 | G | 327 | D | 327 | D | 327 | D | |
| 373 | D | 304 | D | 328 | E | 328 | E | 328 | E | |
| 374 | Q | 305 | K | 329 | D | 329 | K | 329 | K | |
| 375 | G | 306 | R | 330 | G | 330 | G | 330 | G | |
| 376 | T | 307 | L | 331 | Y | 331 | Y | 331 | Y | |
| 377 | L | 308 | L | 332 | L | 332 | L | 332 | L | x |
| 378 | L | 309 | L | 333 | L | 333 | L | 333 | L | x |
| 379 | Q | 310 | Q | 334 | Q | 334 | Q | 334 | Q | x |
| 380 | I | 311 | I | 335 | I | 335 | I | 335 | I | x |
| 381 | F | 312 | F | 336 | F | 336 | F | 336 | F | x |
| 382 | T | 313 | S | 337 | T | 337 | T | 337 | T | |
| 383 | K | 314 | E | 338 | K | 338 | K | 338 | K | |
| 384 | P | 315 | T | 339 | P | 339 | P | 339 | P | |
| 385 | L | 316 | L | 340 | V | 340 | V | 340 | M | |
| 386 | G | 317 | M | 341 | Q | 341 | Q | 341 | Q | |
| 387 | D | 318 | G | 342 | D | 342 | D | 342 | D | |
| 388 | R | | - | 343 | R | 343 | R | 343 | R | |
| 389 | P | | - | 344 | P | 344 | P | 344 | P | |
| 390 | T | 319 | P | 345 | T | 345 | T | 345 | T | |
| 391 | I | 320 | V | 346 | V | 346 | L | 346 | L | |
| 392 | F | 321 | F | 347 | F | 347 | F | 347 | F | x |
| 393 | I | 322 | F | 348 | F | 348 | L | 348 | L | |
| 394 | E | 323 | E | 349 | E | 349 | E | 349 | E | x |
| 395 | I | 324 | F | 350 | I | 350 | V | 350 | V | |
| 396 | I | 325 | I | 351 | I | 351 | I | 351 | I | x |
| 397 | Q | 326 | Q | 352 | E | 352 | Q | 352 | Q | |
| 398 | R | 327 | R | 353 | R | 353 | R | 353 | R | x |
| 399 | V | 328 | K | 354 | H | 354 | H | 354 | H | |
| 400 | G | | - | | - | | - | | | |
| 401 | C | | - | | - | | - | | | |
| 402 | M | | - | | - | | - | | | |
| 403 | M | | - | | - | | - | | | |
| 404 | K | | - | | - | | - | | | |
| 405 | D | | - | | - | | - | | | |
| 406 | E | | - | | - | | - | | | |
| 407 | E | | - | | - | | - | | | |
| 408 | G | | - | | - | | - | | | |
| 409 | K | | - | | - | | - | | | |
| 410 | A | | - | | - | | - | | | |
| 411 | Y | | - | | - | | - | | | |
| 412 | Q | | - | | - | | - | | | |
| 413 | S | | - | | - | | - | | | |
| 414 | G | | - | | - | | - | | | |
| 415 | G | 329 | G | 355 | G | 355 | N | 355 | N | |
| 416 | C | 330 | D | 356 | S | 356 | H | 356 | H | |
| 417 | G | 331 | D | 357 | M | 357 | Q | 357 | Q | |
| 418 | G | 332 | G | 358 | G | 358 | G | 358 | G | x |
| 419 | F | 333 | F | 359 | F | 359 | F | 359 | F | x |
| 420 | G | 334 | G | 360 | G | 360 | G | 360 | G | x |
| 421 | K | 335 | E | 361 | K | 361 | A | 361 | A | |
| 422 | G | 336 | G | 362 | G | 362 | G | 362 | G | x |
| 423 | N | 337 | N | 363 | N | 363 | N | 363 | N | x |
| 424 | F | 338 | F | 364 | F | 364 | F | 364 | F | x |
| 425 | S | 339 | K | 365 | K | 365 | N | 365 | N | |
| 426 | E | 340 | A | 366 | A | 366 | S | 366 | S | |
| 427 | L | 341 | L | 367 | L | 367 | L | 367 | L | x |
| 428 | F | 342 | F | 368 | F | 368 | F | 368 | F | x |
| 429 | K | 343 | E | 369 | E | 369 | K | 369 | K | |
| 430 | S | 344 | S | 370 | A | 370 | A | 370 | A | |
| 431 | I | 345 | I | 371 | I | 371 | F | 371 | F | |
| 432 | E | 346 | E | 372 | E | 372 | E | 372 | E | x |
| 433 | E | 347 | R | 373 | R | 373 | E | 373 | E | |
| 434 | Y | 348 | D | 374 | E | 374 | E | 374 | E | |
| 435 | E | 349 | Q | 375 | Q | 375 | Q | 375 | Q | |
| 436 | K | 350 | V | 376 | E | 376 | N | 376 | A | |
| 437 | T | 351 | R | 377 | K | 377 | L | 377 | L | |
| 438 | L | 352 | R | 378 | R | 378 | R | 378 | R | |
| 439 | E | 353 | G | 379 | G | 379 | G | 379 | G | |
| 440 | A | 354 | V | 380 | N | 380 | N | 380 | N | |
| 441 | K | 355 | L | 381 | L | 381 | L | 381 | L | |
| 442 | Q | 356 | A | | | 382 | T | 382 | T | |
| 443 | L | 357 | T | | | 383 | N | 383 | D | |
| 444 | V | 358 | D | | | 384 | M | 384 | L | |
| 445 | G | | | | | 385 | E | 385 | E | |
| | | | | | | 386 | T | 386 | T | |
| | | | | | | 387 | N | 387 | N | |
| | | | | | | 388 | G | 388 | G | |
| | | | | | | 389 | V | 389 | V | |
| | | | | | | 390 | V | 390 | R | |
| | | | | | | 391 | P | 391 | S | |
| | | | | | | 392 | G | 392 | G | |
| | | | | | | 393 | M | 393 | M | |
| | | | | | | 394 | A | 394 | | |
| | | | | | | 395 | E | 395 | | |
| | | | | | | 396 | N | 396 | | |
| | | | | | | 397 | L | 397 | | |
| | | | | | | 398 | Y | 398 | | |
| | | | | | | 399 | F | 399 | | |
| | | | | | | 400 | Q | 400 | | |

A sequence analysis of more than 700 HPPD sequences from public data bases including sequences of HPPD proteins and predicted HPPD proteins such as from plants, mammals, fungi and bacteria was performed using ClustalX. The alignment was corrected using the information of the available 3D structures. Identical amino acid sequences with different identifiers were included only once and some sequences with obvious sequence errors were excluded. The alignment also includes incomplete sequences. Table 2b shows the sequence alignment for a representative set of HPPD proteins and includes sequences from plants, bacteria, mammals.

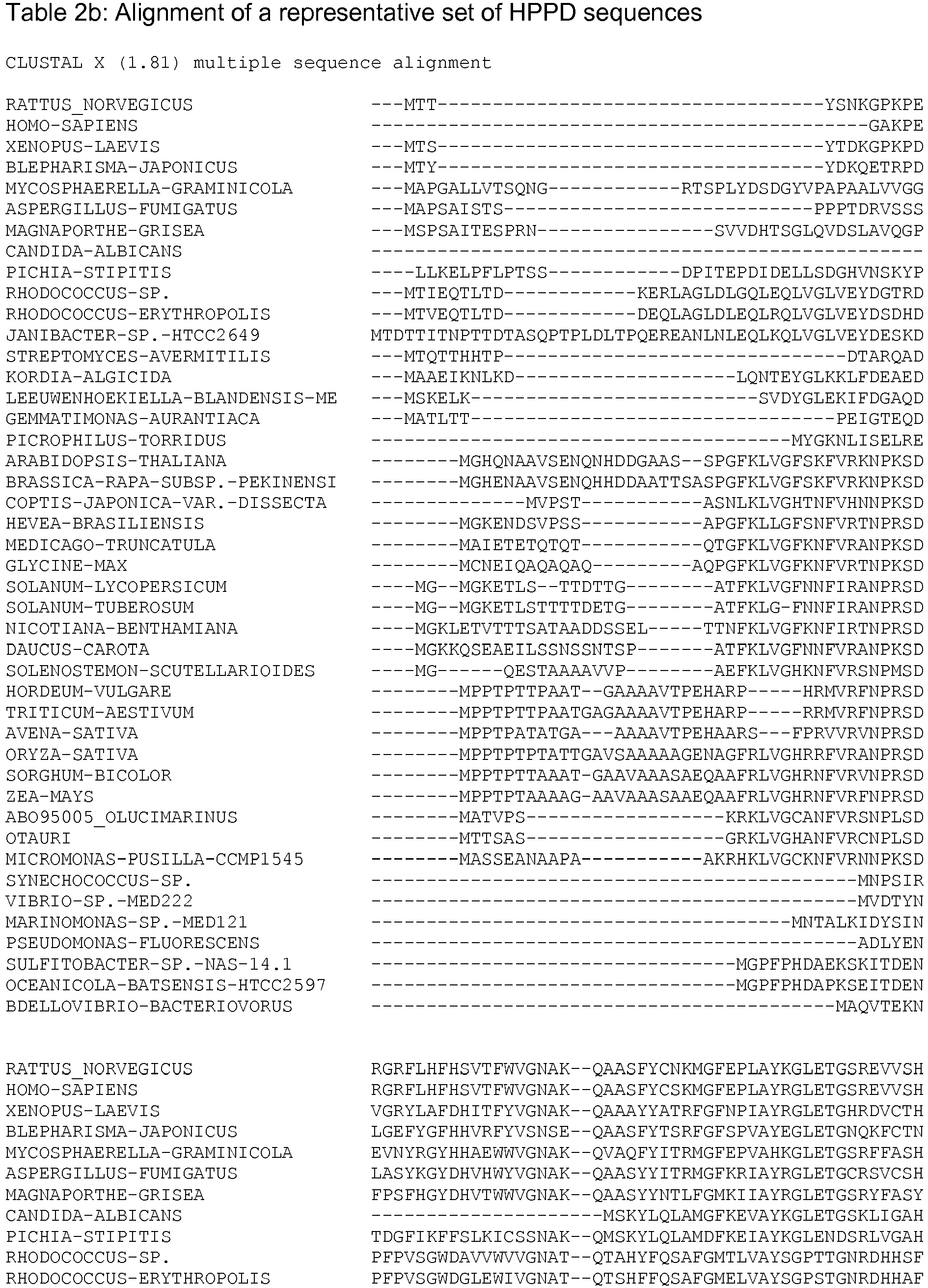

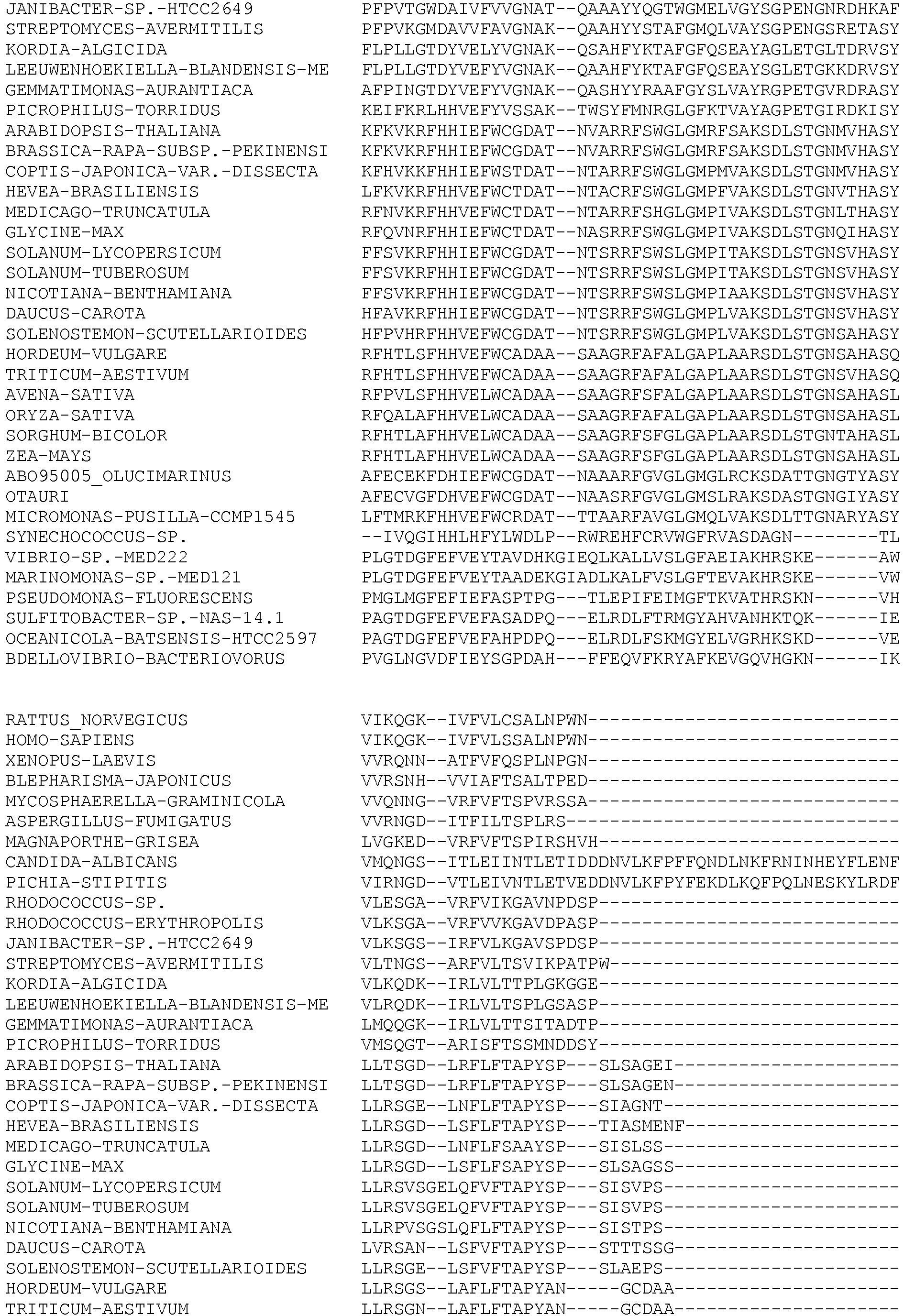

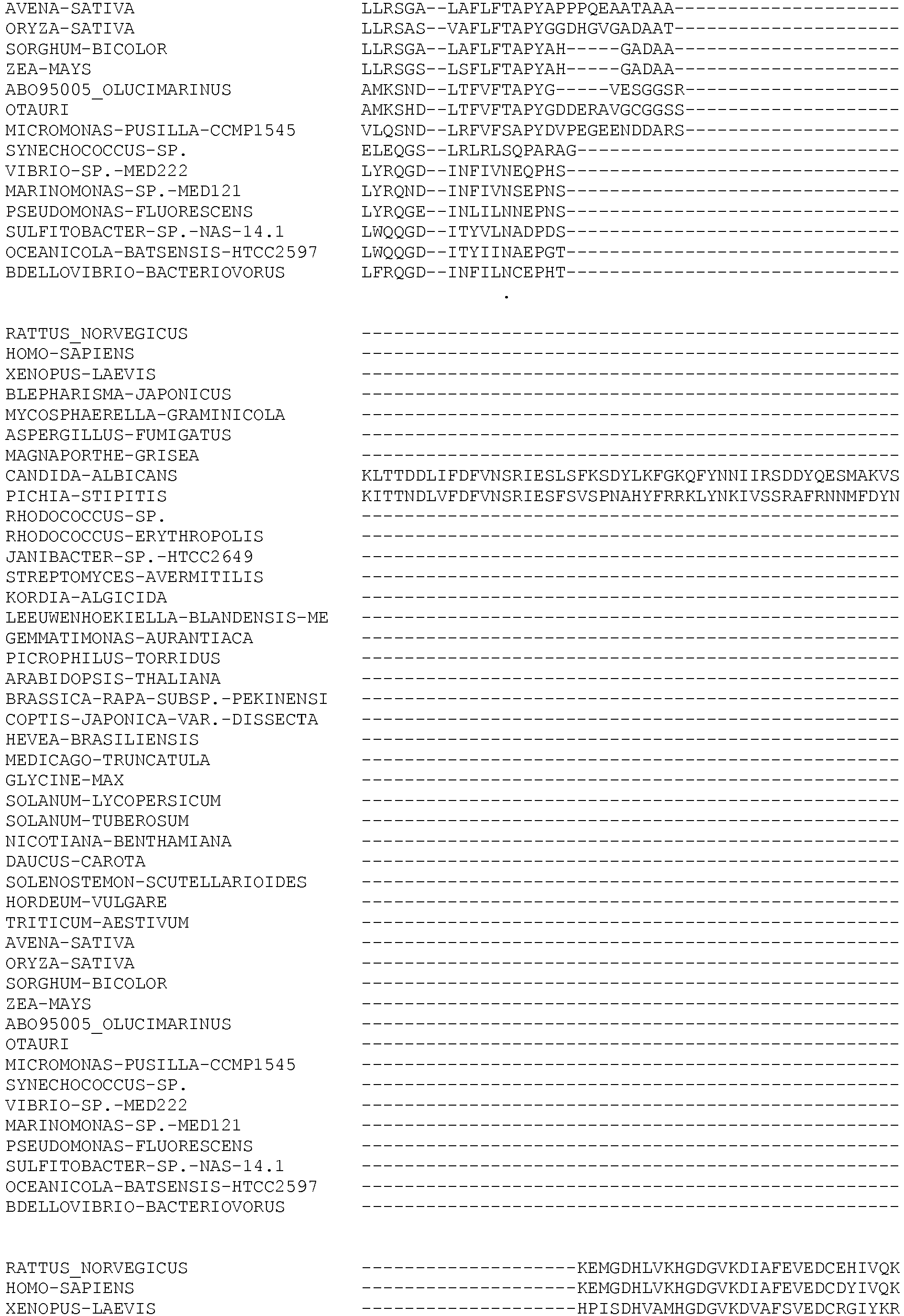

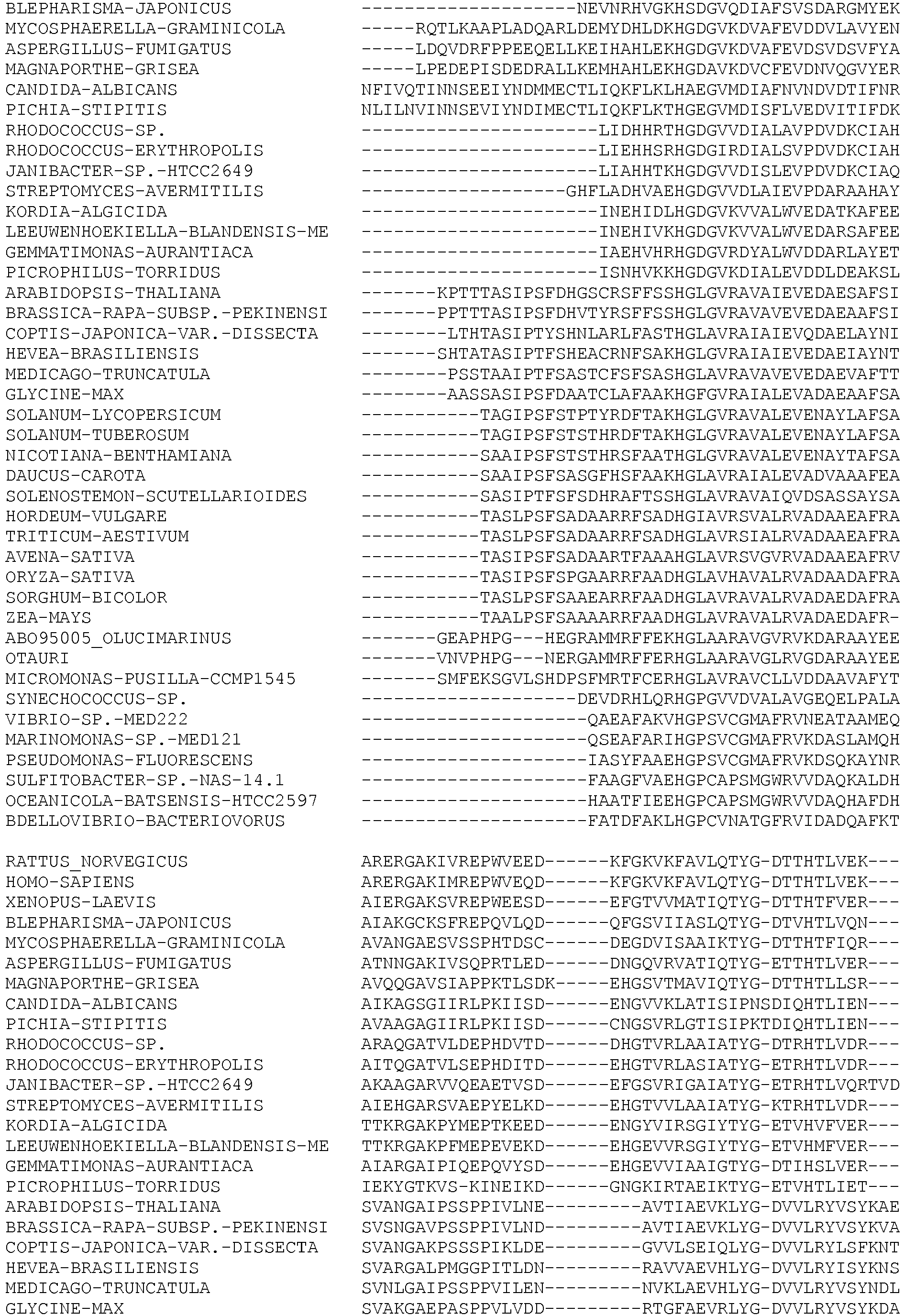

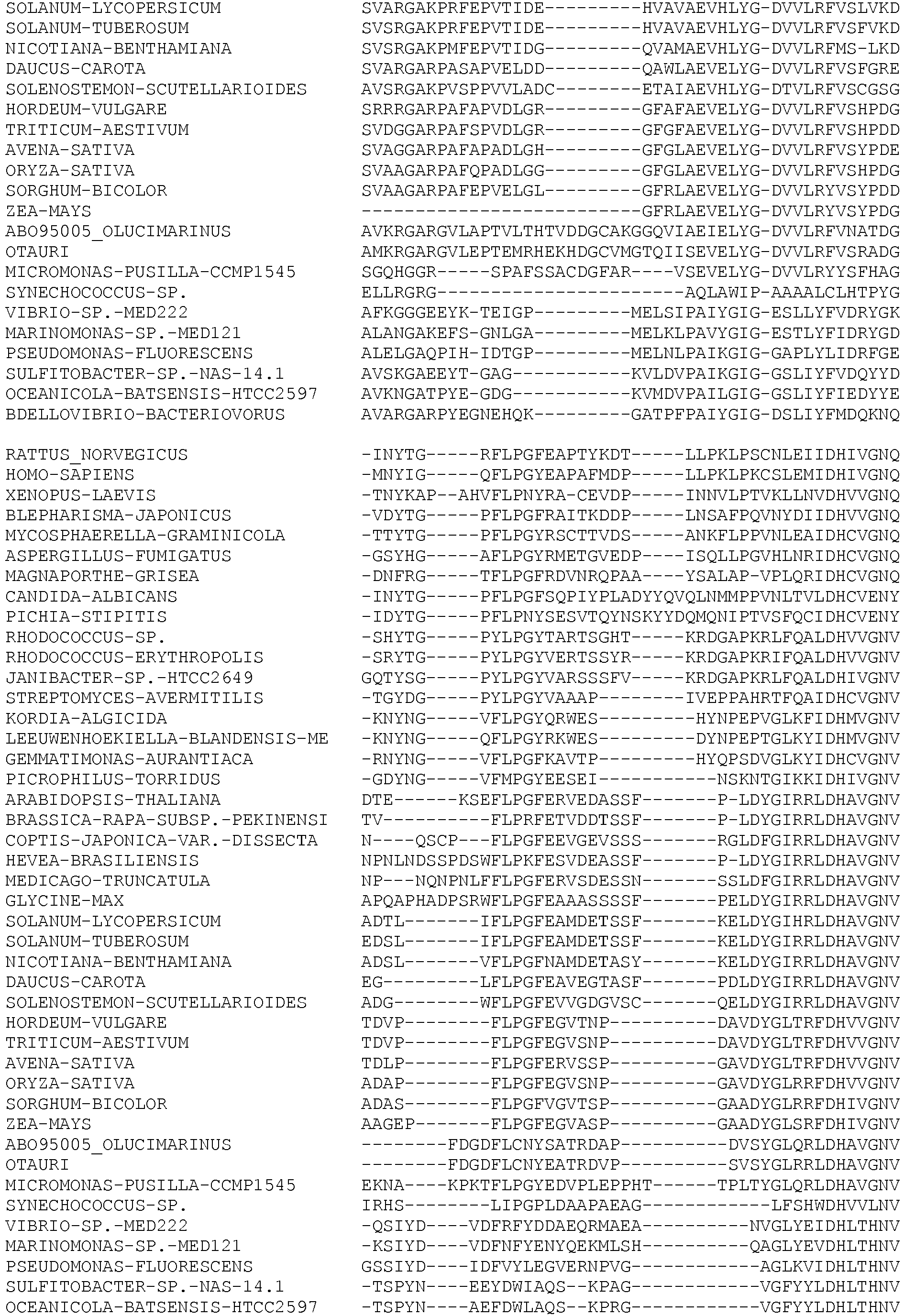

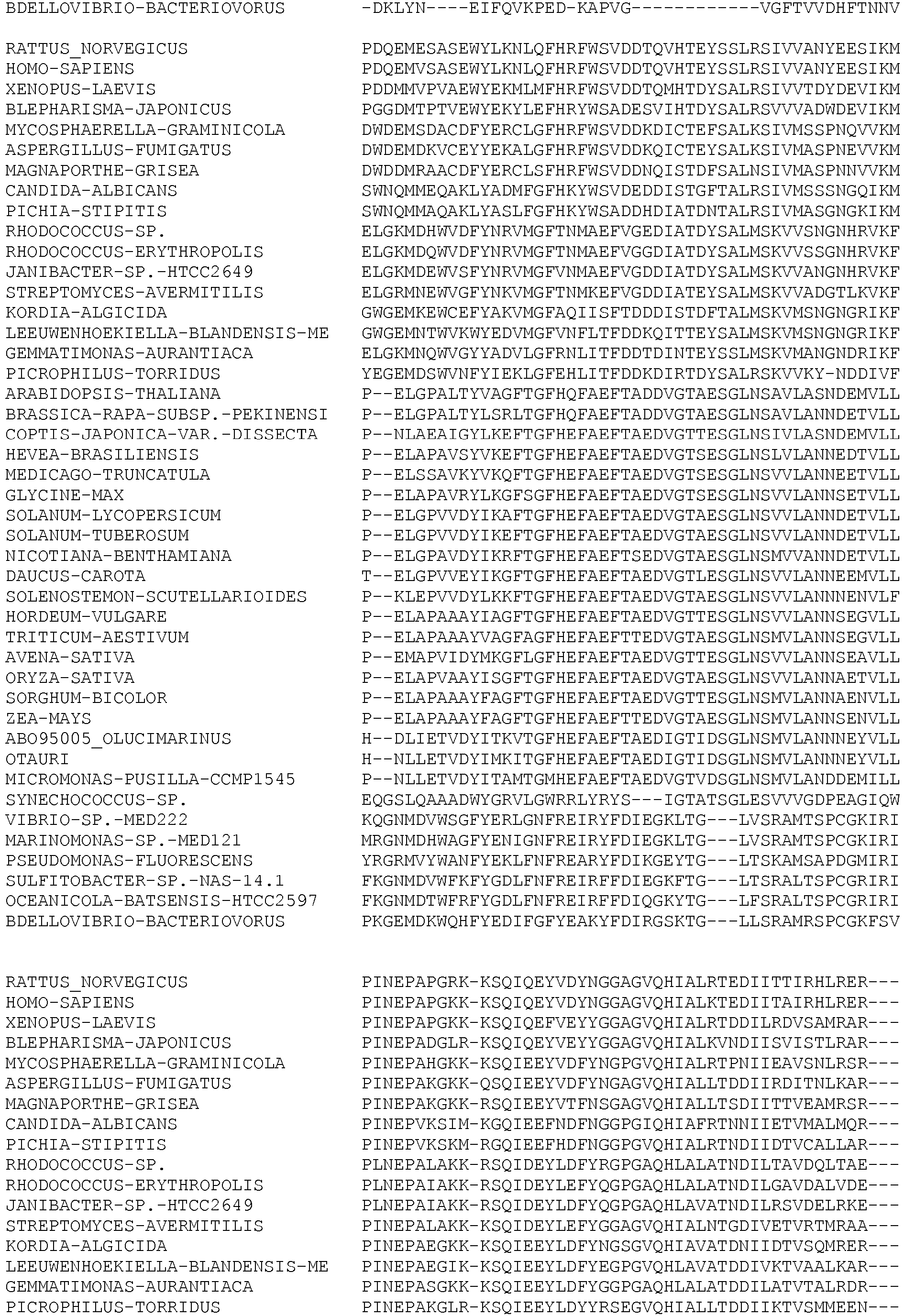

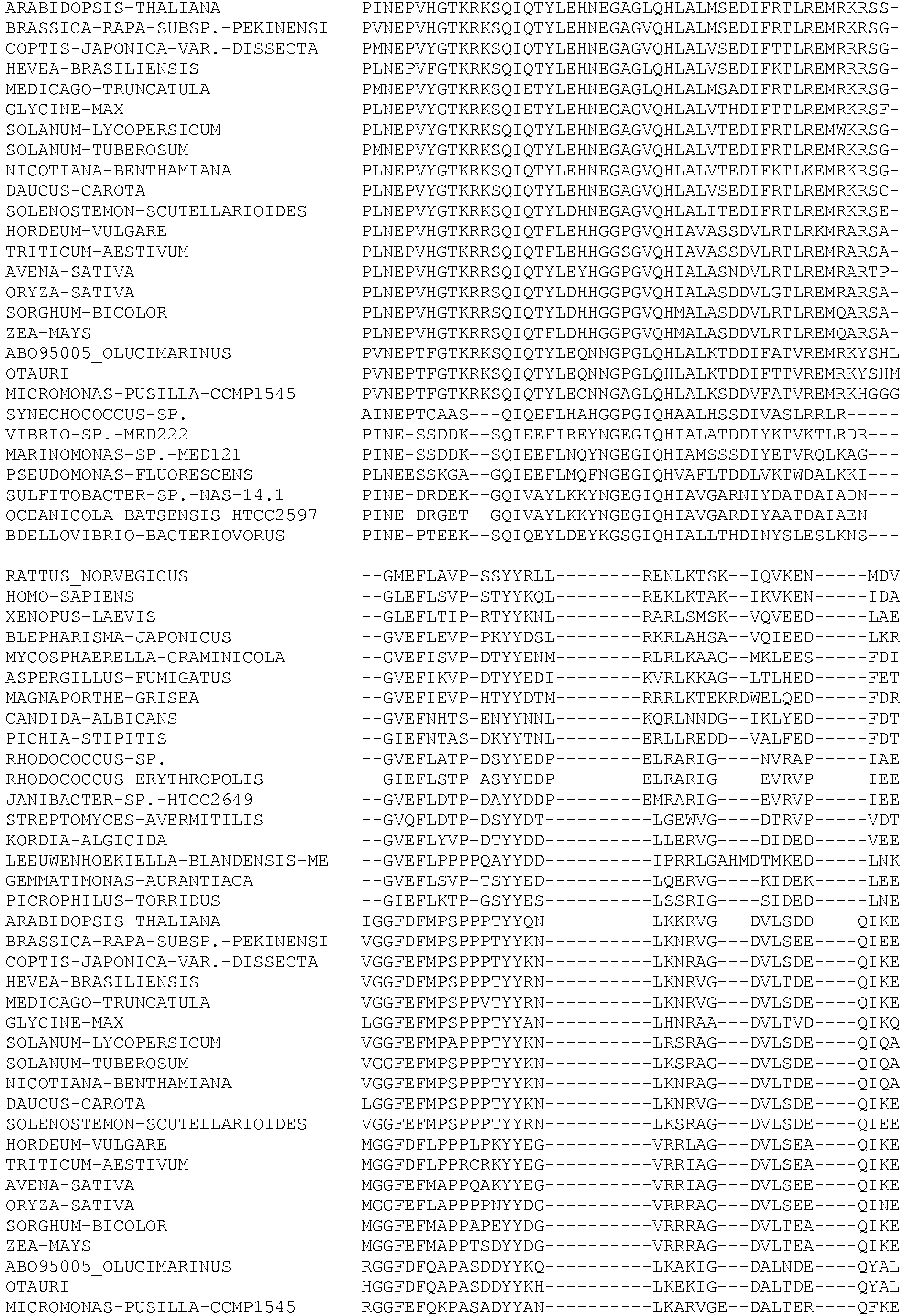

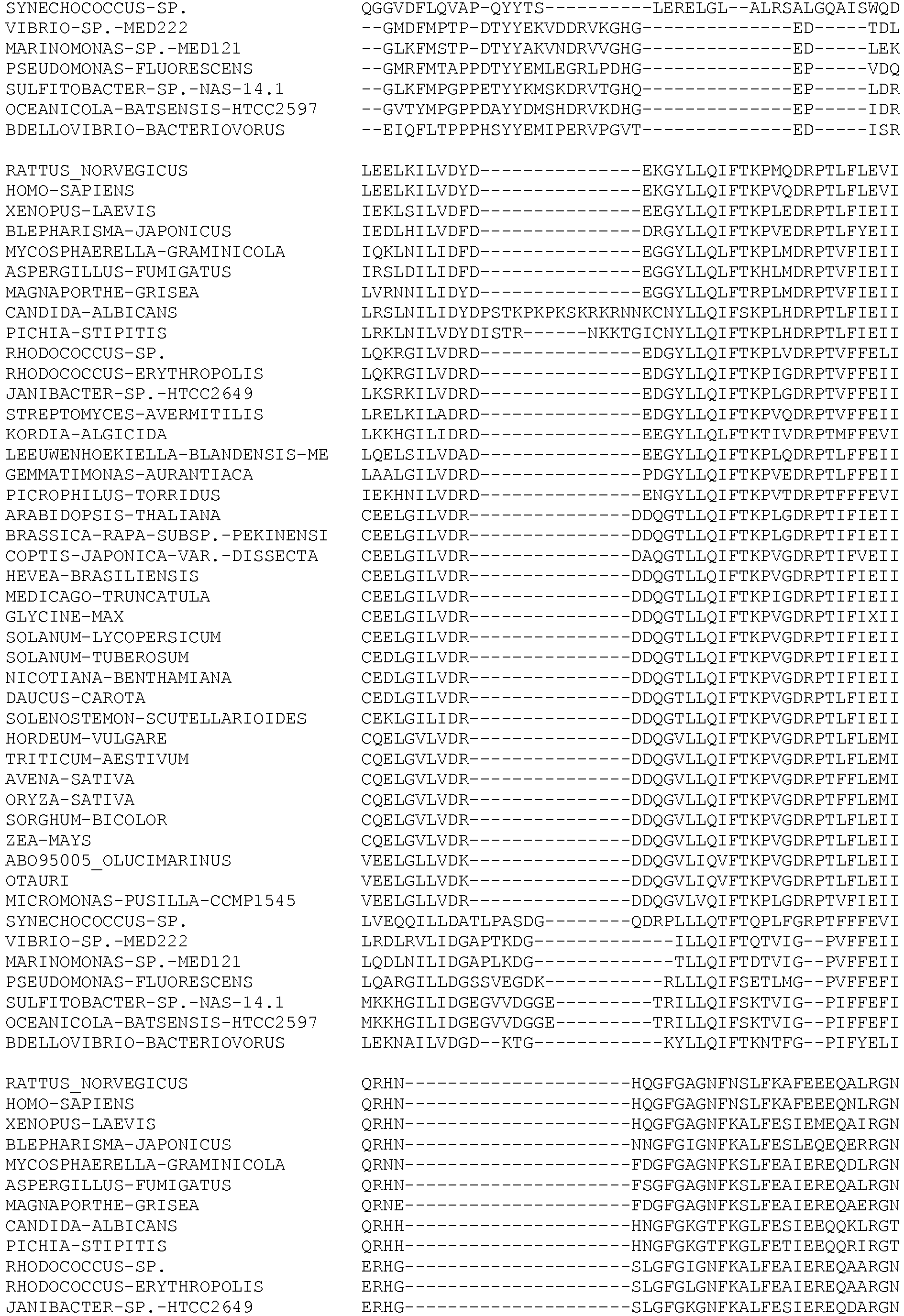

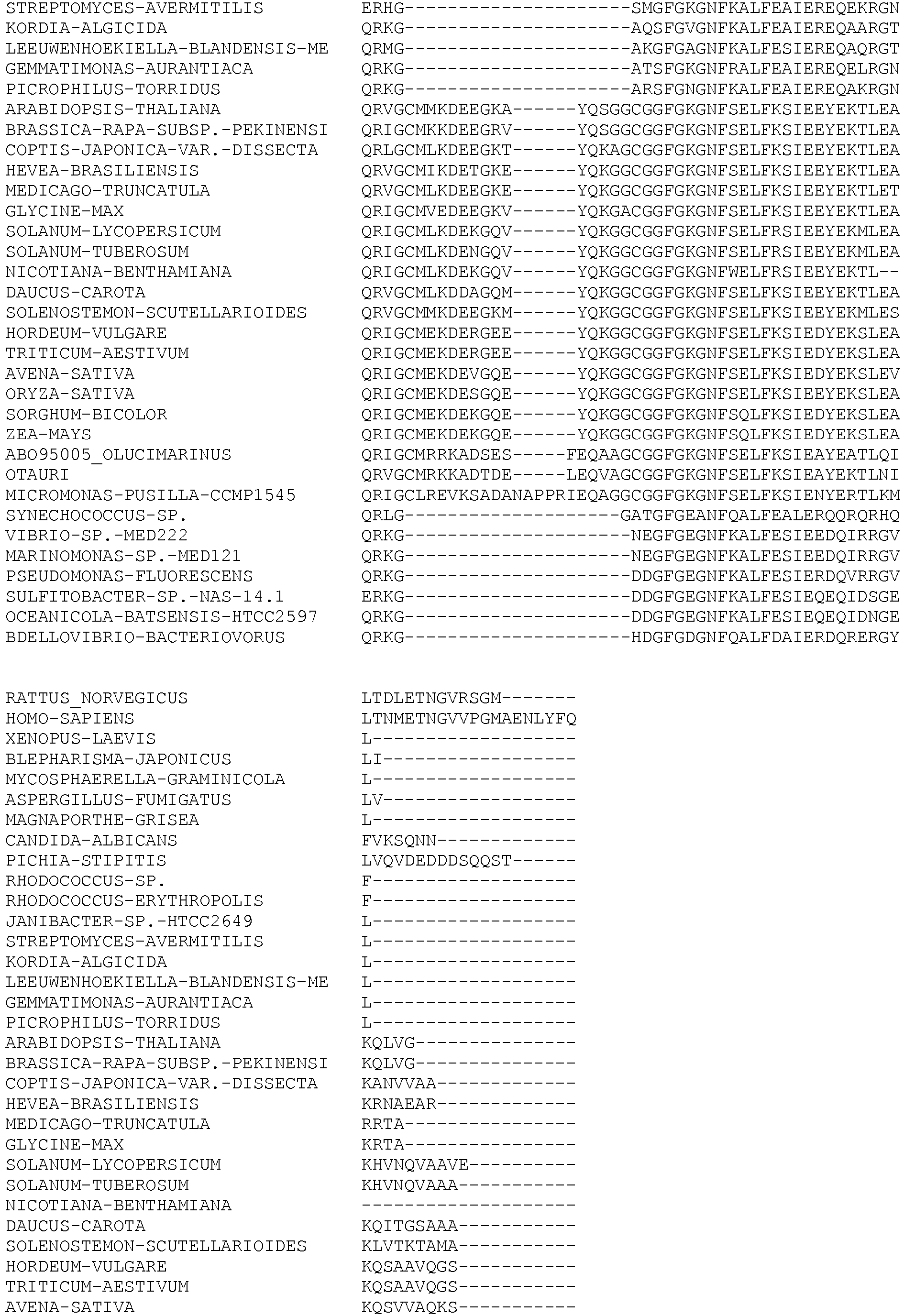

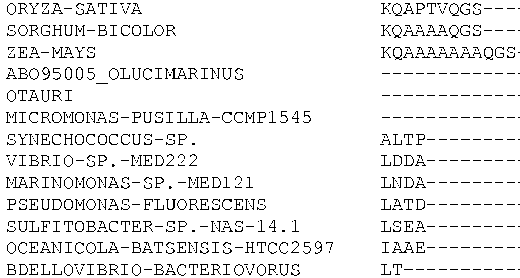

The overall sequence identity between individual full length HPPD sequences is in general quite low and is shown for the representative HHPD proteins in Table 3. Table 4a shows the sequence alignment of the binding pocket. In contrast, the sequence identity of the 36 amino acids forming the binding site is significantly higher which is shown for the representative HPPD proteins in Table 4b. In particular, the amino acids at 8 positions are strictly conserved in all species and illustrate that these amino acids have a key role (e.g. His226, His308, Glu394 binding the iron required for catalysis). These positions, with reference to the HPPD from Arabidopsis (SEQ ID No. 2) are His226, Ser267, Asn282, His308, Tyr342, Glu394, Gly420, Asn423 (Table 5a). A mutation of any of the amino acids at either of these positions will most likely lead to an inactive protein. The variability at other positions within the binding site is higher. Table 5b shows the 28 variable positions in the binding site and the amino acids which were identified at these positions using the sequence alignment. Some positions have only limited variability which reflects their role in the 3D environment. An example for this represents the position 269. All HPPD proteins have at this position either a Val, Ala or Thr. Looking at the 3D structures it seems that at this position a small apolar amino acid is required and a mutation to a polar amino acid such as Arg, His or Lys will disturb the protein structure locally. Another example represents position 379. Most sequences have a glutamine at position 379. However, there are also some bacterial sequences which have a histidine at this position. Looking at the 3D structure, it seems that only few amino acids are tolerated at this position. Gln379 in *A.thaliana* stabilizes via its H-bond donor the side chain conformation of the strictly conserved Glu394 which in turn interacts with the catalytic iron. In addition, with its H-bond acceptors Gln379 stabilizes the side chain conformation of the strictly conserved Asn423 which in turn interacts with the strictly conserved Tyr342. Only glutamine, asparagine and histidine have an H-bond donor and acceptor required for the stabilization of this particular 3D arrangement which very likely play a key role in the interaction of the C-terminal helix with the core of the HPPD protein. Limited variability is also seen at position 381 with either a phenylalanine or a tyrosine in all HPPD sequences. The aromatic ring stabilizes the binding of HPPD inhibitors and very likely also the binding of the substrate to the HPPD binding. However, the presence of the additional hydroxyl group in tyrosine compared to phenylalanine does not disturb the catalytic activity. The third category of positions includes those positions which display a very high natural variability. These positions may not be crucial for substrate binding and catalysis but influence inhibitor binding. These positions include adjacent positions in strand 248 to 255 and positions in the C-terminal helix 419-427. It can be assumed that the interaction of this particular strand and the C-terminal helix with the core of the protein plays a crucial role in inhibitor binding. Table 5b includes for each variable position those amino acids which have been identified in the sequence alignments using all known HPPD sequences.

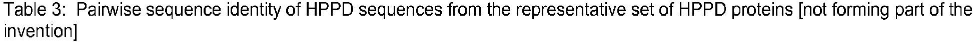

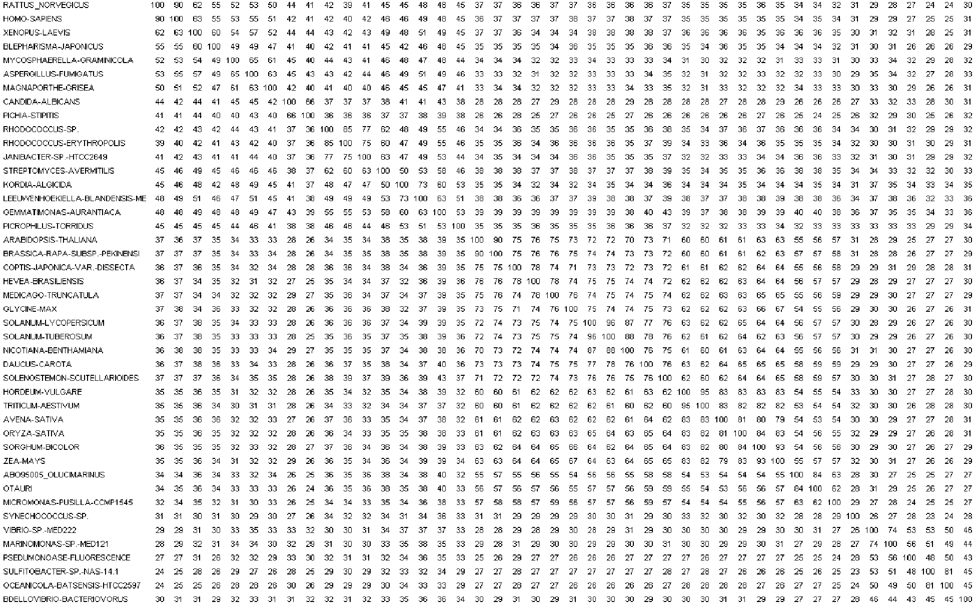

**Table 4a: Sequence alignment of amino acids forming the binding pocket in the representative set of HPPD sequences [not forming part of the invention]**

| | |
|---|---|
| HOMO-SAPIENS | HVHFWSVLSIWAPNQIQHLYLQFFEFGAGNFNSLF |
| RATTUS_NORVEGICUS | HVHFWSVLSIWAPNQIQHLYLQFFEFGAGNFNSLF |
| XENOPUS-LAEVIS | HVHFWSVLSIWTPNQIQHLYLQFFEFGAGNFKALI |
| ASPERGILLUS-FUMIGATUS | HVHFWSVLSIVMAPNQIQHIYLQFFEFGAGNFKSLI |
| MAGNAPORTHE-GRISEA | HVHFWSVLSIVMAPNQIQHIYLQFFEFGAGNFKSLI |
| MYCOSPHAERELLA-GRAMINICOLA | HVHFWSVLSIVMSPNQIQHIYLQFFEFGAGNFKSLI |
| CANDIDA-ALBICANS | HVHYWSVLSIVMSPNQIQHNYLQFFEFGKGTFKGLI |
| PICHIA-STIPITIS | HVHYWSALSIVMAPNQIQHNYLQFFEFGKGTFKGLI |
| ARABIDOPSIS-THALIANA | HVHFAEFLSAVLAPNQIQHMYLQFFEFGKGNFSELI |
| BRASSICA-RAPA-SUBSP.-PEKINENSI | HVHFAEFLSAVLAPNQIQHMYLQFFEFGKGNFSELI |
| HEVEA-BRASILIENSIS | HVHFAEFLSLVLAPNQIQHMYLQFFEFGKGNFSELI |
| COPTIS-JAPONICA-VAR.-DISSECTA | HVHFAEFLSIVLAPNQIQHMYLQFFEFGKGNFSELI |
| SOLENOSTEMON-SCUTELLARIOIDES | HVHFAEFLSVVLAPNQIQHMYLQFFEFGKGNFSELI |
| AVENA-SATIVA | HVHFAEFLSVVLAPNQIQHMYLQFFEFGKGNFSELI |
| DAUCUS-CAROTA | HVHFAEFLSVVLAPNQIQHMYLQFFEFGKGNFSELI |
| SOLANUM-TUBEROSUM | HVHFAEFLSVVLAPNQIQHMYLQFFEFGKGNFSELI |
| SOLANUM-LYCOPERSICUM | HVHFAEFLSVVLAPNQIQHMYLQFFEFGKGNFSELI |
| MEDICAGO-TRUNCATULA | HVHFAEFLSVVLAPNQIQHMYLQFFEFGKGNFSELI |
| GLYCINE-MAX | HVHFAEFLSVVLAPNQIQHMYLQFFXFGKGNFSELI |
| HORDEUM-VULGARE | HVHFAEFLSVVLAPNQIQHLYLQFFEFGKGNFSELI |
| ORYZA-SATIVA | HVHFAEFLSVVLAPNQIQHLYLQFFEFGKGNFSELI |
| SORGHUM-BICOLOR | HVHFAEFLSMVLAPNQIQHMYLQFFEFGKGNFSQLI |
| ZEA-MAYS | HVHFAEFLSMVLAPNQIQHMYLQFFEFGKGNFSQLI |
| ABO95005_OLUCIMARINUS | HVHFAEFLSMVLAPNQIQHQYLQFFEFGKGNFSELI |
| OTAURI | HVHFAEFLSMVLAPNQIQHQYLQFFEFGKGNFSELI |
| MICROMONAS-PUSILLA-CCMP1545 | HVHFAEFLSMVLAPNQIQHQYLQFFEFGKGNFSELI |
| TRITICUM-AESTIVUM | HVHFAEFLSMVLAPNQIQHLYLQFFEFGKGNFSELI |
| NICOTIANA-BENTHAMIANA | HVHFAEFLSMVVAPNQIQHMYLQFFEFGKGNFWELI |
| RHODOCOCCUS-SP. | HVTMAEFLSKVVSPNQIQHLYLQFFEFGIGNFKALI |
| RHODOCOCCUS-ERYTHROPOLIS | HVTMAEFLSKVVSPNQIQHLYLQFFEFGLGNFKALI |
| STREPTOMYCES-AVERMITILIS | HVTMKEFLSKVVAPNQIQHLYLQFFEFGKGNFKALI |
| JANIBACTER-SP.-HTCC2649 | HVVMAEFLSKVVAPNQIQHLYLQFFEFGKGNFKALI |
| KORDIA-ALGICIDA | HVAIISFLSKVMSPNQIQHLYLQFFEFGVGNFKALI |
| LEEUWENHOEKIELLA-BLANDENSIS-ME | HVVFLTFLSKVMSPNQIQHLYLQFFEFGAGNFKALI |
| PICROPHILUS-TORRIDUS | HVELITFLSKVVKPNQIQHLYLQFFEFGNGNFKALI |
| GEMMATIMONAS-AURANTIACA | HVRLITFLSKVMAPNQIQHLYLQFFEFGKGNFRALI |
| VIBRIO-SP.-MED222 | HTRIRYFLSRAMTPNQIQHMYLQFFEFGEGNFKALI |
| MARINOMONAS-SP.-MED121 | HTRIRYFLSRAMTPNQIQHMYLQFFEFGEGNFKALI |
| SULFITOBACTER-SP.-NAS-14.1 | HTRIRFFLSRALTPNQIQHMYLQFFEFGEGNFKALI |
| OCEANICOLA-BATSENSIS-HTCC2597 | HTRIRFFLSRALTPNQIQHMYLQFFEFGEGNFKALI |
| PSEUDOMONAS-FLUORESCENS | HTRARYFLSKAMSPNQIQHMYLQFFEFGEGNFKALI |
| BDELLOVIBRIO-BACTERIOVORUS | HTYAKYFLSRAMRPNQIQHLYLQFFEFGDGNFQALI |
| BLEPHARISMA-JAPONICUS | HVHYWSALSWVAPNQIQHLYLQFFEFGIGNFKALL |
| SYNECHOCOCCUS-SP. | HVRLYRYLSVVVGANQIQHLYLQFFEFGEANFQALL |

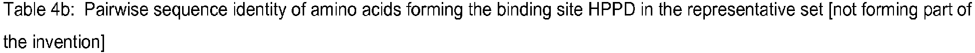

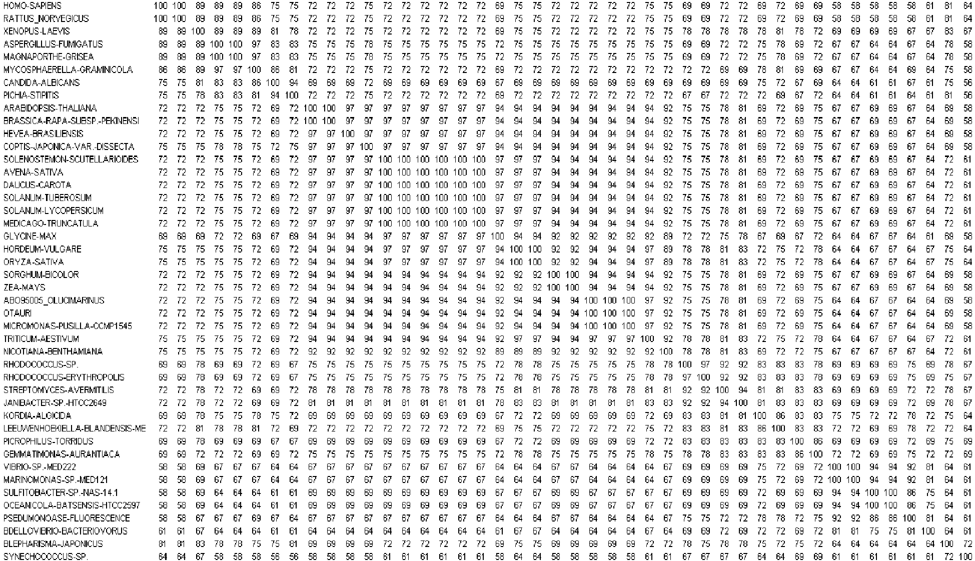

**Table 5a: Amino acid positions which are strictly conserved shown for the crystal structures**

| *Arabidopsis thaliana* | | *Pseudomonas fluorescens* | | *Streptomyces avermitilis* | | *Homos apiens* | | *Rattus norvegicus* | |
|---|---|---|---|---|---|---|---|---|---|
| Position | Amino Acid | Position | Amino Acid | Position | Amino Acid | Position | Amino Acid | Position | Amino Acid |
| 226 | H | 162 | H | 187 | H | 183 | H | 183 | H |
| 267 | S | 202 | S | 230 | S | 226 | S | 226 | S |
| 282 | N | 217 | N | 245 | N | 241 | N | 241 | N |
| 308 | H | 241 | H | 270 | H | 266 | H | 266 | H |
| 342 | Y | 271 | Y | 299 | Y | 295 | Y | 295 | Y |
| 394 | E | 323 | E | 349 | E | 349 | E | 349 | E |
| 420 | G | 334 | G | 360 | G | 360 | G | 360 | G |
| 423 | N | 337 | N | 363 | N | 363 | N | 363 | N |

**Table 5b: Amino acid position with the amino acids at this positions in the known crystal structures and the amino acids a these position within all HPPD sequences**

| *Arabidopsis thaliana* | | *Pseudomonas fluorescens* | | *Streptomyces avermitilis* | | *Homo sapiens* | | *Rattus norvegicus* | | Natural Variabaility |
|---|---|---|---|---|---|---|---|---|---|---|
| Pos. | Amino Acid | Pos. | Amino Acid | Pos. | Amino Acid | Pos. | Amino Acid | Pos. | Amino Acid | Amino Acid |
| 228 | V | 164 | T | 189 | V | 185 | V | 185 | V | VTCAG |
| 248 | H | 186 | R | 211 | T | 207 | H | 207 | H | HRQTKEYLAGSN |
| 250 | F | 188 | A | 213 | M | 209 | F | 209 | F | FYILVAQEDGTSMRK |
| 251 | A | 189 | R | 214 | K | 210 | W | 210 | W | AWILSRKHDEPGNY |
| 252 | E | 190 | Y | 215 | E | 211 | S | 211 | S | ESTYFHQNGLMVILIR |
| 253 | F | 191 | F | 216 | F | 212 | V | 212 | V | FVIALWMQHY |
| 265 | L | 200 | L | 228 | L | 224 | L | 224 | L | LMVIA |
| 268 | A | 203 | K | 231 | K | 227 | I | 227 | I | AVLMIKRQY |
| 269 | V | 204 | A | 232 | V | 228 | V | 228 | V | VAT |
| 270 | L | 205 | M | 233 | V | 229 | V | 229 | V | LVMIA |
| 271 | A | 206 | S | 234 | A | 230 | A | 230 | A | ASTVRKELIMHG |
| 280 | P | 215 | P | 243 | P | 239 | P | 239 | P | PAVTNI |
| 293 | Q | 226 | Q | 255 | Q | 251 | Q | 251 | Q | QLAVRSGVFM |
| 294 | I | 227 | I | 256 | I | 252 | I | 252 | I | IMVTASP |
| 307 | Q | 240 | Q | 269 | Q | 265 | Q | 265 | Q | QHN |
| 335 | M | 264 | M | 293 | L | 289 | L | 289 | L | MLIN |
| 368 | L | 295 | L | 323 | L | 323 | L | 323 | L | LM |
| 379 | Q | 310 | Q | 334 | Q | 334 | Q | 334 | Q | QH |
| 381 | F | 312 | F | 336 | F | 336 | F | 336 | F | FY |
| 392 | F | 321 | F | 347 | F | 347 | F | 347 | F | FS |
| 419 | F | 333 | F | 359 | F | 359 | F | 359 | F | FY |
| 421 | K | 335 | E | 361 | K | 361 | A | 361 | A | KQAILVNDEGS |
| 422 | G | 336 | G | 362 | G | 362 | G | 362 | G | GAPVTM |
| 424 | F | 338 | F | 364 | F | 364 | F | 364 | F | FAVIL |
| 425 | S | 339 | K | 365 | K | 365 | N | 365 | N | SNKGRAPSIKQR |
| 426 | E | 340 | A | 366 | A | 366 | S | 366 | S | EQSAIVFT |
| 427 | L | 341 | L | 367 | L | 367 | L | 367 | L | LR |
| 431 | I | 345 | I | 371 | I | 371 | F | 371 | F | IFVMLQR |

**Table 5c: Amino acid position with the amino acids at this positions in the known crystal structures and most common amino acids a these position within all HPPD sequences**

| Arabidopsis thaliana | | Pseudomonas fluorescens | | Streptomyces avermitilis | | Homo sapiens | | Rattus norvegicus | | Most common |
|---|---|---|---|---|---|---|---|---|---|---|
| Pos. | Amino Acid | Pos. | Amino Acid | Pos. | Amino Acid | Pos. | Amino Acid | Pos. | Amino Acid | Amino Acid |
| 228 | V | 164 | T | 189 | V | 185 | V | 185 | V | VTCA |
| 248 | H | 186 | R | 211 | T | 207 | H | 207 | H | HRQTKEYA |
| 250 | F | 188 | A | 213 | M | 209 | F | 209 | F | FYILVAEDTM |
| 251 | A | 189 | R | 214 | K | 210 | W | 210 | W | AWILRKHDEPY |
| 252 | E | 190 | Y | 215 | E | 211 | S | 211 | S | ESTRY |
| 253 | F | 191 | F | 216 | F | 212 | V | 212 | V | FVIALWY |
| 265 | L | 200 | L | 228 | L | 224 | L | 224 | L | LMVA |
| 268 | A | 203 | K | 231 | K | 227 | I | 227 | I | AVLMIKRQY |
| 269 | V | 204 | A | 232 | V | 228 | V | 228 | V | VA |
| 270 | L | 205 | M | 233 | V | 229 | V | 229 | V | LVMI |
| 271 | A | 206 | S | 234 | A | 230 | A | 230 | A | ASTVREGK |
| 280 | P | 215 | P | 243 | P | 239 | P | 239 | P | PAVT |
| 293 | Q | 226 | Q | 255 | Q | 251 | Q | 251 | Q | QLA |
| 294 | I | 227 | I | 256 | I | 252 | I | 252 | I | IMVA |
| 307 | Q | 240 | Q | 269 | Q | 265 | Q | 265 | Q | QHN |
| 335 | M | 264 | M | 293 | L | 289 | L | 289 | L | MLI |
| 368 | L | 295 | L | 323 | L | 323 | L | 323 | L | LM |
| 379 | Q | 310 | Q | 334 | Q | 334 | Q | 334 | Q | QH |
| 381 | F | 312 | F | 336 | F | 336 | F | 336 | F | FY |
| 392 | F | 321 | F | 347 | F | 347 | F | 347 | F | F |
| 419 | F | 333 | F | 359 | F | 359 | F | 359 | F | FY |
| 421 | K | 335 | E | 361 | K | 361 | A | 361 | A | KQAIVLDE |
| 422 | G | 336 | G | 362 | G | 362 | G | 362 | G | GA |
| 424 | F | 338 | F | 364 | F | 364 | F | 364 | F | FAVL |
| 425 | S | 339 | K | 365 | K | 365 | N | 365 | N | SNKRAIQ |
| 426 | E | 340 | A | 366 | A | 366 | S | 366 | S | ESAIVF |
| 427 | L | 341 | L | 367 | L | 367 | L | 367 | L | LR |
| 431 | I | 345 | I | 371 | I | 371 | F | 371 | F | IFVMQRL |

Not all amino acids occurring at the variable positions have the same likelihood to be present in an active protein. In some cases most sequences have the same amino acids at a particular position while other amino acids are present at that position in only few HPPD sequences. An example is position 392. Most sequences have at a corresponding position a phenylalanine while few i.e. the *burkholderia* sequences have a serine. Whereas in some rare cases, the rare amino acids may be the result of a sequencing error, in most other cases the resulting protein is active. Table 5c shows a list with the most common amino acids at the variable positions.

The influence of the amino acids at the variable positions is different. Some of these positions are crucial for catalysis and/or the interaction of HPPD with an inhibitor while others may have less impact. For instances changes at positions 269 and 280 which are in direct contact with the inhibitor and the substrate will very likely have a great impact upon catalysis and inhibitor binding. Also modifications at positions involved in the helix movement induced by inhibitor or substrate binding such as positions 252, 421 and 422 are likely to have high impact on inhibitor binding. In contrast modifications at positions such as 293 are less likely to influence inhibitor binding because this position is quite distant from the active site. Table 6a shows the position with very high impact and Table 6b with high impact on activity and inhibitor binding.

From these observations, the present inventors drew the conclusion that amino acids are preferred in the binding sites which naturally occur at the corresponding positions. This means, that those modifications which exchange a naturally occurring amino acid into another are likely providing a catalytically active HPPD protein which may exert a modified or even increased tolerance to HPPD herbicide inhibitors. Even more promising are those mutant proteins which have the amino acid most often found in nature at the variable position chosen from Table 5c.

**Table 6a: Amino acid positions with high priority shown for the x-ray structures [not forming part of the invention]**

| *Arabidopsis thaliana* | | *Pseudomonas fluorescens* | | *Streptomyces avermitilis* | | *Homo sapiens* | | *Rattus norvegicus* | |
|---|---|---|---|---|---|---|---|---|---|
| Pos. | Amino Acid | Pos. | Amino Acid | Pos. | Amino Acid | Pos. | Amino Acid | Pos. | Amino Acid |
| 228 | V | 164 | T | 189 | V | 185 | V | 185 | V |
| 250 | F | 188 | A | 213 | M | 209 | F | 209 | F |
| 251 | A | 189 | R | 214 | K | 210 | W | 210 | W |
| 252 | E | 190 | Y | 215 | E | 211 | S | 211 | S |
| 253 | F | 191 | F | 216 | F | 212 | V | 212 | V |
| 265 | L | 200 | L | 228 | L | 224 | L | 224 | L |
| 268 | A | 203 | K | 231 | K | 227 | I | 227 | I |
| 269 | V | 204 | A | 232 | V | 228 | V | 228 | V |
| 270 | L | 205 | M | 233 | V | 229 | V | 229 | V |
| 271 | A | 206 | S | 234 | A | 230 | A | 230 | A |
| 280 | P | 215 | P | 243 | P | 239 | P | 239 | P |
| 307 | Q | 240 | Q | 269 | Q | 265 | Q | 265 | Q |
| 335 | M | 264 | M | 293 | L | 289 | L | 289 | L |
| 368 | L | 295 | L | 323 | L | 323 | L | 323 | L |
| 379 | Q | 310 | Q | 334 | Q | 334 | Q | 334 | Q |
| 392 | F | 321 | F | 347 | F | 347 | F | 347 | F |
| 421 | K | 335 | E | 361 | K | 361 | A | 361 | A |
| 422 | G | 336 | G | 362 | G | 362 | G | 362 | G |
| 426 | E | 340 | A | 366 | A | 366 | S | 366 | S |
| 427 | L | 341 | L | 367 | L | 367 | L | 367 | L |

**Table 6b: Amino acid positions with very high priority shown for the x-ray structures [not forming part of the invention]**

| *Arabidopsis thaliana* | | *Pseudomonas fluorescens* | | *Streptomyces avermitilis* | | *Homo sapiens* | | *Rattus norvegicus* | |
|---|---|---|---|---|---|---|---|---|---|
| Pos. | Amino Acid | Pos. | Amino Acid | Pos. | Amino Acid | Pos. | Amino Acid | Pos. | Amino Acid |
| 252 | E | 190 | Y | 215 | E | 211 | S | 211 | S |
| 269 | V | 204 | A | 232 | V | 228 | V | 228 | V |
| 280 | P | 215 | P | 243 | P | 239 | P | 239 | P |
| 335 | M | 264 | M | 293 | L | 289 | L | 289 | L |
| 368 | L | 295 | L | 323 | L | 323 | L | 323 | L |
| 421 | K | 335 | E | 361 | K | 361 | A | 361 | A |
| 422 | G | 336 | G | 362 | G | 362 | G | 362 | G |

In another embodiment, in the isolated nucleic acid of the invention as defined above, said amino acid is selected from
Ala, Asp, Glu, Phe, Thr, Val or Tyr at a position in an HPPD protein, said position corresponding to position 250 of the amino acid sequence of SEQ ID No. 2;
Various sequences of HPPD proteins or predicted HPPD proteins are known in the art. These include the HPPD sequences of Streptomyces avermitilis (Genebank SAV11864), Daucus carota (Genebank DCU 87257), Arabidopsis thaliana (Genebank AF047834), Mycosphaerella graminicola (Genebank AF038152), oryza sativa / rice [BAD26248], Zea mays / corn [ACN36372], avena sativa [ABZ23427], Pseudomonas fluorescens [ABF50055], Synechococcus sp. [YP_473959], Blepharisma japonicum [BAF91881], Rhodococcus RHA1 sp. ro0240 [YP_702005], Rhodococcus RHA1 sp. ro0341 [YP_703002], Picrophilus torridus [YP_024147], Kordia algicida [ZP_02161490], Sorghum bicolor [XP_002453359], Triticum aestivum / wheat [AAZ67144], or Hordeum vulgare / barley [O48604].

The sequence of the HPPD protein taken as a starting point may be any amino acid sequence encoding a catalytically active HPPD protein. In one embodiment of the isolated nucleic acid of the invention, said HPPD protein comprises the amino acid sequence of SEQ ID No. 4 [Oryza sativa], wherein the resulting amino acid sequence comprises an amino acid selected from Ala, Asp, Glu, Gly, Lys, Gln, Arg, Ser, Thr, Val, or Tyr at position 247 of the amino acid sequence of SEQ ID No. 4 (corresponding to position 250 of SEQ ID No. 2);
In another embodiment of the isolated nucleic acid of the invention, said HPPD protein comprises the amino acid sequence of SEQ ID No. 6 [Zea mays], wherein the resulting amino acid sequence comprises an amino acid selected from Ala, Asp, Glu, Gly, Lys, Gln, Arg, Ser, Thr, Val, or Tyr at position 224 of the amino acid sequence of SEQ ID No. 6 (corresponding to position 250 of SEQ ID No. 2);
In another embodiment of the isolated nucleic acid of the invention, said HPPD protein comprises the amino acid sequence of SEQ ID No. 8 [Avena sativa], wherein the resulting amino acid sequence comprises an amino acid selected from Ala, Asp, Glu, Gly, Lys, Gln, Arg, Ser, Thr, Val, Tyr, or Tyr at position 241 of the amino acid sequence of SEQ ID No. 8 (corresponding to position 250 of SEQ ID No. 2), preferably Ala ;
In another embodiment, of the isolated nucleic acid of the invention, said HPPD protein comprises the amino acid sequence of SEQ ID No. 10 [Pseudomonas fluorescens], wherein the resulting amino acid sequence comprises anamino acid selected from Phe, Asp, Glu, Gly, Lys, Gln, Arg, Ser, Thr, Val, or Tyr at position 188 of the amino acid sequence of SEQ ID No. 10 (corresponding to position 250 of SEQ ID No. 2),
In another embodiment of the isolated nucleic acid of the invention, said HPPD protein comprises the amino acid sequence of SEQ ID No. 14 [Synechococcus sp.], wherein the resulting amino acid sequence comprises an amino acid selected from Ala, Asp, Glu, Phe, Gly, Lys, Gln, Arg, Ser, Thr, Val, or Tyr at position 169 of the amino acid sequence of SEQ ID No. 14 (corresponding to position 250 of SEQ ID No. 2), preferably Ala or Phe;
In another embodiment of the isolated nucleic acid of the invention, said HPPD protein comprises the amino acid sequence of SEQ ID No. 16 [Blepharisma japonicum], wherein the resulting amino acid sequence comprises an amino acid selected from Ala, Asp, Glu, Phe, Gly, Lys, Gln, Arg, Ser, Thr, or Val at position 209 of the amino acid sequence of SEQ ID No. 16 (corresponding to position 250 of SEQ ID No. 2), preferably Ala or Phe;
In another embodiment, of the isolated nucleic acid of the invention, said HPPD protein comprises the amino acid sequence of SEQ ID No. 18 [Rhodococcus RHA1 sp. ro0240], wherein the resulting amino acid sequence comprises an amino acid selected from Ala, Asp, Glu, Phe, Gly, Lys, Gln, Arg, Ser, Thr, Val, or Tyr at position 231 of the amino acid sequence of SEQ ID No. 18 (corresponding to position 250 of SEQ ID No. 2), preferably Phe or Ala;
In another embodiment, of the isolated nucleic acid of the invention, said HPPD protein comprises the amino acid sequence of SEQ ID No. 30 [Rhodococcus RHA1 sp. 0341], wherein the resulting amino acid sequence comprises an amino acid selected from Ala, Asp, Glu, Phe, Gly, Lys, Gln, Arg, Ser, Thr, Val, or Tyr at position 232 of the amino acid sequence of SEQ ID No. 30 (corresponding to position 250 of SEQ ID No. 2), preferably Phe or Ala;
In another embodiment, of the isolated nucleic acid of the invention, said HPPD protein comprises the amino acid sequence of SEQ ID No. 20 [Picrophilus torridus], wherein the resulting amino acid sequence comprises an amino acid selected from Ala, Asp, Glu, Phe, Gly, Lys, Gln, Arg, Ser, Thr, Val, or Tyr at position 201 of the amino acid sequence of SEQ ID No. 20 (corresponding to position 250 of SEQ ID No. 2), preferably Phe or Ala;
In another embodiment, of the isolated nucleic acid of the invention, said HPPD protein comprises the amino acid sequence of SEQ ID No. 22 [Kordia algicida], wherein the resulting amino acid sequence comprises an amino acid selected from Ala, Asp, Glu, Phe, Gly, Lys, Gln, Arg, Ser, Thr, Val, or Tyr at position 219 of the amino acid sequence of SEQ ID No. 22 (corresponding to position 250 of SEQ ID No. 2), preferably Phe or Ala;
In another embodiment, of the isolated nucleic acid of the invention, said HPPD protein comprises the amino acid sequence of SEQ ID No. 24 [Sorghum bicolor], wherein the resulting amino acid sequence comprises an amino acid selected from Ala, Asp, Glu, Gly, Lys,Gln, Arg, Ser, Thr, Val , or Tyr at position 241 of the amino acid sequence of SEQ ID No. 24 (corresponding to position 250 of SEQ ID No. 2), preferably Ala;
In another embodiment, of the isolated nucleic acid of the invention, said HPPD protein comprises the amino acid sequence of SEQ ID No. 26 [Triticum aestivum / wheat], wherein the resulting amino acid sequence comprises an amino acid selected from Ala, Asp, Glu, Gly, Lys,Gln, Arg, Ser, Thr, Val, or Tyr at position 237 of the amino acid sequence of SEQ ID No. 26 (corresponding to position 250 of SEQ ID No. 2), preferably Ala;
In another embodiment, of the isolated nucleic acid of the invention, said HPPD protein comprises the amino acid sequence of SEQ ID No. 2 [Arabidopsis thaliana], wherein the resulting amino acid sequence comprises an amino acid selected from Ala, Asp, Glu, Gly, Lys, Gln, Arg, Ser, Thr, Val, or Tyr at position 250 of the amino acid sequence of SEQ ID No. 2;
In a further embodiment of the nucleic acid of the invention, in said mutated HPPD protein at least two amino acids have been replaced.

An isolated nucleic acid comprising a nucleotide sequence encoding a mutated HPPD protein,
wherein said mutated HPPD protein has HPPD activity,
wherein in said mutated HPPD protein at least one amino acid at position 228, 248, 270, 271, 379 and/or 427 has been replaced by another amino acid.

In an alternative embodiment of the nucleic acid of the invention having at least one amino acid at position 228, 248, 270, 271, 379 and/or 427 deleted or replaced by another amino acid as defined above, said mutated HPPD protein comprises
a) a His at a position in an HPPD protein, said position corresponding to position 226 of the amino acid sequence of SEQ ID No. 2;
b) a Ser at a position in an HPPD protein, said position corresponding to position 267 comprising the amino acid sequence of SEQ ID No. 2 or at a position corresponding thereto in a different HPPD enzyme;
c) an Asn at a position in an HPPD protein, said position corresponding to position 282 of the amino acid sequence of SEQ ID No. 2;
d) a His at a position in an HPPD protein, said position corresponding to position 308 of the amino acid sequence of SEQ ID No. 2;
e) a Tyr at a position in an HPPD protein, said position corresponding to position 342 of the amino acid sequence of SEQ ID No. 2;
f) a Glu at a position in an HPPD protein, said position corresponding to position 394 of the amino acid sequence of SEQ ID No. 2;
g) a Gly at a position in an HPPD protein, said position corresponding to position 420 of the amino acid sequence of SEQ ID No. 2; and
h) an Asn at a position in an HPPD protein, said position corresponding to position 423 of the amino acid sequence of SEQ ID No. 2.

In another embodiment of the isolated nucleic acid as defined above, in said mutated HPPD protein at least one amino acid has been replaced so that the resulting amino acid sequence has at least one selected from
a. Ala, Cys, Gly, Thr or Val at a position in an HPPD protein, said position corresponding to position 228 of the amino acid sequence of SEQ ID No. 2;
b. Ala, Glu, Gly, His, Lys, Leu, Asn, Gln, Arg, Ser, Thr or Tyr at a position in an HPPD protein, said position corresponding to position 248 of the amino acid sequence of SEQ ID No. 2;
c. Ala, Ile, Leu, Met or Val at a position in an HPPD protein, said position corresponding to position 270 of the amino acid sequence of SEQ ID No. 2;
d. Ala, Glu, Hils, Ile, Lys, Leu, Met, Arg, Ser, Thr or Val at a position in an HPPD protein, said position corresponding to position 271 of the amino acid sequence of SEQ ID No. 2;
e. His or Gln at a position in an HPPD protein, said position corresponding to position 379 of the amino acid sequence of SEQ ID No. 2; and
f. Leu or Arg at a position in an HPPD protein, said position corresponding to position 427 of the amino acid sequence of SEQ ID No. 2.

In another embodiment of the isolated nucleic acid as defined above, in said mutated HPPD protein at least one amino acid has been replaced so that the resulting amino acid sequence has at least one selected from
a. Val or Thr at a position in an HPPD protein, said position corresponding to position 228 of the amino acid sequence of SEQ ID No. 2;
b. Leu, Met or Val at a position in an HPPD protein, said position corresponding to position 270 of the amino acid sequence of SEQ ID No. 2;
c. Ala or Ser at a position in an HPPD protein, said position corresponding to position 271 of the amino acid sequence of SEQ ID No. 2,
d. Gln at a position in an HPPD protein, said position corresponding to position 379 of the amino acid sequence of SEQ ID No. 2; and
e. Leu at a position in an HPPD protein, said position corresponding to position 427 of the amino acid sequence of SEQ ID No. 2.

In another embodiment of the nucleic acid as defined above, said mutated HPPD protein is capable of increasing the tolerance of a plant to at least one herbicide acting on HPPD (also called HPPD inhibitor herbicide).

In another embodiment, the present invention relates to a protein encoded by the isolated nucleic acid of the invention.

In a further embodiment, the present invention relates to a chimeric gene comprising a coding sequence comprising the nucleic acid of the invention operably linked to a plant-expressible promoter and optionally a transcription termination and polyadenylation region.

As a regulatory sequence which functions as a promoter in plant cells and plants, use may be made of any promoter sequence of a gene which is naturally expressed in plants, in particular a promoter which is expressed especially in the leaves of plants, such as for example "constitutive" promoters of bacterial, viral or plant origin, or "light-dependent" promoters, such as that of a plant ribulose-biscarboxylase/oxygenase (RuBisCO) small subunit gene, or any suitable known promoter-expressible which may be used. Among the promoters of plant origin, mention will be made of the histone promoters as described in EP 0 507 698 A1, the rice actin promoter (US 5,641,876), or a plant ubiquitin promoter (US 5,510,474). Among the promoters of a plant virus gene, mention will be made of that of the cauliflower mosaic virus (CaMV 19S or 35S, Sanders et al. (1987), Nucleic Acids Res. 15(4):1543-58.), the circovirus (AU 689 311) or the Cassava vein mosaic virus (CsVMV, US 7,053,205).

In one embodiment of this invention, a promoter sequence specific for particular regions or tissues of plants can be used to express the HPPD proteins of the invention, such as promoters specific for seeds (Datla, R. et al., 1997, Biotechnology Ann. Rev. 3, 269-296), especially the napin promoter (EP 255 378 A1), the phaseolin promoter, the glutenin promoter, the helianthinin promoter (WO 92/17580), the albumin promoter (WO 98/45460), the oleosin promoter (WO 98/45461), the SAT1 promoter or the SAT3 promoter (PCT/US98/06978).

Use may also be made of an inducible promoter advantageously chosen from the phenylalanine ammonia lyase (PAL), HMG-CoA reductase (HMG), chitinase, glucanase, proteinase inhibitor (PI), PR1 family gene, nopaline synthase (nos) and vspB promoters (US 5 670 349, Table 3), the HMG2 promoter (US 5 670 349), the apple beta-galactosidase (ABG1) promoter and the apple aminocyclopropane carboxylate synthase (ACC synthase) promoter (WO 98/45445).

According to the invention, use may also be made, in combination with the promoter, of other regulatory sequences, which are located between the promoter and the coding sequence, such as transcription activators ("enhancers"), for instance the translation activator of the tobacco mosaic virus (TMV) described in Application WO 87/07644, or of the tobacco etch virus (TEV) described by Carrington & Freed 1990, J. Virol. 64: 1590-1597, for example, or introns such as the adh1 intron of maize or intron 1 of rice actin.

As a regulatory terminator or polyadenylation sequence, use may be made of any corresponding sequence of bacterial origin, such as for example the nos terminator of *Agrobacterium tumefaciens,* of viral origin, such as for example the CaMV 35S terminator, or of plant origin, such as for example a histone terminator as described in published Patent Application EP 0 633 317 A1.

A method of obtaining a mutated HPPD protein capable of modulating the tolerance of a plant to at least one herbicide acting on HPPD, wherein said mutated HPPD protein has HPPD activity, the method comprising
i. providing an HPPD protein, said HPPD optionally comprising an amino acid sequence, wherein
   a) a His is present at a position in an HPPD protein, said position corresponding to position 226 of the amino acid sequence of SEQ ID No. 2;
   b) a Ser is present at a position in an HPPD protein, said position corresponding to position 267 of the amino acid sequence of SEQ ID No. 2;
   c) an Asn is present at a position in an HPPD protein, said position corresponding to position 282 of the amino acid sequence of SEQ ID No. 2;
   d) a His is present at a position in an HPPD protein, said position corresponding to position 308 of the amino acid sequence of SEQ ID No. 2;
   e) a Tyr is present at a position in an HPPD protein, said position corresponding to position 342 of the amino acid sequence of SEQ ID No. 2;
   f) a Glu is present at a position in an HPPD protein, said position corresponding to position 394 of the amino acid sequence of SEQ ID No. 2;
   g) a Gly is present at a position in an HPPD protein, said position corresponding to position 420 of the amino acid sequence of SEQ ID No. 2; and
   h) an Asn is present at a position in an HPPD protein, said position corresponding to position 423 of the amino acid sequence of SEQ ID No. 2
ii) replacing an amino acid in said HPPD enzyme so that the resulting amino acid sequence has at least one selected from
   Ala, Asp, Glu, Phe, Gly, Lys,Gln, Arg, Ser, Thr, Val, or Tyr at a position in an HPPD protein, said position corresponding to position 250 of the amino acid sequence of SEQ ID No. 2;
   a. and an amino acid deletion or replacement at least one position in an HPPD protein, said position corresponding to at least one of positions 228, 248, 270, 271, 379 and 427 of the amino acid sequence of SEQ ID No. 2;
iii) determining the inhibition of the resulting HPPD protein by at least one herbicide acting on HPPD;
   wherein an inhibition of the resulting protein of less or more than that observed with a reference HPPD protein is indicative that the resulting protein is capable of modulating the tolerance of a plant to said herbicide.

It is to be understood that also the (more specific) amino acids and positions listed above for other embodiments, such as the nucleic acid of the invention, may be applied to the method of obtaining a mutated HPPD protein as described above.

In an alternative embodiment of the method of obtaining a mutated HPPD protein as described above, said mutated HPPD protein is capable of increasing the tolerance of a plant to at least one herbicide acting on HPPD.

Within the above method of obtaining in mutated HPPD protein, different herbicides acting on HPPD may be chosen. Accordingly, in another embodiment of the method of obtaining a mutated HPPD protein as described above, wherein said mutated HPPD protein is capable of increasing the tolerance of a plant to at least one herbicide acting on HPPD, the herbicide acting on HPPD is selected from triketones, or pyrazolinates, preferably tembotrione, mesotrione, topramezone or sulcotrione, bicyclopyrone, pyrasulfotole, pyrazolate, benzofenap and tefuryltrione, particularly tembotrione and such plants containing the HPPD of the invention have an agronomically acceptable tolerance to an HPPD inhibitor herbicide particularly to triketones, or pyrazolinates, preferably tembotrione, mesotrione, topramezone or sulcotrione, bicyclopyrone, pyrasulfotole, pyrazolate, benzofenap and tefuryltrione, particularly tembotrione.

In another embodiment, the present invention relates to a method of producing a transgenic plant comprising introducing into a said plant genome the nucleic acid of the present invention operably linked to a plant expressible promoter, the chimeric gene of the invention or a nucleic acid encoding the HPPD enzyme identified by the method of claim 20 or 21.

In an alternative embodiment of the method of producing a transgenic plant as described above, the nucleic acid of the invention, wherein said mutated HPPD protein is capable of increasing the tolerance of a plant to at least one herbicide acting on HPPD, or a nucleic acid identified by the method of obtaining a mutated HPPD protein, wherein said mutated HPPD protein is capable of increasing the tolerance of a plant to at least one herbicie acting on HPPD, both operably linked to a plant expressible promoter, or the chimeric gene of the invention comprising a nucleic acid, wherein said mutated HPPD protein is capable of increasing the tolerance of a plant to at least one herbicie acting on HPPD, is introduced into said plant.

In another embodiment, the present invention relates to a plant cell comprising the isolated nucleic acid of the invention or the chimeric gene of the invention in its genetic information.

The present invention also relates to a plant, a part of a plant or plant tissue consisting essentially of the plant cells of the invention.

Furthermore, the present invention relates to a plant obtainable from the method of obtaining a mutated HPPD protein capable of modulating or increasing the tolerance of a plant to at least one herbicide acting on HPPD in all alternative aspects described above.

The plant of the present invention can be any plant. Non-limiting examples of plants of the invention include wheat, cotton, canola, rice, corn, soy bean, sorghum,canola, sunflower, tobacco, sugarbeet, cotton, maize, wheat, barley, rice, sorghum, tomato, mango, peach, apple, pear, strawberry, banana, melon, potato, carrot, lettuce, cabbage, onion, soya spp, sugar cane, pea, field beans, poplar, grape, citrus, alfalfa, rye, oats, turf and forage grasses, flax and oilseed rape, and nut producing plants. The present invention also relates to a seed of the plant of the invention.

In a further embodiment, the present invention relates to a method of modulating a plant's tolerance to at least one herbicide acting on HPPD comprising introducing the isolated nucleic acid of the invention operably linked to a plant expressible promoter or the chimeric gene of the invention into a plant's genome
In an alternative embodiment, the present invention relates to a method of increasing a plant's tolerance to at least one herbicide acting on HPPD or of obtaining a plant tolerant to an HPPD inhibitor herbicide comprising introducing the isolated nucleic acid of the invention, wherein said nucleic acid encodes a mutated HPPD protein which is capable of increasing the tolerance of a plant to at least one herbicide acting on HPPD, operably linked to a plant expressible promoter or the chimeric gene of the invention comprising a nucleic acid of the invention, wherein said nucleic acid encodes a mutated HPPD protein which is capable of increasing the tolerance of a plant to at least one herbicide acting on HPPD, into a plant's genome.

Furthermore, the present invention relates to a method for controlling weeds comprising spraying at least one herbicide acting on HPPD on or around a crop plant, wherein said crop plant comprises the nucleic acid of the present invention, wherein said mutated HPPD protein is capable of increasing the tolerance of a plant to at least one herbicide acting on HPPD, operably linked to a plant expressible promoter or the chimeric gene of the invention comprising the nucleic acid of the invention, wherein said mutated HPPD protein is capable of increasing the tolerance of a plant to at least one herbicide acting on HPPD. In alternative embodiment of the method of controlling weeds, the tolerance of said plant to at least one herbicie acting on HPPD is increased.

In addition, the present invention relates to the use of a chimeric gene of the invention or the nucleic acid of the invention operably linked to a plant expressible promoter for modulating the tolerance of a plant to at least one herbicide acting on HPPD.

In an alternative embodiment, the present invention relates to the use of a chimeric gene of the invention or the nucleic acid of the invention operably linked to a plant expressible promoter for increasing the tolerance of a plant to at least one herbicide acting on HPPD. In this embodiment of the invention, the chimeric gene used comprises the nucleic acid of the invention, wherein the mutated HPPD protein encoded thereby is capable of increasing the tolerance of a plant to at least one herbicide acting on HPPD. Alternatively, if a nucleic acid operably linked to a plant expressible promoter is used, said nucleic is chose that the mutated HPPD protein encoded thereby is capable of increasing the tolerance of a plant to at least one herbicide acting on HPPD.

The present invention also relates to the plant cell of the invention and the plant of the invention which may comprise a further useful trait as described further below.

While a number of herbicide-tolerant crop plants are presently commercially available, one issue that has arisen for many commercial herbicides and herbicide/crop combinations is that individual herbicides typically have incomplete spectrum of activity against common weed species. For most individual herbicides which have been in use for some time, populations of herbicide resistant weed species and biotypes have become more prevalent (see, e.g., Tranel and Wright (2002) Weed Science 50: 700-712; Owen and Zelaya (2005) Pest Manag. Sci. 61: 301-311). Transgenic plants which are resistant to more than one herbicide have been described (see, e.g., WO2005/012515). However, improvements in every aspect of crop production, weed control options, extension of residual weed control, and improvement in crop yield are continuously in demand.

The HPPD protein or gene of the invention is advantageously combined in plants with other genes which encode proteins or RNAs that confer useful agronomic properties to such plants. Among the genes which encode proteins or RNAs that confer useful agronomic properties on the transformed plants, mention can be made of the DNA sequences encoding proteins which confer tolerance to one or more herbicides that, according to their chemical structure, differ from HPPD inhibitor herbicides, and others which confer tolerance to certain insects, those which confer tolerance to certain diseases, DNAs that encodes RNAs that provide nematode or insect control, etc...
Such genes are in particular described in published PCT Patent Applications WO 91/02071 and WO95/06128.

Among the DNA sequences encoding proteins which confer tolerance to certain herbicides on the transformed plant cells and plants, mention can be made of a bar or PAT gene or the *Streptomyces coelicolor* gene described in WO2009/152359 which confers tolerance to glufosinate herbicides, a gene encoding a suitable EPSPS which confers tolerance to herbicides having EPSPS as a target, such as glyphosate and its salts (US 4,535,060, US 4,769,061, US 5,094,945, US 4,940,835, US 5,188,642, US 4,971,908, US 5,145,783, US 5,310,667, US 5,312,910, US 5,627,061, US 5,633,435), or a gene encoding glyphosate oxydoreductase (US 5,463,175).
Among the DNA sequences encoding a suitable EPSPS which confer tolerance to the herbicides which have EPSPS as a target, mention will more particularly be made of the gene which encodes a plant EPSPS, in particular maize EPSPS, particularly a maize EPSPS which comprises two mutations, particularly a mutation at amino acid position 102 and a mutation at amino acid position 106
(WO 2004/074443), and which is described in Patent Application US 6566587, hereinafter named double mutant maize EPSPS or 2mEPSPS, or the gene which encodes an EPSPS isolated from Agrobacterium and which is described by sequence ID No. 2 and sequence ID No. 3 of US Patent 5,633,435, also named CP4.

Among the DNA sequences encoding a suitable EPSPS which confer tolerance to the herbicides which have EPSPS as a target, mention will more particularly be made of the gene which encodes an EPSPS GRG23 from Arthrobacter globiformis, but also the mutants GRG23 ACE1, GRG23 ACE2, or GRG23 ACE3, particularly the mutants or variants of GRG23 as described in WO2008/100353, such as GRG23(ace3)R173K of SEQ ID No. 29 in WO2008/100353.

In the case of the DNA sequences encoding EPSPS, and more particularly encoding the above genes, the sequence encoding these enzymes is advantageously preceded by a sequence encoding a transit peptide, in particular the "optimized transit peptide" described in US Patent 5,510,471 or 5,633,448.

In WO 2007/024782, plants being tolerant to glyphosate and at least one ALS (acetolactate synthase) inhibitor are disclosed. More specifically plants containing genes encoding a GAT (Glyphosate-N-Acetyltransferase) polypeptide and a polypeptide conferring resistance to ALS inhibitors are disclosed.
In US 6855533, transgenic tobacco plants containing mutated Arabidopsis ALS/AHAS genes were disclosed.
In US 6,153,401, plants containing genes encoding 2,4-D-monooxygenases conferring tolerance to 2,4-D (2,4-dichlorophenoxyacetic acid) by metabolisation are disclosed.
In US 2008/0119361 and US 2008/0120739, plants containing genes encoding Dicamba monooxygenases conferring tolerance to dicamba (3,6-dichloro-2-methoxybenzoic acid) by metabolisation are disclosed.

All the above mentioned herbicide tolerance traits can be combined with those performing HPPD tolerance which are subject matter of this invention.

Among the DNA sequences encoding proteins concerning properties of tolerance to insects, mention will more particularly be made of the Bt proteins widely described in the literature and well known to those skilled in the art. Mention will also be made of proteins extracted from bacteria such as *Photorhabdus* (WO 97/17432 & WO 98/08932).
Among such DNA sequences encoding proteins of interest which confer novel properties of tolerance to insects, mention will more particularly be made of the Bt Cry or VIP proteins widely described in the literature and well known to those skilled in the art. These include the Cry1 F protein or hybrids derived from a Cry1 F protein (e.g., the hybrid Cry1A-Cry1F proteins described in US 6,326,169; US 6,281,016; US 6,218,188, or toxic fragments thereof), the Cry1A-type proteins or toxic fragments thereof, preferably the Cry1Ac protein or hybrids derived from the Cry1Ac protein (e.g., the hybrid Cry1Ab-Cry1Ac protein described in US 5,880,275) or the Cry1Ab or Bt2 protein or insecticidal fragments thereof as described in EP451878, the Cry2Ae, Cry2Af or Cry2Ag proteins as described in WO02/057664 or toxic fragments thereof, the Cry1A.105 protein described in WO 2007/140256 (SEQ ID No. 7) or a toxic fragment thereof, the VIP3Aa19 protein of NCBI accession ABG20428, the VIP3Aa20 protein of NCBI accession ABG20429 (SEQ ID No. 2 in WO 2007/142840), the VIP3A proteins produced in the COT202 or COT203 cotton events (WO 2005/054479 and WO 2005/054480, respectively), the Cry proteins as described in WO01/47952, the VIP3Aa protein or a toxic fragment thereof as described in Estruch et al. (1996), Proc Natl Acad Sci USA. 28;93(11):5389-94 and US 6,291,156, the insecticidal proteins from *Xenorhabdus* (as described in WO98/50427), *Serratia* (particularly from *S*. *entomophila*) or *Photorhabdus* species strains, such as Tc-proteins from Photorhabdus as described in WO98/08932 (e.g., Waterfield et al., 2001, Appl Environ Microbiol. 67(11):5017-24; Ffrench-Constant and Bowen, 2000, Cell Mol Life Sci.; 57(5):828-33). Also any variants or mutants of any one of these proteins differing in some (1-10, preferably 1-5) amino acids from any of the above sequences, particularly the sequence of their toxic fragment, or which are fused to a transit peptide, such as a plastid transit peptide, or another protein or peptide, is included herein.

The FIGURES show:
- FIGURE1:: Superpositon of the x-ray structure of HPPD from *Arabidopsis thaliana* (dark grey) and (a) *Pseudomonas fluorescens* (light grey), (b) *Streptomyces avermitilis,* (c) *Homo sapiens,* (d) *Rattus norvegicus.* The structures are shown as ribbon plot.
- FIGURE2:: Amino acids forming the binding site of (a) Arabidopsis thaliana, (b) *Pseudomonas fluorescens* (c) *Streptomyces avermitilis (d) Homo sapiens, and (e) Rattus norvegicus* (represented by bold lines). The core of the HPPD protein is shown as Cₐₗₚₕₐ trace and the iron is marked.
- FIGURE3:: Brown test color in *Escherichia coli*

### SEQUENCES LISTING

SEQ ID No. 1: Nucleic acid sequence encoding Arabidopsis thaliana HPPD
SEQ ID No. 2: Protein encoded by SEQ ID No. 1
SEQ ID No. 3: Nucleic acid sequence encoding Oryza sativa HPPD
SEQ ID No. 4: Protein encoded by SEQ ID No. 3
SEQ ID No. 5: Nucleic acid sequence encoding Zea mays HPPD
SEQ ID No. 6: Protein encoded by SEQ ID No. 5
SEQ ID No. 7: Nucleic acid sequence encoding Avena sativa HPPD
SEQ ID No. 8: Protein encoded by SEQ ID No. 7
SEQ ID No. 9: Nucleic acid sequence encoding Pseudomonas fluorescens HPPD
SEQ ID No. 10: Protein encoded by SEQ ID No. 9
SEQ ID No. 13: Nucleic acid sequence encoding Synechococcus sp. HPPD
SEQ ID No. 14: Protein encoded by SEQ ID No. 13
SEQ ID No. 15: Nucleic acid sequence encoding Blepharsima japonicum HPPD
SEQ ID No. 16: Protein encoded by SEQ ID No. 15
SEQ ID No. 17: Nucleic acid sequence encoding Rhodococcus RHA1 isolate ro0341 HPPD
SEQ ID No. 18: Protein encoded by SEQ ID No. 17
SEQ ID No. 19: Nucleic acid sequence encoding Picrophilus torridus HPPD
SEQ ID No. 20: Protein encoded by SEQ ID No. 19
SEQ ID No. 21: Nucleic acid sequence encoding Kordia algicida HPPD
SEQ ID No. 22: Protein encoded by SEQ ID No. 21
SEQ ID No. 23: Nucleic acid sequence encoding Sorghum bicolor HPPD
SEQ ID No. 24: Protein encoded by SEQ ID No. 23
SEQ ID No. 25: Nucleic acid sequence encoding Triticum aestivum HPPD
SEQ ID No. 26: Protein encoded by SEQ ID No. 25
SEQ ID No. 27: Nucleic acid sequence encoding Hordeum vulgare HPPD
SEQ ID No. 28: Protein encoded by SEQ ID No. 27
SEQ ID No. 29: Nucleic acid sequence encoding Rhodococcus RHA1 isolate ro0240 HPPD
SEQ ID No. 30: Protein encoded by SEQ ID No. 29
SEQ ID No. 31: Nucleic acid sequence encoding Arabidopsis thaliana wild type HPPD polypeptide, plus containing at the 5' end a nucleic acid encoding an alanine and 6 histidine amino acids
SEQ ID No. 32: Protein encoded by SEQ ID No. 31
SEQ ID No. 33: Nucleic acid sequence encoding mutant HPPD polypeptide, plus containing at the 5' end a nucleic acid encoding an alanine and 6 histidine amino acids
SEQ ID No. 34: Protein encoded by SEQ ID No. 33,
SEQ ID No. 35: Nucleic acid sequence encoding mutant HPPD polypeptide, plus containing at the 5' end a nucleic acid encoding an alanine and 6 histidine amino acids
SEQ ID No. 36: Protein encoded by SEQ ID No. 35,
SEQ ID No. 37: Nucleic acid sequence encoding mutant HPPD polypeptide, plus containing at the 5' end a nucleic acid encoding an alanine and 6 histidine amino acids
SEQ ID No. 38: Protein encoded by SEQ ID No. 37,
SEQ ID No. 39: Nucleic acid sequence encoding mutant HPPD polypeptide, plus containing at the 5' end a nucleic acid encoding an alanine and 6 histidine amino acids
SEQ ID No. 40: Protein encoded by SEQ ID No. 39,
SEQ ID No. 41: Nucleic acid sequence encoding mutant HPPD polypeptide, plus containing at the 5' end a nucleic acid encoding an alanine and 6 histidine amino acids
SEQ ID No. 42: Protein encoded by SEQ ID No. 41,
SEQ ID No. 43: Nucleic acid sequence encoding mutant HPPD polypeptide, plus containing at the 5' end a nucleic acid encoding an alanine and 6 histidine amino acids
SEQ ID No. 44: Protein encoded by SEQ ID No. 43,
SEQ ID No. 45: Nucleic acid sequence encoding mutant HPPD polypeptide, plus containing at the 5' end a nucleic acid encoding an alanine and 6 histidine amino acids
SEQ ID No. 46: Protein encoded by SEQ ID No. 45
SEQ ID No. 47: Nucleic acid sequence encoding mutant HPPD polypeptide, plus containing at the 5' end a nucleic acid encoding an alanine and 6 histidine amino acids
SEQ ID No. 48: Protein encoded by SEQ ID No. 47
SEQ ID No. 49: Nucleic acid sequence encoding mutant HPPD polypeptide, plus containing at the 5' end a nucleic acid encoding an alanine and 6 histidine amino acids
SEQ ID No. 50: Protein encoded by SEQ ID No. 49,

### The Examples illustrate the invention

### Example1:

A mutation of strictly conserved amino acids leads to an inactive protein.
The point mutants S267A (SEQ ID No. 34) in *A.thaliana* and N282A (SEQ ID No. 36) in *A.thaliana* are inactive. The amino acid position given are referring to the position of SEQ ID No. 2.

Mutant HPPD polypeptides of the presenting invention have amino acids changes at one or more positions relative to the starting wild type sequence from which they are derived.

The DNA sequence SEQ ID No. 2 encoding the wild HPPD protein from Arabidopsis thaliana (1335 bp; Genebank AF047834; WO 96/38567) was cloned into the vector pSE420(RI)NX (modified from the the cloning and expression vector pSE420(RI)NX (5261 bp) is based on the plasmid pSE420 by Invitrogen (Karlsruhe, Germany)). At the 5' end, directly downstream to the ATG was inserted a nucleic acid sequence coding for an alanine amino acid and a nucleic acid sequence encoding a N-terminal HIS6-Tag (6x HIS, encoded by: cat cat cat cac cat cat). The resulting sequence is presented as SEQ ID No. 31. Upstream to the ATG, two additional cysteine base pairs were added in order to obtain a sequence corresponding to the recognition site of the restriction enzyme Ncol and downstream to the stop codon the sequences corresponding to the recognition site of the restriction enzyme Xbal were added. The resulting plasmid was then used to transform E. Coli cells BL21 (DE3) with 50µg/ml kanamycin or 100µg/ml carbenicillin selection as described in European patent applications "EP09015984.9"; EP09015985.6"; EP 09015986.4" EP 09015987.2"; and EP09015988.0" all filed on 23. December 2009.

### Quick test of the activity of HPPD protein: Production of brown color

### Colorimetric Screening Test for Active HPPD Enzymes:

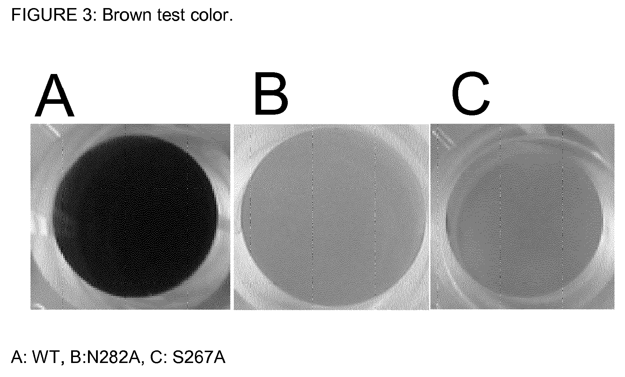

A YT-broth-type culture medium with 1% agarose, 5mM L-Tyrosine and 42mM Succinate, which contains the selection agent for the vector pSE420 is poured into deep well plates. E.coli culture in the exponentional growth phase which contains the vector pSE420-HPPDx (any gene coding for a putative HPPD enzyme/protein) is applied to each well. After 16 hours at 37°C, the wells which do not contain the culture medium, those which have been seeded with an E. coil culture containing the empty vector pSE420 are transparent, or those which have been seeded with an E. coli culture containing a vector pSE420 containing a gene coding for an inactive HPPD are transparent, while the wells seeded with an E. coli culture containing the vector pSE420-HPPD coding for active HPPD are brown. It has been previously demonstrated that this test refelects the HPPD activity, whatever the orgin of this activity, and allows the identification of HPPD activities (US 6,768,044). As it can be seen on Figure 3, the culture from bacteria containing the gene coding for the mutant HPPD did not develop a brown color while the one containing the gene coding of the wild type HPPD developed a strong brown color reflecting the activity of the HPPD enzymes. It can be concluded that the two HPPD mutants are not able to convert HPP into homogentisate. The two HPPD mutants are inactive. One can conclude that the amino acid positions 267 and 282 (referring to position in SEQ ID No. 2) are essential for HPPD activity.

The expression of the HPPD protein was then done as following.

Overnight cultures grown at 37°C were used to inoculate LB media at a ratio 1:100. Cells were allowed to grow until OD reached 0.5, then expression was initiated from the trp-lac (trc) promoter by induction with 1 mM IPTG which binds to the *lac* repressor and causes its dissociation from the *lac* operon. Expression was carried out over 15 h at 28 °C.

To prepare the pre-starter culture, 2 mL of TB medium (100 µg*mL⁻¹ carbenicillin) were inoculated with 50 µL of an *E. coli* K-12 BL21 glycerol stock. The pre-starter culture was incubated at 37 °C with shaking at 140 rpm for 15 h. 200µl of the pre-starter culture was used to initiate the starter culture (5mL TB supplement with 100 µg*L⁻¹), which was incubated 3 h at 37°C.

To prepare the main culture, 400 mL of TB medium (100 µg*mL⁻¹ carbenicillin) were inoculated with 4 mL of the starter culture. This starter culture was incubated at 37 °C with shaking at 140 rpm until OD₆₀₀ 0.5 was reached. Then recombinant protein expression was induced with 400 µl of 1 M IPTG solution. The cells were allowed to grow for an additional hour under these conditions, then the temperature was lowered to 28°C and the culture was shaken at 140 rpm for 15 h. Cells were harvested by centrifugation at 6000 x g for 15 min at 4 °C. Then cell pellets were stored at -80 °C.

### Isolation and purification of HiS₆-AtHPPD in native form

### Lysis of cells

Cells were lysed using Lysozyme, an enzyme that cleaves the 1,4-β-linkages between N-acetylmuramic acid and N-acetyl-D-glucosamine residues in peptidoglycan which forms the bacterial cell wall. Cell membranes were then disrupted by the internal pressure of the bacterial cell. In addition, the lysis buffer contained Benzonase^{®} Nuclease, an endonuclease that hydrolyzes all forms of DNA and RNA without damaging proteins and thereby largely reduces viscosity of the cell lysate. Lysis under native conditions was carried out on ice.
For purification of His₆-tagged proteins the QIA*express*^{®} Ni-NTA Fast Start Kit was used following the user manual instruction.

### Purification of His₆-tagged proteins by immobilized metal ion affinity chromatography (IMAC)

The cleared cell lysate (10 mL) obtained after centrifugation of the lysis reaction was loaded onto a Ni-NTA Fast Start Column from the QIA*express^{®}* Ni-NTA Fast Start Kit (Qiagen, Hilden, Germany) and purification was carried out according to the instruction manual. The His₆-tagged protein was eluted with 2.5 mL of elution buffer.

### Desalting of HPPD solutions by gel filtration

HPPD solutions eluted from a Ni-NTA Fast Start Column with 2.5 mL of elution buffer were applied to a Sephadex G-25 PD-10 column (GE Healthcare, Freiburg, Germany) following the user manual instruction. After the whole sample had entered the gel bed, elution was performed with 3.5 mL of storage buffer.
The HPPD solutions eluted from the desalting column were frozen at -80 °C in 1 mL aliquots.

Determination of HPPD protein concentration using the Bradford protein assay Protein concentration was determined using the standard Bradford assay (Bradford, (1976), Anal Biochem 72: 248-254).
Determination of purity of HPPD solutions using SDS-PAGE

The integrity of the eluted protein was checked by SDS-PAGE protein gel electrophoresis using the gel NuPAGE^{®} Novex 4-12 % Bis-Tris Gels (Invitrogen, Karlsruhe, Germany), approximately 10 µg of protein were loaded. 10 µL of Laemmli Sample Buffer was added to 1-10 µL of protein solution and the mixture was incubated at 90 °C for 10 min. After short centrifugation step, the whole mixture was loaded into a slot of an SDS gel previously fixed in a XCell *SureLock*™ Novex Mini-Cell gel chamber filled with NuPAGE^{®} MOPS SDS Running Buffer (diluted from the 20 x-solution with ddH₂O). A voltage of 150 was then applied to the gel chamber for 1 h. For staining of protein bands, the gel was immersed in Coomassie Brilliant Blue R-250 Staining Solution. For destaining of the polyacrylamide gel, it was immersed in Coomassie Brilliant Blue R-250 Destaining Solution until protein bands appear blue on a white gel.

The HPPD activity was checked by the standard spectrophotmetric assay (method extensively described in WO 2009/144079)

In this content, pl₅₀-value means the log value of the concentration of inhibitor necessary to inhibit 50% of the enzyme activity in molar concentration.

pl₅₀-values for HPPD inhibitors were determined from dose-response plots of HPPD activity versus inhibitor concentration using the assay extensively described in WO 2009/144079 at 2 mM fixed HPP concentration and 3 minutes fixed incubation time using the ID Business Solutions Ltd. XLfit software suite.

Table 7: Determination of pI50 HPPD enzymes (*Arabidopsis thaliana* wild type HPPD "SEQ ID No. 32", the mutants S267A (SEQ ID No. 34) and N282A (SEQ ID No. 36)" and the tolerance to the several listed below HPPD inhibitors tembotrione, diketonitrile, mesotrione, bicyclopyrone, pyrasulfotole, sulcotrione, pyrazolate, tefuryltrione, and benzofenap. The symbol ">" means that the value was far higher than the one indicated but could not be precisely calculated within in the range of concentration of inhibitor tested (2.5x10⁻⁶, 5.0x10⁻⁶, 1.0x10⁻⁵, 2.5x10⁻⁵, 6.3x10⁻⁵, and 2.5x10⁻⁴M).

| | Bicyclopyrone | Benzofenap | Diketonitrile | Mesotrione | |
|---|---|---|---|---|---|
| WT (SEQ ID No. 32) | 5.2 | >5.6 | >5.6 | >5.6 | |
| S267A (SEQ ID No. 34) | nd - la (P) | nd - la (P) | nd - la (P) | nd - la (P) | |
| N282A (SEQ ID No. 36) | nd - la (P) | nd - la (P) | nd - la (P) | nd - la (P) | |

| | Pyrasulfotole | Pyrazolate | Sulcotrione | Tefuryltrione | Tembotrione |
|---|---|---|---|---|---|
| WT (SEQ ID No. 32) | 5.4 | 5.4 | >5.6 | >5.6 | >5.6 |
| S267A (SEQ ID No. 34) | nd - la (P) | nd - la (P) | nd - la (P) | nd - la (P) | nd - la (P) |
| N282A (SEQ ID No. 36) | nd - la (P) | nd - la (P) | nd - la (P) | nd - la (P) | nd - la (P) |

As previously demonstrated, the 2 protein mutants are not able to convert HPP into Homogentisate, confirming that the 2 mutant proteins are inactive. It confirms the hypothesis that the position 267 and 282 (referring to position in SEQ ID No. 2) are absolutely essential for obtaining an active HPPD

Example 2: Single point mutants displayed increased tolerance to HPPD inhibitor herbicides:
Kinetic characterization and evaluation of tolerance to HPPD inhibitors of HPPD enzyme "SEQ ID No. 32".

The HPPD activity was checked by the standard spectrophotmetric assay (method extensively described in WO 2009/144079)

### Determination of HPPD in vitro kinetic properties

Kₘ, Vₘₐₓ, and k_{cat} values for different HPPD enzyme preparations and Kᵢ, K₁=Kₒₙ, and K₋₁=K_{off} for different HPPD inhibitors were determined using a HPLC assay for measurements of HPPD activity. The assay mixtures contained in a volume of 1 ml 150 mM Tris-HCl buffer at pH 7.8, 10 mM sodium ascorbate, 650 units of bovine catalase (Sigma C30 (Sigma-Aldrich, Munich,Germany), 34 mg protein/ml, 23,000 units/mg), and appropriate amounts of HPP, purified HPPD enzyme and HPPD inhibitors. For Kₘ, Vₘₐₓ, and k_{cat} value determination HPP concentrations in the assay mixture were varied between 10 and 400 µM. For Kᵢ, K₁=Kₒₙ, and K₋₁=K_{off} value determination 2 mM HPP was used. All assays were started by the addition of HPPD enzyme to the assay mixture and stopped at a series of times between 0 and 240 s by addition of 200 µl of the reaction mixture to reaction assay tubes containing 20 µl 10% perchloric acid. Precipitated protein was pelleted by a 5 minute centrifugation at 10,000 g. 100 µl of the supernatant were loaded onto a 250 x 4mm Knauer (Berlin, Germany) Eurospher 100-5 C18-column equilibrated with 10% methanol, 0.1% trifluoroacetic acid (buffer A). The column was eluted, also at 1.5 ml/min, using a 4 minute wash with buffer A, followed by a 3 min wash with 95% methanol and by a further 2 minute wash with buffer A. The elution of HGA (homogentisic acid) and HPP (hydroxyphenylpyruvate) was monitored at 292 nm. HGA elutes at around 5 minutes and HPP elutes later. A standard set of concentrations of HGA were used to provide a standard curve in order to calibrate the 292 nm absorbance of the HGA peak versus HGA concentration.
For Kₘ and Vₘₐₓ value determinations the initial rates of the HPPD reaction at different substrate concentrations were determined from plots of HGA formed versus time and fitted to the Michaelis-Menten equation for unireactant enzymes using the ID Business Solutions Ltd. (www.idbs.com) XLfit software suite. For the determination of Kᵢ, K₁=Kₒₙ, and K₋₁=K_{off} values the time-courses of the HPPD reaction at different inhibitor concentrations were fitted to the equations for Mechanism A, competitive inhibition, for tight-binding inhibitors (Cha, S. (1975) Tight-binding inhibitors - I. Kinetic behaviour. Biochemical Pharmacology 24, 2177-2185) using the ID Business Solutions Ltd. XLfit software suite

### Table 8

Protein SEQ ID No. 32 consists of protein SEQ ID No. 2 with an insertion of one amino acid Ala and six amino acids His directly behind the first methionine.

Kinetic characterization of HPPD enzymes (*Arabidopsis thaliana* "SEQ ID No. 32" and the respective tolerance to the HPPD inhibitors tembotrione and diketonitrile. In below given table1, "Kₘ" (Michaelis-Menten constant) means the kinetic parameter that is used to characterise an enzyme, and it is defined as the concentration of substrate that permits half maximal rate of the reaction. Kₘ is further defined as the substrate concentration at which the reaction rate reaches half of its maximum value (*V*ₘₐₓ/2) where Vmax has the meaning of being the maximum velocity of the reaction. Kₒₙ=K₁ equals the association rate constant of the enzyme-substrate binding and K_{off}=K₋₁ equals the rate constant of the enzyme-inhibitor complex dissociation. Ki defines the inhibition constant.

| | HPP | | Tembotrione | | | Diketonitrile | | |
|---|---|---|---|---|---|---|---|---|
| | Kₘ (µM) | Vₘₐₓ (µM) | k₁ (M⁻¹ S⁻¹) | k₋₁ (S⁻¹) | Kᵢ (µM) | k₁ (M⁻¹ S⁻¹) | k₋₁ (S⁻¹) | Kᵢ (µM) |
| SEQ ID No. 32 | 6.3 | 1.2 | 2.3E+05 | 3.5E-03 | 0.015 | 6.1E+05 | 1.1E-02 | 0.018 |

The kinetic parameters Kₘ and Vₘₐₓ of the plant HPPD "SEQ ID No. 32" summarized in Table 8 are well in the range of the specific activities measured from purified plant HPPD as for example for the carrot HPPD (Garcia et al. 2000, Biochemistry, 39, 7501-7507). The Arabidopsis thaliana HPPD is also sensitive in the smimilar range as the carrot HPPD to diketonitrile. It is the first time, that the kinetic parameter of Arabidopsis thaliana HPPD in presence of tembotrione are measured and being in a comparable range as the one measured in presence of the HPPD inhibitor diketonitrile.

### Determination of HPPD activity in presence of several HPPD inhibitors

In this content, pI₅₀-value means the log value of the concentration of inhibitor necessary to inhibit 50% of the enzyme activity in molar concentration.

pI₅₀-values for HPPD inhibitors were determined from dose-response plots of HPPD activity versus inhibitor concentration using the assay extensively described in WO 2009/144079 at 2 mM fixed HPP concentration and 3 minutes fixed incubation time using the ID Business Solutions Ltd. XLfit software suite.

## Claims

1. An isolated nucleic acid comprising a nucleotide sequence encoding a mutated HPPD protein, wherein said mutated HPPD protein has HPPD activity, and wherein in said mutated HPPD protein the amino acid at a position corresponding to position 250 of SEQ ID No. 2 has been replaced so that the resulting amino acid sequence comprises an amino acid selected from Ala, Asp, Glu, Phe, Gly, Lys, Gln, Arg, Ser, Thr, Val or Tyr at the position corresponding to position 250 of SEQ ID No. 2.

2. The isolated nucleic acid of claim 1, wherein said HPPD protein is derived from Streptomyces avermitilis (Genebank SAV11864), Daucus carota (Genebank DCU 87257), Arabidopsis thaliana (Genebank AF047834), Mycosphaerella graminicola (Genebank AF038152), oryza sativa / rice [BAD26248], Zea mays / corn [ACN36372], avena sativa [ABZ23427], Pseudomonas fluorescens [ABF50055], Synechococcus sp. [YP_473959], Blepharisma japonicum [BAF91881], Rhodococcus RHA1 sp. ro0240 [YP_702005], Rhodococcus RHA1 sp. ro0341 [YP_703002], Picrophilus torridus [YP_024147], Kordia algicida [ZP_02161490], Sorghum bicolor [XP_002453359], Triticum aestivum / wheat [AAZ67144],
or Hordeum vulgare / barley [048604].

3. The isolated nucleic acid of claim 1, wherein said HPPD protein comprises the amino acid sequence of SEQ ID No. 4 and wherein the resulting amino acid sequence comprises an amino acid selected from Ala, Asp, Glu, Gly, Lys, Gln, Arg, Ser, Thr, Val or Tyr, at position 247 of the amino acid sequence of SEQ ID No. 4 (corresponding to position 250 of SEQ ID No. 2).

4. The isolated nucleic acid of claim 1, wherein said HPPD protein comprises the amino acid sequence of SEQ ID No. 6 and wherein the resulting amino acid sequence comprises an amino acid selected from Ala, Asp, Glu, Gly, Lys, Gln, Arg, Ser, Thr, Val or Tyr at position 224 of the amino acid sequence of SEQ ID No. 6 (corresponding to position 250 of SEQ ID No. 2).

5. The isolated nucleic acid of claim 1, wherein said HPPD protein comprises the amino acid sequence of SEQ ID No. 8 and wherein the resulting amino acid sequence comprises an amino acid selected from Ala, Asp, Glu, Gly, Lys, Gln, Arg, Ser, Thr, Val or Tyr at position 241 of the amino acid sequence of SEQ ID No. 8 (corresponding to position 250 of SEQ ID No. 2).

6. The isolated nucleic acid of claim 1, wherein said HPPD protein comprises the amino acid sequence of SEQ ID No. 10 and wherein the resulting amino acid sequence comprises an amino acid selected from Phe, Asp, Glu, Gly, Lys, Gln, Arg, Ser, Thr, Val or Tyr at position 188 of the amino acid sequence of SEQ ID No. 10 (corresponding to position 250 of SEQ ID No. 2).

7. The isolated nucleic acid of claim 1, wherein said HPPD protein comprises the amino acid sequence of SEQ ID No. 14 and wherein the resulting amino acid sequence comprises an amino acid selected from Ala, Asp, Glu, Phe, Gly, Lys, Gln, Arg, Ser, Thr, Val or Tyr at position 169 of the amino acid sequence of SEQ ID No. 14 (corresponding to position 250 of SEQ ID No. 2).

8. The isolated nucleic acid of claims 1, wherein said HPPD protein comprises the amino acid sequence of SEQ ID No. 16 and wherein the resulting amino acid sequence comprises an amino acid selected from Ala, Asp, Glu, Phe, Gly, Lys, Gln, Arg, Ser, Thr or Val at position 209 of the amino acid sequence of SEQ ID No. 16 (corresponding to position 250 of SEQ ID No. 2).

9. The isolated nucleic acid of claim 1, wherein said HPPD protein comprises the amino acid sequence of SEQ ID No. 18 and wherein the resulting amino acid sequence comprises an amino acid selected from Ala, Asp, Glu, Phe, Gly, Lys, Gln, Arg, Ser, Thr, Val or Tyr at position 231 of the amino acid sequence of SEQ ID No. 18 (corresponding to position 250 of SEQ ID No. 2).

10. The isolated nucleic acid of claim 1, wherein said HPPD protein comprises the amino acid sequence of SEQ ID No. 30 and wherein the resulting amino acid sequence comprises an amino acid selected from Ala, Asp, Glu, Phe, Gly, Lys, Gln, Arg, Ser, Thr, Val or Tyr at position 232 of the amino acid sequence of SEQ ID No. 30 (corresponding to position 250 of SEQ ID No. 2).

11. The isolated nucleic acid of claims 1, wherein said HPPD protein comprises the amino acid sequence of SEQ ID No. 20 and wherein the resulting amino acid sequence comprises an amino acid selected from Ala, Asp, Glu, Phe, Gly, Lys, Gln, Arg, Ser, Thr, Val or Tyr at position 201 of the amino acid sequence of SEQ ID No. 20 (corresponding to position 250 of SEQ ID No. 2).

12. The isolated nucleic acid of claim 1, wherein said HPPD protein comprises the amino acid sequence of SEQ ID No. 22 and wherein the resulting amino acid sequence comprises an amino acid selected from Ala, Asp, Glu, Phe, Gly, Lys, Gln, Arg, Ser, Thr, Val or Tyr at position 219 of the amino acid sequence of SEQ ID No. 22 (corresponding to position 250 of SEQ ID No. 2),

13. The isolated nucleic acid of claim 1, wherein said HPPD protein comprises the amino acid sequence of SEQ ID No. 24 and wherein the resulting amino acid sequence comprises an amino acid selected from Ala, Asp, Glu, Gly, Lys,Gin, Arg, Ser, Thr, Val or Tyr at position 241 of the amino acid sequence of SEQ ID No. 24 (corresponding to position 250 of SEQ ID No. 2).

14. The isolated nucleic acid of claim 1, wherein said HPPD protein comprises the amino acid sequence of SEQ ID No. 26 and wherein the resulting amino acid sequence comprises an amino acid selected from Ala, Asp, Glu, Gly, Lys,Gin, Arg, Ser, Thr, Val or Tyr at position 237 of the amino acid sequence of SEQ ID No. 26 (corresponding to position 250 of SEQ ID No. 2).

15. The isolated nucleic acid of claim 1, wherein said HPPD protein comprises the amino acid sequence of SEQ ID No. 2 and wherein the resulting amino acid sequence comprises an amino acid selected from Ala, Asp, Glu, Gly, Lys, Gln, Arg, Ser, Thr, Val or Tyr at position 250 of the amino acid sequence of SEQ ID No. 2.

16. The nucleic acid of any one of claims 1 to 15, wherein said mutated HPPD protein is capable of increasing the tolerance of a plant to at least one herbicide acting on HPPD.

17. A protein encoded by the isolated nucleic acid of any one of claims 1 to 15.

18. A chimeric gene comprising a coding sequence comprising the nucleic acid of any one of claims 1 to 15 operably linked to a plant-expressible promoter and optionally a transcription termination and polyadenylation region.

19. A method of obtaining a mutated HPPD protein capable of modulating the tolerance of a plant to at least one herbicide acting on HPPD, wherein said mutated HPPD protein has HPPD activity, the method comprising
i) providing an HPPD protein;
ii) replacing one amino acid in said HPPD protein so that the resulting amino acid sequence comprises one selected from Ala, Asp, Glu, Phe, Gly, Lys,Gin, Arg, Ser, Thr, Val or Tyr at a position in an HPPD protein, said position corresponding to position 250 of the amino acid sequence of SEQ ID No. 2;
iii) determining the inhibition of the resulting HPPD protein by at least one herbicide acting on HPPD,
wherein an inhibition of the resulting protein of less or more than that observed with a reference HPPD protein is indicative that the resulting protein is capable of modulating the tolerance of a plant to said herbicide.

20. The method of claim 19, wherein said mutated HPPD protein is capable of increasing the tolerance of a plant to at least one HPPD inhibitor herbicide.

21. The nucleic acid of claim 16 or the method of claim 20, wherein the herbicide acting on HPPD is tembotrione.

22. A method of producing a transgenic plant comprising introducing into a said plant the nucleic acid of any one of claims 1 to 16 or a nucleic acid encoding the HPPD enzyme identified by the method of any one of claims 19 to 20, both operably linked to a plant expressible promoter or the chimeric gene of claim 19.

23. The method of claim 22, wherein the nucleic acid of claim 16 or a nucleic acid identified by the method of claim 19 or 20, both operably linked to a plant expressible promoter, or the chimeric gene of claim 18 comprising the nucleic acid of any one of claims 1 to 16 is introduced into said plant.

24. The nucleic acid of any one of claims 1 to 15 or the method of any one of claims 22 to 23, wherein said HPPD protein comprises
a) a His at a position in an HPPD protein, said position corresponding to position 226 of the amino acid sequence of SEQ ID No. 2;
b) a Ser at a position in an HPPD protein, said position corresponding to position 267 of the amino acid sequence of SEQ ID No. 2;
c) an Asn at a position in an HPPD protein, said position corresponding to position 282 of the amino acid sequence of SEQ ID No. 2;
d) a His at a position in an HPPD protein, said position corresponding to position 308 of the amino acid sequence of SEQ ID No. 2;
e) a Tyr at a position in an HPPD protein, said position corresponding to position 342 of the amino acid sequence of SEQ ID No. 2;
f) a Glu at a position in an HPPD protein, said position corresponding to position 394 of the amino acid sequence of SEQ ID No. 2;
g) a Gly at a position in an HPPD protein, said position corresponding to position 420 of the amino acid sequence of SEQ ID No. 2; and
h) an Asn at a position in an HPPD protein, said position corresponding to position 423 of the amino acid sequence of SEQ ID No. 2

25. A plant cell comprising the isolated nucleic acid of any one of claims 1 to 15 or the chimeric gene of claim 19 in its genetic information.

26. A plant, part thereof or plant tissue consisting essentially of the plant cells of claim 25.

27. A plant obtainable from the method of any one of claims 22 or 23.

28. The plant of claim 25 or 26 which is selected from wheat, cotton, canola, rice, corn, soy bean, sorghum.

29. A seed of the plant of any one of claims 25 to 27, comprising the isolated nucleic acid of any one of claims 1 to 15 or the chimeric gene of claim 18 in its genetic information.

30. Progeny of the plant of any one of claims 25 to 28 or the seed of claim 29, comprising the isolated nucleic acid of any one of claims 1 to 15 or the chimeric gene of claim 18 in its genetic information.

31. A method of modulating a plant's tolerance to at least one herbicide acting on HPPD or of obtaining a plant tolerant to an HPPD inhibitor herbicide comprising introducing the isolated nucleic acid of any one of claims 1 to 15 operably linked to a plant expressible promoter or the chimeric gene of claim 18 into a plant's genome.

32. Use of a chimeric gene of claim 18 or the nucleic acid of any one of claims 1 to 15 operably linked to a plant expressible promoter for modulating the tolerance of a plant to at least one HPPD inhibitor herbicide applied for controlling weeds.

33. The method of claim 19, 22, 31 or the use of claim 32, wherein the tolerance of a plant to at least one HPPD inhibitor herbicide is increased.

## Patentansprüche

1. Isolierte Nukleinsäure, die eine Nukleotidsequenz, die für ein mutiertes HPPD-Protein codiert, umfasst, wobei das mutierte HPPD-Protein HPPD-Aktivität aufweist, und wobei in dem mutierten HPPD-Protein die Aminosäure an einer Position entsprechend Position 250 von SEQ ID No. 2 so ersetzt worden ist, dass die entstandene Aminosäuresequenz eine Aminosäure, ausgewählt aus Ala, Asp, Glu, Phe, Gly, Lys, Gln, Arg, Ser, Thr, Val oder Tyr an der Position entsprechend Position 250 von SEQ ID No. 2, umfasst.

2. Isolierte Nukleinsäure nach Anspruch 1, wobei das HPPD-Protein von Streptomyces avermitilis (Genebank SAV11864), Daucus carota (Genebank DCU 87257), Arabidopsis thaliana (Genebank AF047834), Mycosphaerella graminicola (Genebank AF038152), Oryza sativa/Reis [BAD26248], Zea mays/Mais [ACN36372], Avena sativa [ABZ23427], Pseudomonas fluorescens [ABF50055], Synechococcus sp. [YP_473959], Blepharisma japonicum [BAF91881], Rhodococcus RHA1 sp. ro0240 [YP_702005], Rhodococcus RHA1 sp. ro0341 [YP_703002], Picrophilus torridus [YP_024147], Kordia algicida [ZWISCHENPOSITION_02161490], Sorghum bicolor [XP_002453359], Triticum aestivum/Weizen [AAZ67144] oder Hordeum vulgare/Gerste [048604] abgeleitet ist.

3. Isolierte Nukleinsäure nach Anspruch 1, wobei das HPPD-Protein die Aminosäuresequenz von SEQ ID No. 4 umfasst und wobei die entstandene Aminosäuresequenz eine Aminosäure, ausgewählt aus Ala, Asp, Glu, Gly, Lys, Gln, Arg, Ser, Thr, Val oder Tyr an Position 247 der Aminosäuresequenz von SEQ ID No. 4 (entsprechend Position 250 von SEQ ID No. 2), umfasst.

4. Isolierte Nukleinsäure nach Anspruch 1, wobei das HPPD-Protein die Aminosäuresequenz von SEQ ID No. 6 umfasst und wobei die entstandene Aminosäuresequenz eine Aminosäure, ausgewählt aus Ala, Asp, Glu, Gly, Lys, Gln, Arg, Ser, Thr, Val oder Tyr an Position 224 der Aminosäuresequenz von SEQ ID No. 6 (entsprechend Position 250 von SEQ ID No. 2), umfasst.

5. Isolierte Nukleinsäure nach Anspruch 1, wobei das HPPD-Protein die Aminosäuresequenz von SEQ ID No. 8 umfasst und wobei die entstandene Aminosäuresequenz eine Aminosäure, ausgewählt aus Ala, Asp, Glu, Gly, Lys, Gln, Arg, Ser, Thr, Val oder Tyr an Position 241 der Aminosäuresequenz von SEQ ID No. 8 (entsprechend Position 250 von SEQ ID No. 2), umfasst.

6. Isolierte Nukleinsäure nach Anspruch 1, wobei das HPPD-Protein die Aminosäuresequenz von SEQ ID No. 10 umfasst und wobei die entstandene Aminosäuresequenz eine Aminosäure, ausgewählt aus Phe, Asp, Glu, Gly, Lys, Gln, Arg, Ser, Thr, Val oder Tyr an Position 188 der Aminosäuresequenz von SEQ ID No. 10 (entsprechend Position 250 von SEQ ID No. 2), umfasst.

7. Isolierte Nukleinsäure nach Anspruch 1, wobei das HPPD-Protein die Aminosäuresequenz von SEQ ID No. 14 umfasst und wobei die entstandene Aminosäuresequenz eine Aminosäure, ausgewählt aus Ala, Asp, Glu, Phe, Gly, Lys, Gln, Arg, Ser, Thr, Val oder Tyr an Position 169 der Aminosäuresequenz von SEQ ID No. 14 (entsprechend Position 250 von SEQ ID No. 2), umfasst.

8. Isolierte Nukleinsäure nach Anspruch 1, wobei das HPPD-Protein die Aminosäuresequenz von SEQ ID No. 16 umfasst und wobei die entstandene Aminosäuresequenz eine Aminosäure, ausgewählt aus Ala, Asp, Glu, Phe, Gly, Lys, Gln, Arg, Ser, Thr oder Val an Position 209 der Aminosäuresequenz von SEQ ID No. 16 (entsprechend Position 250 von SEQ ID No. 2), umfasst.

9. Isolierte Nukleinsäure nach Anspruch 1, wobei das HPPD-Protein die Aminosäuresequenz von SEQ ID No. 18 umfasst und wobei die entstandene Aminosäuresequenz eine Aminosäure, ausgewählt aus Ala, Asp, Glu, Phe, Gly, Lys, Gln, Arg, Ser, Thr, Val oder Tyr an Position 231 der Aminosäuresequenz von SEQ ID No. 18 (entsprechend Position 250 von SEQ ID No. 2), umfasst.

10. Isolierte Nukleinsäure nach Anspruch 1, wobei das HPPD-Protein die Aminosäuresequenz von SEQ ID No. 30 umfasst und wobei die entstandene Aminosäuresequenz eine Aminosäure, ausgewählt aus Ala, Asp, Glu, Phe, Gly, Lys, Gln, Arg, Ser, Thr, Val oder Tyr an Position 232 der Aminosäuresequenz von SEQ ID No. 30 (entsprechend Position 250 von SEQ ID No. 2), umfasst.

11. Isolierte Nukleinsäure nach Anspruch 1, wobei das HPPD-Protein die Aminosäuresequenz von SEQ ID No. 20 umfasst und wobei die entstandene Aminosäuresequenz eine Aminosäure, ausgewählt aus Ala, Asp, Glu, Phe, Gly, Lys, Gln, Arg, Ser, Thr, Val oder Tyr an Position 201 der Aminosäuresequenz von SEQ ID No. 20 (entsprechend Position 250 von SEQ ID No. 2), umfasst.

12. Isolierte Nukleinsäure nach Anspruch 1, wobei das HPPD-Protein die Aminosäuresequenz von SEQ ID No. 22 umfasst und wobei die entstandene Aminosäuresequenz eine Aminosäure, ausgewählt aus Ala, Asp, Glu, Phe, Gly, Lys, Gln, Arg, Ser, Thr, Val oder Tyr an Position 219 der Aminosäuresequenz von SEQ ID No. 22 (entsprechend Position 250 von SEQ ID No. 2), umfasst.

13. Isolierte Nukleinsäure nach Anspruch 1, wobei das HPPD-Protein die Aminosäuresequenz von SEQ ID No. 24 umfasst und wobei die entstandene Aminosäuresequenz eine Aminosäure, ausgewählt aus Ala, Asp, Glu, Gly, Lys, Gln, Arg, Ser, Thr, Val oder Tyr an Position 241 der Aminosäuresequenz von SEQ ID No. 24 (entsprechend Position 250 von SEQ ID No. 2), umfasst.

14. Isolierte Nukleinsäure nach Anspruch 1, wobei das HPPD-Protein die Aminosäuresequenz von SEQ ID No. 26 umfasst und wobei die entstandene Aminosäuresequenz eine Aminosäure, ausgewählt aus Ala, Asp, Glu, Gly, Lys, Gln, Arg, Ser, Thr, Val oder Tyr an Position 237 der Aminosäuresequenz von SEQ ID No. 26 (entsprechend Position 250 von SEQ ID No. 2), umfasst.

15. Isolierte Nukleinsäure nach Anspruch 1, wobei das HPPD-Protein die Aminosäuresequenz von SEQ ID No. 2 umfasst und wobei die entstandene Aminosäuresequenz eine Aminosäure, ausgewählt aus Ala, Asp, Glu, Gly, Lys, Gln, Arg, Ser, Thr, Val oder Tyr an Position 250 der Aminosäuresequenz von SEQ ID No. 2, umfasst.

16. Nukleinsäure nach einem der Ansprüche 1 bis 15, wobei das mutierte HPPD-Protein fähig ist, die Toleranz einer Pflanze gegenüber mindestens einem auf HPPD einwirkenden Herbizid zu erhöhen.

17. Protein, das von der isolierten Nukleinsäure nach einem der Ansprüche 1 bis 15 codiert wird.

18. Chimäres Gen, das eine Codiersequenz, umfassend die Nukleinsäure nach einem der Ansprüche 1 bis 15 in operativer Verknüpfung mit einem in Pflanzen exprimierbarem Promoter und gegebenenfalls einer Transkriptionsterminations- und Polyadenylierungsregion, umfasst.

19. Verfahren zum Erhalten eines mutierten HPPD-Proteins, das fähig ist, die Toleranz einer Pflanze gegenüber mindestens einem auf HPPD einwirkenden Herbizid zu modulieren, wobei das mutierte HPPD-Protein HPPD-Aktivität aufweist, wobei das Verfahren Folgendes umfasst:
i) Bereitstellen eines HPPD-Proteins;
ii) Ersetzen einer Aminosäure in dem HPPD-Protein so, dass die erhaltene Aminosäuresequenz eine ausgewählt aus Ala, Asp, Glu, Phe, Gly, Lys, Gln, Arg, Ser, Thr, Val oder Tyr an einer Position in einem HPPD-Protein umfasst, wobei die Position Position 250 der Aminosäuresequenz gemäß SEQ ID No. 2 entspricht;
iii) Bestimmen der Hemmung des erhaltenen HPPD-Proteins durch mindestens ein auf HPPD einwirkendes Herbizid,
wobei eine Hemmung des entstandenen Proteins von weniger oder mehr als derjenigen, die bei einem Referenz-HPPD-Protein beobachtet wird, anzeigt, dass das entstandene Protein fähig ist, die Toleranz einer Pflanze gegenüber dem Herbizid zu modulieren.

20. Verfahren nach Anspruch 19, wobei das mutierte HPPD-Protein fähig ist, die Toleranz einer Pflanze gegenüber mindestens einem HPPD-Hemmer-Herbizid zu erhöhen.

21. Nukleinsäure nach Anspruch 16 oder Verfahren nach Anspruch 20, wobei es sich bei dem auf HPPD einwirkenden Herbizid um Tembotrion handelt.

22. Verfahren zum Herstellen einer transgenen Pflanze, bei dem man die Nukleinsäure nach einem der Ansprüche 1 bis 16 oder eine Nukleinsäure, die für das nach dem Verfahren nach einem der Ansprüche 19 bis 20 identifizierte HPPD-Enzym codiert, beide in operativer Verknüpfung mit einem in Pflanzen exprimierbaren Promoter oder das chimäre Gen nach Anspruch 19 in eine die Pflanze einführt.

23. Verfahren nach Anspruch 22, wobei die Nukleinsäure nach Anspruch 16 oder eine nach dem Verfahren nach Anspruch 19 oder 20 identifizierte Nukleinsäure, beide in operativer Verknüpfung mit einem in Pflanzen exprimierbaren Promoter, oder das chimäre Gen nach Anspruch 18 umfassend die Nukleinsäure nach einem der Ansprüche 1 bis 16 in die Pflanze eingeführt wird.

24. Nukleinsäure nach einem der Ansprüche 1 bis 15 oder Verfahren nach einem der Ansprüche 22 bis 23, wobei das HPPD-Protein Folgendes umfasst:
a) ein His in einer Position in einem HPPD-Protein, wobei die Position Position 226 der Aminosäuresequenz von SEQ ID No. 2 entspricht;
b) ein Ser in einer Position in einem HPPD-Protein, wobei die Position Position 267 der Aminosäuresequenz von SEQ ID No. 2 entspricht;
c) ein Asn in einer Position in einem HPPD-Protein, wobei die Position Position 282 der Aminosäuresequenz von SEQ ID No. 2 entspricht;
d) ein His in einer Position in einem HPPD-Protein, wobei die Position Position 308 der Aminosäuresequenz von SEQ ID No. 2 entspricht;
e) ein Tyr in einer Position in einem HPPD-Protein, wobei die Position Position 342 der Aminosäuresequenz von SEQ ID No. 2 entspricht;
f) ein Glu in einer Position in einem HPPD-Protein, wobei die Position Position 394 der Aminosäuresequenz von SEQ ID No. 2 entspricht;
g) ein Gly in einer Position in einem HPPD-Protein, wobei die Position Position 420 der Aminosäuresequenz von SEQ ID No. 2 entspricht; und
h) ein Asn in einer Position in einem HPPD-Protein, wobei die Position Position 423 der Aminosäuresequenz von SEQ ID No. 2 entspricht.

25. Pflanzenzelle, die die isolierte Nukleinsäure nach einem der Ansprüche 1 bis 15 oder das chimäre Gen nach Anspruch 19 in ihrer genetischen Information umfasst.

26. Pflanze, Teil davon oder Pflanzengewebe, die/der/das im Wesentlichen aus den Pflanzenzellen nach Anspruch 25 besteht.

27. Pflanze, die von dem Verfahren nach einem der Ansprüche 22 oder 23 erhältlich ist.

28. Pflanze nach Anspruch 25 oder 26, die aus Weizen, Baumwolle, Canola, Reis, Mais, Sojabohne und Sorghum ausgewählt ist.

29. Samen der Pflanze nach einem der Ansprüche 25 bis 27, der die isolierte Nukleinsäure nach einem der Ansprüche 1 bis 15 oder das chimäre Gen nach Anspruch 18 in seiner genetischen Information umfasst.

30. Nachkomme der Pflanze nach einem der Ansprüche 25 bis 28 oder des Samens nach Anspruch 29, der die isolierte Nukleinsäure nach einem der Ansprüche 1 bis 15 oder das chimäre Gen nach Anspruch 18 in seiner genetischen Information umfasst.

31. Verfahren zum Modulieren der Toleranz einer Pflanze gegenüber mindestens einem auf HPPD einwirkenden Herbizid oder zum Erhalten einer Pflanze, die gegen ein HPPD-Hemmer-Herbizid tolerant ist, bei dem man die isolierte Nukleinsäure nach einem der Ansprüche 1 bis 15 in operativer Verknüpfung mit einem in Pflanzen exprimierbaren Promoter oder das chimäre Gen nach Anspruch 18 in das Genom einer Pflanze einführt.

32. Verwendung eines chimären Gens nach Anspruch 18 oder der Nukleinsäure nach einem der Ansprüche 1 bis 15 in operativer Verknüpfung mit einem in Pflanzen exprimierbaren Promoter zum Modulieren der Toleranz einer Pflanze gegenüber mindestens einem HPPD-Hemmer-Herbizid, das für die Bekämpfung von Unkräutern ausgebracht wird.

33. Verfahren nach Anspruch 19, 22, 31 oder Verwendung nach Anspruch 32, wobei die Toleranz einer Pflanze gegenüber mindestens einem HPPD-Hemmer-Herbizid erhöht ist.

## Revendications

1. Acide nucléique isolé comprenant une séquence de nucléotides codant pour une protéine HPPD mutée, **caractérisé en ce que** ladite protéine HPPD mutée possède une activité HPPD, et **caractérisé en ce que** dans ladite protéine HPPD mutée l'acide aminé à une position correspondant à la position 250 de SEQ ID No. 2 a été remplacé de sorte que la séquence d'acides aminés résultante comprenne un acide aminé choisi parmi Ala, Asp, Glu, Phe, Gly, Lys, Gln, Arg, Ser, Thr, Val ou Tyr à la position correspondant à la position 250 de SEQ ID No. 2.

2. Acide nucléique isolé de la revendication 1, **caractérisé en ce que** ladite protéine HPPD est dérivée de Streptomyces avermitilis (Genebank SAV11864), Daucus carota (Genebank DCU 87257), Arabidopsis thaliana (Genebank AF047834), Mycosphaerella graminicola (Genebank AF038152), Oryza sativa / riz [BAD26248], Zea mays / maïs [ACN36372], Avena sativa [ABZ23427], Pseudomonas fluorescens [ABF50055], Synechococcus sp. [YP_473959], Blepharisma japonicum [BAF91881], Rhodococcus RHA1 sp. ro0240 [YP_702005], Rhodococcus RHA1 sp. ro0341 [YP_703002], Picrophilus torridus [YP_024147], Kordia algicida [ZP_02161490], Sorghum bicolor [XP_002453359], Triticum aestivum / blé [AAZ67144], ou Hordeum vulgare / orge [048604].

3. Acide nucléique isolé de la revendication 1, **caractérisé en ce que** ladite protéine HPPD comprend la séquence d'acides aminés de SEQ ID No. 4 et **caractérisé en ce que** la séquence d'acides aminés résultante comprend un acide aminé choisi parmi Ala, Asp, Glu, Gly, Lys, Gln, Arg, Ser, Thr, Val ou Tyr, à la position 247 de la séquence d'acides aminés de SEQ ID No. 4 (correspondant à la position 250 de SEQ ID No. 2).

4. Acide nucléique isolé de la revendication 1, **caractérisé en ce que** ladite protéine HPPD comprend la séquence d'acides aminés de SEQ ID No. 6 et **caractérisé en ce que** la séquence d'acides aminés résultante comprend un acide aminé choisi parmi Ala, Asp, Glu, Gly, Lys, Gln, Arg, Ser, Thr, Val ou Tyr à la position 224 de la séquence d'acides aminés de SEQ ID No. 6 (correspondant à la position 250 de SEQ ID No. 2).

5. Acide nucléique isolé de la revendication 1, **caractérisé en ce que** ladite protéine HPPD comprend la séquence d'acides aminés de SEQ ID No. 8 et **caractérisé en ce que** la séquence d'acides aminés résultante comprend un acide aminé choisi parmi Ala, Asp, Glu, Gly, Lys, Gln, Arg, Ser, Thr, Val ou Tyr à la position 241 de la séquence d'acides aminés de SEQ ID No. 8 (correspondant à la position 250 de SEQ ID No. 2).

6. Acide nucléique isolé de la revendication 1, **caractérisé en ce que** ladite protéine HPPD comprend la séquence d'acides aminés de SEQ ID No. 10 et **caractérisé en ce que** la séquence d'acides aminés résultante comprend un acide aminé choisi parmi Phe, Asp, Glu, Gly, Lys, Gln, Arg, Ser, Thr, Val ou Tyr à la position 188 de la séquence d'acides aminés de SEQ ID No. 10 (correspondant à la position 250 de SEQ ID No. 2).

7. Acide nucléique isolé de la revendication 1, **caractérisé en ce que** ladite protéine HPPD comprend la séquence d'acides aminés de SEQ ID No. 14 et **caractérisé en ce que** la séquence d'acides aminés résultante comprend un acide aminé choisi parmi Ala, Asp, Glu, Phe, Gly, Lys, Gln, Arg, Ser, Thr, Val ou Tyr à la position 169 de la séquence d'acides aminés de SEQ ID No. 14 (correspondant à la position 250 de SEQ ID No. 2).

8. Acide nucléique isolé de la revendication 1, **caractérisé en ce que** ladite protéine HPPD comprend la séquence d'acides aminés de SEQ ID No. 16 et **caractérisé en ce que** la séquence d'acides aminés résultante comprend un acide aminé choisi parmi Ala, Asp, Glu, Phe, Gly, Lys, Gln, Arg, Ser, Thr ou Val à la position 209 de la séquence d'acides aminés de SEQ ID No. 16 (correspondant à la position 250 de SEQ ID No. 2).

9. Acide nucléique isolé de la revendication 1, **caractérisé en ce que** ladite protéine HPPD comprend la séquence d'acides aminés de SEQ ID No. 18 et **caractérisé en ce que** la séquence d'acides aminés résultante comprend un acide aminé choisi parmi Ala, Asp, Glu, Phe, Gly, Lys, Gln, Arg, Ser, Thr, Val ou Tyr à la position 231 de la séquence d'acides aminés de SEQ ID No. 18 (correspondant à la position 250 de SEQ ID No. 2).

10. Acide nucléique isolé de la revendication 1, **caractérisé en ce que** ladite protéine HPPD comprend la séquence d'acides aminés de SEQ ID No. 30 et **caractérisé en ce que** la séquence d'acides aminés résultante comprend un acide aminé choisi parmi Ala, Asp, Glu, Phe, Gly, Lys, Gln, Arg, Ser, Thr, Val ou Tyr à la position 232 de la séquence d'acides aminés de SEQ ID No. 30 (correspondant à la position 250 de SEQ ID No. 2).

11. Acide nucléique isolé de la revendication 1, **caractérisé en ce que** ladite protéine HPPD comprend la séquence d'acides aminés de SEQ ID No. 20 et **caractérisé en ce que** la séquence d'acides aminés résultante comprend un acide aminé choisi parmi Ala, Asp, Glu, Phe, Gly, Lys, Gln, Arg, Ser, Thr, Val ou Tyr à la position 201 de la séquence d'acides aminés de SEQ ID No. 20 (correspondant à la position 250 de SEQ ID No. 2).

12. Acide nucléique isolé de la revendication 1, **caractérisé en ce que** ladite protéine HPPD comprend la séquence d'acides aminés de SEQ ID No. 22 et **caractérisé en ce que** la séquence d'acides aminés résultante comprend un acide aminé choisi parmi Ala, Asp, Glu, Phe, Gly, Lys, Gln, Arg, Ser, Thr, Val ou Tyr à la position 219 de la séquence d'acides aminés de SEQ ID No. 22 (correspondant à la position 250 de SEQ ID No. 2).

13. Acide nucléique isolé de la revendication 1, **caractérisé en ce que** ladite protéine HPPD comprend la séquence d'acides aminés de SEQ ID No. 24 et **caractérisé en ce que** la séquence d'acides aminés résultante comprend un acide aminé choisi parmi Ala, Asp, Glu, Gly, Lys, Gln, Arg, Ser, Thr, Val ou Tyr à la position 241 de la séquence d'acides aminés de SEQ ID No. 24 (correspondant à la position 250 de SEQ ID No. 2).

14. Acide nucléique isolé de la revendication 1, **caractérisé en ce que** ladite protéine HPPD comprend la séquence d'acides aminés de SEQ ID No. 26 et **caractérisé en ce que** la séquence d'acides aminés résultante comprend un acide aminé choisi parmi Ala, Asp, Glu, Gly, Lys, Gln, Arg, Ser, Thr, Val ou Tyr à la position 237 de la séquence d'acides aminés de SEQ ID No. 26 (correspondant à la position 250 de SEQ ID No. 2).

15. Acide nucléique isolé de la revendication 1, **caractérisé en ce que** ladite protéine HPPD comprend la séquence d'acides aminés de SEQ ID No. 2 et **caractérisé en ce que** la séquence d'acides aminés résultante comprend un acide aminé choisi parmi Ala, Asp, Glu, Gly, Lys, Gln, Arg, Ser, Thr, Val ou Tyr à la position 250 de la séquence d'acides aminés de SEQ ID No. 2.

16. Acide nucléique de l'une quelconque des revendications 1 à 15, **caractérisé en ce que** ladite protéine HPPD mutée est capable d'augmenter la tolérance d'une plante à au moins un herbicide agissant sur HPPD.

17. Protéine codée par l'acide nucléique isolé de l'une quelconque des revendications 1 à 15.

18. Gène chimérique comprenant une séquence codante comprenant l'acide nucléique de l'une quelconque des revendications 1 à 15 fonctionnellement lié à un promoteur pouvant être exprimé par une plante et facultativement une terminaison de transcription et une région de polyadénylation.

19. Procédé d'obtention d'une protéine HPPD mutée capable de moduler la tolérance d'une plante à au moins un herbicide agissant sur HPPD, où ladite protéine HPPD mutée possède une activité HPPD, le procédé comprenant
i) la fourniture d'une protéine HPPD ;
ii) le remplacement d'un acide aminé dans ladite protéine HPPD de sorte que la séquence d'acides aminés résultante comprenne l'un choisi parmi Ala, Asp, Glu, Phe, Gly, Lys, Gln, Arg, Ser, Thr, Val ou Tyr à une position dans une protéine HPPD, ladite position correspondant à la position 250 de la séquence d'acides aminés de SEQ ID No. 2 ;
iii) la détermination de l'inhibition de la protéine HPPD résultante par au moins un herbicide agissant sur HPPD, dans lequel une inhibition de la protéine résultante inférieure ou supérieure à celle observée avec une protéine HPPD de référence indique que la protéine résultante est capable de moduler la tolérance d'une plante audit herbicide.

20. Procédé de la revendication 19, dans lequel ladite protéine HPPD mutée est capable d'augmenter la tolérance d'une plante à au moins un herbicide inhibiteur de HPPD.

21. Acide nucléique de la revendication 16 ou procédé de la revendication 20, **caractérisé en ce que** l'herbicide agissant sur HPPD est la tembotrione.

22. Procédé de production d'une plante transgénique comprenant l'introduction dans un ladite plante de l'acide nucléique de l'une quelconque des revendications 1 à 16 ou d'un acide nucléique codant pour l'enzyme HPPD identifiée par le procédé de l'une quelconque des revendications 19 à 20, tous deux fonctionnellement liés à un promoteur pouvant être exprimé par une plante ou le gène chimérique de la revendication 19.

23. Procédé de la revendication 22, dans lequel l'acide nucléique de la revendication 16 ou un acide nucléique identifié par le procédé de la revendication 19 ou 20, tous deux fonctionnellement liés à un promoteur pouvant être exprimé par une plante, ou le gène chimérique de la revendication 18 comprenant l'acide nucléique de l'une quelconque des revendications 1 à 16 est introduit dans ladite plante.

24. Acide nucléique de l'une quelconque des revendications 1 à 15 ou le procédé de l'une quelconque des revendications 22 à 23, **caractérisé en ce que** ladite protéine HPPD comprend
a) un His à une position dans une protéine HPPD, ladite position correspondant à la position 226 de la séquence d'acides aminés de SEQ ID No. 2 ;
b) un Ser à une position dans une protéine HPPD, ladite position correspondant à la position 267 de la séquence d'acides aminés de SEQ ID No. 2 ;
c) un Asn à une position dans une protéine HPPD, ladite position correspondant à la position 282 de la séquence d'acides aminés de SEQ ID No. 2 ;
d) un His à une position dans une protéine HPPD, ladite position correspondant à la position 308 de la séquence d'acides aminés de SEQ ID No. 2 ;
e) un Tyr à une position dans une protéine HPPD, ladite position correspondant à la position 342 de la séquence d'acides aminés de SEQ ID No. 2 ;
f) un Glu à une position dans une protéine HPPD, ladite position correspondant à la position 394 de la séquence d'acides aminés de SEQ ID No. 2 ;
g) un Gly à une position dans une protéine HPPD, ladite position correspondant à la position 420 de la séquence d'acides aminés de SEQ ID No. 2 ; et
h) un Asn à une position dans une protéine HPPD, ladite position correspondant à la position 423 de la séquence d'acides aminés de SEQ ID No. 2.

25. Une cellule de plante comprenant l'acide nucléique isolé de l'une quelconque des revendications 1 à 15 ou le gène chimérique de la revendication 19 dans ses informations génétiques.

26. Plante, partie de celle-ci ou tissu de plante essentiellement constitué des cellules de plante de la revendication 25.

27. Plante pouvant être obtenu par le procédé de l'une quelconque des revendications 22 ou 23.

28. Plante de la revendication 25 ou 26 qui est choisi parmi le blé, le coton, le canola, le riz, le maïs, le soja, le sorgho.

29. Graine de la plante de l'une quelconque des revendications 25 à 27, comprenant l'acide nucléique isolé de l'une quelconque des revendications 1 à 15 ou le gène chimérique de la revendication 18 dans ses informations génétiques.

30. Descendance de la plante de l'une quelconque des revendications 25 à 28 ou graine de la revendication 29, comprenant l'acide nucléique isolé de l'une quelconque des revendications 1 à 15 ou le gène chimérique de la revendication 18 dans ses informations génétiques.

31. Procédé de modulation de la tolérance d'une plante à au moins un herbicide agissant sur HPPD ou d'obtention d'une plante tolérante à un herbicide inhibiteur de HPPD comprenant l'introduction de l'acide nucléique isolé de l'une quelconque des revendications 1 à 15 fonctionnellement lié à un promoteur pouvant être exprimé par une plante ou le gène chimérique de la revendication 18 dans le génome d'une plante.

32. Utilisation d'un gène chimérique de la revendication 18 ou de l'acide nucléique de l'une quelconque des revendications 1 à 15 fonctionnellement lié à un promoteur pouvant être exprimé par une plante pour moduler la tolérance d'une plante à au moins un herbicide inhibiteur de HPPD appliqué pour lutter contre les mauvaises herbes.

33. Procédé de la revendication 19, 22, 31 ou utilisation de la revendication 32, dans lequel la tolérance d'une plante à au moins un herbicide inhibiteur de HPPD est augmentée.
